# EUROPEAN PATENT SPECIFICATION

(11) **EP 2 471 941 B1**
(45) Date of publication and mention of the grant of the patent: **02.09.2015**
(21) Application number: 12161803.7
(22) Date of filing: 02.05.2007
(51) Int. Cl.: C12P 7/62, C12P 7/40

(54) **Production of peracids using an enzyme having perhydrolysis activity**
Herstellung von Peraciden über ein Enzym mit Perhydrolyse-Aktivität
Production de peracides au moyen d'une enzyme ayant une activité de perhydrolyse

(30) Priority: 12.12.2006 US 638635
(43) Date of publication of application: 04.07.2012
(62) Divisional of application: 07776622.8
(73) Proprietor: E. I. du Pont de Nemours and Company, Wilmington, DE 19898 (US)
(72) Inventor: Dicosimo, Robert, Chadds Ford, PA Pennsylvania 19317 (US); Gavagan, John E., Wilmington, DE Delaware 19808 (US); Payne, Mark S., Wilmington, DE Delaware 19808 (US); Cooling, Frederick B., III, Wilmington, DE Delaware 19808 (US)
(74) Representative: Webber, Philip Michael

(56) References cited:
- WO-A-2006/119060
- WO-A-2007/070609
- US-A- 5 296 161
- US-A- 5 338 676
- VINCENT F ET AL: "Multifunctional Xylooligosaccharide/Cephalosporin C Deacetylase Revealed by the Hexameric Structure of the Bacillus subtilis Enzyme at 1.9 Angst. Resolution", JOURNAL OF MOLECULAR BIOLOGY, LONDON, GB, vol. 330, no. 3, 11 July 2003 (2003-07-11) , pages 593-606, XP004434207, ISSN: 0022-2836

### SEQUENCE LISTING

<110> E.I. duPont de Nemours and Company Inc.
   Di Cosimo, Robert
   Cooling, Frederick
   Gavagan, John
   Payne, Mark
<120> PRODUCTION OF PERACIDS USING AN ENZYME HAVING PERHYDROLYSIS ACTIVITY
<130> CL3345 US CIP1
<150> US 11/638,635
   <151> 2006-12-12
<150> US 11/743354
   <151> 2007-05-02
<150> US 60/750,092
   <151> 2005-12-13
<150> US 60/853,065
   <151> 2006-10-20
<160> 74
<170> PatentIn version 3.4
<210> 1
   <211> 960
   <212> DNA
   <213> Bacillus subtilis ATCC 31954
<220>
   <221> CDS
   <222> (1)..(960)
<400> 1
   atg caa cta ttc gat ctg ccg ctc gac caa ttg caa aca tat aag cct        48
   Met Gln Leu Phe Asp Leu Pro Leu Asp Gln Leu Gln Thr Tyr Lys Pro
   1               5                   10                  15
   gaa aaa aca gca ccg aaa gat ttt tct gag ttt tgg aaa ttg tct ttg        96
   Glu Lys Thr Ala Pro Lys Asp Phe Ser Glu Phe Trp Lys Leu Ser Leu
               20                  25                  30
   gag gaa ctt gca aaa gtc caa gca gaa cct gat tta cag ccg gtt gac       144
   Glu Glu Leu Ala Lys Val Gln Ala Glu Pro Asp Leu Gln Pro Val Asp
           35                  40                  45
   tat cct gct gac gga gta aaa gtg tac cgt ctc aca tat aaa agc ttc       192
   Tyr Pro Ala Asp Gly Val Lys Val Tyr Arg Leu Thr Tyr Lys Ser Phe
       50                  55                  60
   gga aac gcc cgc att acc gga tgg tac gcg gtg cct gac aag caa ggc       240
   Gly Asn Ala Arg Ile Thr Gly Trp Tyr Ala Val Pro Asp Lys Gln Gly
   65                  70                  75                  80
   ccg cat ccg gcg atc gtg aaa tat cat ggc tac aat gca agc tat gat       288
   Pro His Pro Ala Ile Val Lys Tyr His Gly Tyr Asn Ala Ser Tyr Asp
                   85                  90                  95
   ggt gag att cat gaa atg gta aac tgg gca ctc cat ggc tac gcc gca       336
   Gly Glu Ile His Glu Met Val Asn Trp Ala Leu His Gly Tyr Ala Ala
               100                 105                 110
   ttc ggc atg ctt gtc cgc ggc cag cag agc agc gag gat acg agt att      384
   Phe Gly Met Leu Val Arg Gly Gln Gln Ser Ser Glu Asp Thr Ser Ile
           115                 120                 125
   tca ctg cac ggt cac gct ttg ggc tgg atg acg aaa gga att ctt gat      432
   Ser Leu His Gly His Ala Leu Gly Trp Met Thr Lys Gly Ile Leu Asp
       130                 135                 140
   aaa gat aca tac tat tac cgc ggt gtt tat ttg gac gcc gtc cgc gcg      480
   Lys Asp Thr Tyr Tyr Tyr Arg Gly Val Tyr Leu Asp Ala Val Arg Ala
   145                 150                 155                 160
   ctt gag gtc atc agc agc ttc gac gag gtt gac gaa aca agg atc ggt      528
   Leu Glu Val Ile Ser Ser Phe Asp Glu Val Asp Glu Thr Arg Ile Gly
                   165                 170                 175
   gtg aca gga gga agc caa ggc gga ggt tta acc att gcc gca gca gcg      576
   Val Thr Gly Gly Ser Gln Gly Gly Gly Leu Thr Ile Ala Ala Ala Ala
               180                 185                 190
   ctg tca gac att cca aaa gcc gcg gtt gcc gat tat cct tat tta agc      624
   Leu Ser Asp Ile Pro Lys Ala Ala Val Ala Asp Tyr Pro Tyr Leu Ser
           195                 200                 205
   aac ttc gaa cgg gcc att gat gtg gcg ctt gaa cag ccg tac ctt gaa      672
   Asn Phe Glu Arg Ala Ile Asp Val Ala Leu Glu Gln Pro Tyr Leu Glu
       210                 215                 220
   atc aat tcc ttc ttc aga aga aat ggc agc ccg gaa aca gaa gtg cag      720
   Ile Asn Ser Phe Phe Arg Arg Asn Gly Ser Pro Glu Thr Glu Val Gln
   225                 230                 235                 240
   gcg atg aag aca ctt tca tat ttc gat att atg aat ctc gct gac cga      768
   Ala Met Lys Thr Leu Ser Tyr Phe Asp Ile Met Asn Leu Ala Asp Arg
                   245                 250                 255
   gtg aag gtg cct gtc ctg atg tca atc ggc ctg att gac aag gtc acg      816
   Val Lys Val Pro Val Leu Met Ser Ile Gly Leu Ile Asp Lys Val Thr
               260                 265                 270
   ccg ccg tcc acc gtg ttt gcc gcc tac aat cat ttg gaa aca gag aaa      864
   Pro Pro Ser Thr Val Phe Ala Ala Tyr Asn His Leu Glu Thr Glu Lys
           275                 280                 285
   gag ctg aag gtg tac cgc tac ttc gga cat gag tat atc cct gct ttt      912
   Glu Leu Lys Val Tyr Arg Tyr Phe Gly His Glu Tyr Ile Pro Ala Phe
       290                 295                 300
   caa acg gaa aaa ctt gct ttc ttt aag cag cat ctt aaa ggc tga taa      960
   Gln Thr Glu Lys Leu Ala Phe Phe Lys Gln His Leu Lys Gly
   305                 310                 315
<210> 2
   <211> 318
   <212> PRT
   <213> Bacillus subtilis ATCC 31954
<400> 2
   Met Gln Leu Phe Asp Leu Pro Leu Asp Gln Leu Gln Thr Tyr Lys Pro
   1               5                   10                  15
   Glu Lys Thr Ala Pro Lys Asp Phe Ser Glu Phe Trp Lys Leu Ser Leu
               20                  25                  30
   Glu Glu Leu Ala Lys Val Gln Ala Glu Pro Asp Leu Gln Pro Val Asp
           35                  40                  45
   Tyr Pro Ala Asp Gly Val Lys Val Tyr Arg Leu Thr Tyr Lys Ser Phe
       50                  55                  60
   Gly Asn Ala Arg Ile Thr Gly Trp Tyr Ala Val Pro Asp Lys Gln Gly
   65                  70                  75                  80
   Pro His Pro Ala Ile Val Lys Tyr His Gly Tyr Asn Ala Ser Tyr Asp
                   85                  90                  95
   Gly Glu Ile His Glu Met Val Asn Trp Ala Leu His Gly Tyr Ala Ala
               100                 105                 110
   Phe Gly Met Leu Val Arg Gly Gln Gln Ser Ser Glu Asp Thr Ser Ile
           115                 120                 125
   Ser Leu His Gly His Ala Leu Gly Trp Met Thr Lys Gly Ile Leu Asp
       130                 135                 140
   Lys Asp Thr Tyr Tyr Tyr Arg Gly Val Tyr Leu Asp Ala Val Arg Ala
   145                 150                 155                 160
   Leu Glu Val Ile Ser Ser Phe Asp Glu Val Asp Glu Thr Arg Ile Gly
                   165                 170                 175
   Val Thr Gly Gly Ser Gln Gly Gly Gly Leu Thr Ile Ala Ala Ala Ala
               180                 185                 190
   Leu Ser Asp Ile Pro Lys Ala Ala Val Ala Asp Tyr Pro Tyr Leu Ser
           195                 200                 205
   Asn Phe Glu Arg Ala Ile Asp Val Ala Leu Glu Gln Pro Tyr Leu Glu
       210                 215                 220
   Ile Asn Ser Phe Phe Arg Arg Asn Gly Ser Pro Glu Thr Glu Val Gln
   225                 230                 235                 240
   Ala Met Lys Thr Leu Ser Tyr Phe Asp Ile Met Asn Leu Ala Asp Arg
                   245                 250                 255
   Val Lys Val Pro Val Leu Met Ser Ile Gly Leu Ile Asp Lys Val Thr
               260                 265                 270
   Pro Pro Ser Thr Val Phe Ala Ala Tyr Asn His Leu Glu Thr Glu Lys
   275 280 285
   Glu Leu Lys Val Tyr Arg Tyr Phe Gly His Glu Tyr Ile Pro Ala Phe
   290 295 300
   Gln Thr Glu Lys Leu Ala Phe Phe Lys Gln His Leu Lys Gly
   305 310 315
<210> 3
   <211> 24
   <212> DNA
   <213> artificial sequence
<220>
   <223> Primer
<400> 3
   atgcaactat tcgatctgcc gctc 24
<210> 4
   <211> 26
   <212> DNA
   <213> artificial sequence
<220>
   <223> Primer
<400> 4
   ttatcagcct ttaagatgct gcttaa 26
<210> 5
   <211> 957
   <212> DNA
   <213> Bacillus subtilis subsp. subtilis strain 168
<400> 5
   atgcaactat tcgatctgcc gctcgaccaa ttgcaaacat ataagcctga aaaaacagca 60
   ccgaaagatt tttctgagtt ttggaaattg tctttggagg aacttgcaaa agtccaagca 120
   gaacctgatt tacagccggt tgactatcct gctgacggag taaaagtgta ccgtctcaca 180
   tataaaagct tcggaaacgc ccgcattacc ggatggtacg cggtgcctga caaggaaggc 240
   ccgcatccgg cgatcgtgaa atatcatggc tacaatgcaa gctatgatgg tgagattcat 300
   gaaatggtaa actgggcact ccatggctac gccacattcg gcatgcttgt ccgcggccag 360
   cagagcagcg aggatacgag tatttcaccg cacggtcacg ctttgggctg gatgacgaaa 420
   ggaattcttg ataaagatac atactattac cgcggtgttt atttggacgc cgtccgcgcg 480
   cttgaggtca tcagcagctt cgacgaggtt gacgaaacaa ggatcggtgt gacaggagga 540
   agccaaggcg gaggtttaac cattgccgca gcagcgctgt cagacattcc aaaagccgcg 600
   gttgccgatt atccttattt aagcaacttc gaacgggcca ttgatgtggc gcttgaacag 660
   ccgtaccttg aaatcaattc cttcttcaga agaaatggca gcccggaaac agaagtgcag 720
   gcgatgaaga cactttcata tttcgatatt atgaatctcg ctgaccgagt gaaggtgcct 780
   gtcctgatgt caatcggcct gattgacaag gtcacgccgc cgtccaccgt gtttgccgcc 840
   tacaatcatt tggaaacaaa gaaagagctg aaggtgtacc gctacttcgg acatgagtat 900
   atccctgctt ttcaaactga aaaacttgct ttctttaagc agcatcttaa aggctga 957
<210> 6
   <211> 318
   <212> PRT
   <213> Bacillus subtilis subsp. subtilis strain 168
<400> 6
   Met Gln Leu Phe Asp Leu Pro Leu Asp Gln Leu Gln Thr Tyr Lys Pro
   1               5                   10                  15
   Glu Lys Thr Ala Pro Lys Asp Phe Ser Glu Phe Trp Lys Leu Ser Leu
               20                  25                  30
   Glu Glu Leu Ala Lys Val Gln Ala Glu Pro Asp Leu Gln Pro Val Asp
           35                  40                  45
   Tyr Pro Ala Asp Gly Val Lys Val Tyr Arg Leu Thr Tyr Lys Ser Phe
       50                  55                  60
   Gly Asn Ala Arg Ile Thr Gly Trp Tyr Ala Val Pro Asp Lys Glu Gly
   65                  70                  75                  80
   Pro His Pro Ala Ile Val Lys Tyr His Gly Tyr Asn Ala Ser Tyr Asp
                   85                  90                  95
   Gly Glu Ile His Glu Met Val Asn Trp Ala Leu His Gly Tyr Ala Thr
               100                 105                 110
   Phe Gly Met Leu Val Arg Gly Gln Gln Ser Ser Glu Asp Thr Ser Ile
           115                 120                 125
   Ser Pro His Gly His Ala Leu Gly Trp Met Thr Lys Gly Ile Leu Asp
       130                 135                 140
   Lys Asp Thr Tyr Tyr Tyr Arg Gly Val Tyr Leu Asp Ala Val Arg Ala
   145                 150                 155                 160
   Leu Glu Val Ile Ser Ser Phe Asp Glu Val Asp Glu Thr Arg Ile Gly
                   165                 170                 175
   Val Thr Gly Gly Ser Gln Gly Gly Gly Leu Thr Ile Ala Ala Ala Ala
               180                 185                 190
   Leu Ser Asp Ile Pro Lys Ala Ala Val Ala Asp Tyr Pro Tyr Leu Ser
           195                 200                 205
   Asn Phe Glu Arg Ala Ile Asp Val Ala Leu Glu Gln Pro Tyr Leu Glu
       210                 215                 220
   Ile Asn Ser Phe Phe Arg Arg Asn Gly Ser Pro Glu Thr Glu Val Gln
   225                 230                 235                 240
   Ala Met Lys Thr Leu Ser Tyr Phe Asp Ile Met Asn Leu Ala Asp Arg
                   245                 250                 255
   Val Lys Val Pro Val Leu Met Ser Ile Gly Leu Ile Asp Lys Val Thr
               260                 265                 270
   Pro Pro Ser Thr Val Phe Ala Ala Tyr Asn His Leu Glu Thr Lys Lys
           275                 280                 285
   Glu Leu Lys Val Tyr Arg Tyr Phe Gly His Glu Tyr Ile Pro Ala Phe
       290                 295                 300
   Gln Thr Glu Lys Leu Ala Phe Phe Lys Gln His Leu Lys Gly
   305                 310                 315
<210> 7
   <211> 957
   <212> DNA
   <213> Bacillus subtilis ATCC 6633
<400> 7
   atgcaactat tcgatctgcc gctcgaccaa ttgcaaacgt ataagcctga aaaaacaaca 60
   ccgaacgatt tttctgagtt ttggaaatcg tctttggacg aacttgcgaa agtcaaagca 120
   gcacctgatt tacagctggt tgattatcct gctgatggag tcaaggtgta ccgcctcaca 180
   tataaaagct tcggaaacgc ccgcattacc ggatggtacg cagtgcctga caaggaagga 240
   ccgcatccgg cgatcgtcaa atatcatggc tacaacgcta gctatgacgg tgagattcat 300
   gaaatggtaa actgggcgct ccacggttac gccgcattcg gcatgctagt ccgcggccag 360
   cagagcagcg aggatacgag tatttctcca catggccatg ctttgggctg gatgacgaaa 420
   ggaatccttg ataaagatac atactattac cggggcgttt atttggacgc tgtccgcgcg 480
   cttgaggtca tcagcagctt tgacgaagtt gacgaaacaa gaatcggtgt gacaggcgga 540
   agccaaggag gcggcttaac cattgccgca gccgctctgt cagacattcc aaaagccgcg 600
   gttgccgatt atccttattt aagcaacttt gaacgggcca ttgatgtggc gcttgaacag 660
   ccgtaccttg aaatcaattc cttctttaga agaaatggaa gcccggaaac ggaagagaag 720
   gcgatgaaga cactttcata tttcgatatt atgaatctcg ctgaccgagt gaaggtccct 780
   gtcctgatgt cgatcggtct gattgacaag gtcacgccgc cgtccaccgt gtttgccgca 840
   tacaaccact tggagacaga gaaagagctc aaagtgtacc gctacttcgg gcatgagtat 900
   atccctgcct ttcaaacaga aaaacttgct ttctttaagc agcatcttaa aggctga 957
<210> 8
   <211> 318
   <212> PRT
   <213> Bacillus subtilis ATCC 6633
<400> 8
   Met Gln Leu Phe Asp Leu Pro Leu Asp Gln Leu Gln Thr Tyr Lys Pro
   1               5                   10                  15
   Glu Lys Thr Thr Pro Asn Asp Phe Ser Glu Phe Trp Lys Ser Ser Leu
               20                  25                  30
   Asp Glu Leu Ala Lys Val Lys Ala Ala Pro Asp Leu Gln Leu Val Asp
           35                  40                  45
   Tyr Pro Ala Asp Gly Val Lys Val Tyr Arg Leu Thr Tyr Lys Ser Phe
       50                  55                  60
   Gly Asn Ala Arg Ile Thr Gly Trp Tyr Ala Val Pro Asp Lys Glu Gly
   65                  70                  75                  80
   Pro His Pro Ala Ile Val Lys Tyr His Gly Tyr Asn Ala Ser Tyr Asp
                   85                  90                  95
   Gly Glu Ile His Glu Met Val Asn Trp Ala Leu His Gly Tyr Ala Ala
               100                 105                 110
   Phe Gly Met Leu Val Arg Gly Gln Gln Ser Ser Glu Asp Thr Ser Ile
           115                 120                 125
   Ser Pro His Gly His Ala Leu Gly Trp Met Thr Lys Gly Ile Leu Asp
       130                 135                 140
   Lys Asp Thr Tyr Tyr Tyr Arg Gly Val Tyr Leu Asp Ala Val Arg Ala
   145                 150                 155                 160
   Leu Glu Val Ile Ser Ser Phe Asp Glu Val Asp Glu Thr Arg Ile Gly
                   165                 170                 175
   Val Thr Gly Gly Ser Gln Gly Gly Gly Leu Thr Ile Ala Ala Ala Ala
               180                 185                 190
   Leu Ser Asp Ile Pro Lys Ala Ala Val Ala Asp Tyr Pro Tyr Leu Ser
           195                 200                 205
   Asn Phe Glu Arg Ala Ile Asp Val Ala Leu Glu Gln Pro Tyr Leu Glu
       210                 215                 220
   Ile Asn Ser Phe Phe Arg Arg Asn Gly Ser Pro Glu Thr Glu Glu Lys
   225                 230                 235                 240
   Ala Met Lys Thr Leu Ser Tyr Phe Asp Ile Met Asn Leu Ala Asp Arg
                   245                 250                 255
   Val Lys Val Pro Val Leu Met Ser Ile Gly Leu Ile Asp Lys Val Thr
               260                 265                 270
   Pro Pro Ser Thr Val Phe Ala Ala Tyr Asn His Leu Glu Thr Glu Lys
           275                 280                 285
   Glu Leu Lys Val Tyr Arg Tyr Phe Gly His Glu Tyr Ile Pro Ala Phe
       290                 295                 300
   Gln Thr Glu Lys Leu Ala Phe Phe Lys Gln His Leu Lys Gly
   305                 310                 315
<210> 9
   <211> 957
   <212> DNA
   <213> Bacillus licheniformis ATCC 14580
<400> 9
   atgcagcagc cttatgatat gccgcttgaa cagctttatc agtataaacc tgaacggacg      60
   gcaccggccg attttaaaga gttctggaag ggttcattgg aggaattggc aaatgaaaaa     120
   gcgggaccgc agcttgaacc gcatgaatat ccggctgacg gggtaaaagt ctactggctt     180
   acatacagaa gcatcggggg agcgcgaatt aaaggctggt acgcagtacc cgaccgccaa     240
   gggcctcatc ctgcgatcgt caaataccac ggctataacg caagctatga cggagacatt     300
   cacgatattg tcaattgggc tcttcacggc tatgcggcat tcggtatgct ggtccgcgga     360
   cagaacagca gtgaagatac agagatctct catcacggac atgtacccgg ctggatgaca     420
   aaaggaatcc tcgatccgaa aacatattac tacagagggg tctatttaga tgccgtacga     480
   gcagtcgaag tggtcagcgg ttttgctgaa gtcgatgaaa agcggatcgg ggtgatcggg     540
   gcaagccaag gaggcgggct ggccgtcgcg gtttcggcgc tgtccgatat tccaaaagca     600
   gccgtgtcag aataccctta tttaagcaat tttcaacgag cgatcgatac agcgatcgac     660
   cagccatatc tcgaaatcaa ctcctttttc agaagaaaca ccagtccgga tattgagcag     720
   gcggccatgc ataccctgtc ttatttcgat gtcatgaacc ttgcccaatt ggtcaaagcg     780
   accgtactca tgtcgatcgg actggttgac accatcactc cgccatccac cgtctttgcg     840
   gcttacaatc acttggaaac ggataaagaa ataaaagtgt accgttattt tggacacgaa     900
   tacatcccgc cgttccaaac cgaaaagctg gcgtttctga gaaagcatct gaaataa        957
<210> 10
   <211> 318
   <212> PRT
   <213> Bacillus licheniformis ATCC 14580
<400> 10
   Met Gln Gln Pro Tyr Asp Met Pro Leu Glu Gln Leu Tyr Gln Tyr Lys
   1 5 10 15
   Pro Glu Arg Thr Ala Pro Ala Asp Phe Lys Glu Phe Trp Lys Gly Ser
   20 25 30
   Leu Glu Glu Leu Ala Asn Glu Lys Ala Gly Pro Gln Leu Glu Pro His
   35 40 45
   Glu Tyr Pro Ala Asp Gly Val Lys Val Tyr Trp Leu Thr Tyr Arg Ser
   50 55 60
   Ile Gly Gly Ala Arg Ile Lys Gly Trp Tyr Ala Val Pro Asp Arg Gln
   65 70 75 80
   Gly Pro His Pro Ala Ile Val Lys Tyr His Gly Tyr Asn Ala Ser Tyr
   85 90 95
   Asp Gly Asp Ile His Asp Ile Val Asn Trp Ala Leu His Gly Tyr Ala
   100 105 110
   Ala Phe Gly Met Leu Val Arg Gly Gln Asn Ser Ser Glu Asp Thr Glu
   115 120 125
   Ile Ser His His Gly His Val Pro Gly Trp Met Thr Lys Gly Ile Leu
   130 135 140
   Asp Pro Lys Thr Tyr Tyr Tyr Arg Gly Val Tyr Leu Asp Ala Val Arg
   145 150 155 160
   Ala Val Glu Val Val Ser Gly Phe Ala Glu Val Asp Glu Lys Arg Ile
   165 170 175
   Gly Val Ile Gly Ala Ser Gln Gly Gly Gly Leu Ala Val Ala Val Ser
   180 185 190
   Ala Leu Ser Asp Ile Pro Lys Ala Ala Val Ser Glu Tyr Pro Tyr Leu
   195 200 205
   Ser Asn Phe Gln Arg Ala Ile Asp Thr Ala Ile Asp Gln Pro Tyr Leu
   210 215 220
   Glu Ile Asn Ser Phe Phe Arg Arg Asn Thr Ser Pro Asp Ile Glu Gln
   225 230 235 240
   Ala Ala Met His Thr Leu Ser Tyr Phe Asp Val Met Asn Leu Ala Gln
   245 250 255
   Leu Val Lys Ala Thr Val Leu Met Ser Ile Gly Leu Val Asp Thr Ile
   260 265 270
   Thr Pro Pro Ser Thr Val Phe Ala Ala Tyr Asn His Leu Glu Thr Asp
   275 280 285
   Lys Glu Ile Lys Val Tyr Arg Tyr Phe Gly His Glu Tyr Ile Pro Pro
   290 295 300
   Phe Gln Thr Glu Lys Leu Ala Phe Leu Arg Lys His Leu Lys
   305 310 315
<210> 11
   <211> 963
   <212> DNA
   <213> Bacillus pumilis
<400> 11
   atgcaattgt tcgatttatc actagaagag ctaaaaaaat ataaaccaaa gaaaacagca      60
   cgtcctgatt tctcagactt ttggaagaaa tcgctcgaag aactgcgcca agtggaggca     120
   gagccaacac ttgaatctta tgactatcca gtgaaaggcg tcaaggtgta ccgcctgacg     180
   tatcaaagct ttggacattc taaaattgaa ggcttttatg ctgtgcctga tcaaactggt     240
   ccgcatccag cgctcgttcg ttttcatggc tataatgcca gctatgacgg cggcattcac     300
   gacatcgtca actgggcgct gcacggctat gcaacatttg gtatgctcgt ccgcggtcaa     360
   ggtggcagtg aagacacatc agtgacacca ggcgggcatg cattagggtg gatgacaaaa     420
   ggcattttat cgaaagatac gtactattat cgaggcgttt atctagatgc tgttcgtgca     480
   cttgaagtca ttcagtcttt ccccgaagta gatgaacacc gtatcggcgt gatcggtgga     540
   agtcaggggg gtgcgttagc gattgcggcc gcagcccttt cagacattcc aaaagtcgtt     600
   gtggcagact atccttactt atcaaatttt gagcgtgcag ttgatgttgc cttggagcag     660
   ccttatttag aaatcaattc atactttcgc agaaacagtg atccgaaagt ggaggaaaag     720
   gcatttgaga cattaagcta ttttgattta atcaatttag ctggatgggt gaaacagcca     780
   acattgatgg cgatcggtct gattgacaaa ataaccccac catctactgt gtttgcggca     840
   tacaaccatt tagaaacaga taaagacctg aaagtatatc gctattttgg acacgagttt     900
   atccctgctt ttcaaacaga gaagctgtcc tttttacaaa agcatttgct tctatcaaca     960
   taa                                                                   963
<210> 12
   <211> 320
   <212> PRT
   <213> Bacillus pumilis
<400> 12
   Met Gln Leu Phe Asp Leu Ser Leu Glu Glu Leu Lys Lys Tyr Lys Pro
   1 5 10 15
   Lys Lys Thr Ala Arg Pro Asp Phe Ser Asp Phe Trp Lys Lys Ser Leu
   20 25 30
   Glu Glu Leu Arg Gln Val Glu Ala Glu Pro Thr Leu Glu Ser Tyr Asp
   35 40 45
   Tyr Pro Val Lys Gly Val Lys Val Tyr Arg Leu Thr Tyr Gln Ser Phe
   50 55 60
   Gly His Ser Lys Ile Glu Gly Phe Tyr Ala Val Pro Asp Gln Thr Gly
   65 70 75 80
   Pro His Pro Ala Leu Val Arg Phe His Gly Tyr Asn Ala Ser Tyr Asp
   85 90 95
   Gly Gly Ile His Asp Ile Val Asn Trp Ala Leu His Gly Tyr Ala Thr
   100 105 110
   Phe Gly Met Leu Val Arg Gly Gln Gly Gly Ser Glu Asp Thr Ser Val
   115 120 125
   Thr Pro Gly Gly His Ala Leu Gly Trp Met Thr Lys Gly Ile Leu Ser
   130 135 140
   Lys Asp Thr Tyr Tyr Tyr Arg Gly Val Tyr Leu Asp Ala Val Arg Ala
   145 150 155 160
   Leu Glu Val Ile Gln Ser Phe Pro Glu Val Asp Glu His Arg Ile Gly
   165 170 175
   Val Ile Gly Gly Ser Gln Gly Gly Ala Leu Ala Ile Ala Ala Ala Ala
   180 185 190
   Leu Ser Asp Ile Pro Lys Val Val Val Ala Asp Tyr Pro Tyr Leu Ser
   195 200 205
   Asn Phe Glu Arg Ala Val Asp Val Ala Leu Glu Gln Pro Tyr Leu Glu
   210 215 220
   Ile Asn Ser Tyr Phe Arg Arg Asn Ser Asp Pro Lys Val Glu Glu Lys
   225 230 235 240
   Ala Phe Glu Thr Leu Ser Tyr Phe Asp Leu Ile Asn Leu Ala Gly Trp
   245 250 255
   Val Lys Gln Pro Thr Leu Met Ala Ile Gly Leu Ile Asp Lys Ile Thr
   260 265 270
   Pro Pro Ser Thr Val Phe Ala Ala Tyr Asn His Leu Glu Thr Asp Lys
   275 280 285
   Asp Leu Lys Val Tyr Arg Tyr Phe Gly His Glu Phe Ile Pro Ala Phe
   290 295 300
   Gln Thr Glu Lys Leu Ser Phe Leu Gln Lys His Leu Leu Leu Ser Thr
   305 310 315 320
<210> 13
   <211> 963
   <212> DNA
   <213> Clostridium thermocellum ATCC 27405
<400> 13
   atggcacaat tatatgatat gcctttggag gaattaaaaa aatataagcc tgcgcttaca 60
   aaacagaaag attttgatga gttttgggaa aaaagcctta aagagctggc tgaaattcct 120
   ttaaaatatc aacttatacc ttatgatttt ccggcccgga gggtaaaagt tttcagagtt 180
   gaatatcttg gttttaaagg tgcaaatatt gaagggtggc ttgccgttcc cgagggagaa 240
   gggttgtatc ccgggcttgt acagtttcac ggatacaact gggcgatgga tggatgtgtt 300
   cccgatgtgg taaattgggc tttgaatgga tatgccgcat ttcttatgct tgttcgggga 360
   cagcagggaa gaagcgtgga caatattgtg cccggcagcg gtcatgcttt gggatggatg 420
   tcgaaaggta ttttgtcacc ggaggaatat tattatagag gagtatatat ggatgcggtt 480
   cgtgctgttg aaattttggc ttcgcttcct tgtgtggatg aatcgagaat aggagtgaca 540
   gggggcagcc agggtggagg acttgcactg gcggtggctg ctctgtccgg cataccgaaa 600
   gttgcagccg tgcattatcc gtttctggca cattttgagc gtgccattga cgttgcgccg 660
   gacggccctt atcttgaaat taacgaatat ttaagaagaa acagcggtga agaaatagaa 720
   agacaggtaa agaaaaccct ttcctatttt gatatcatga atcttgctcc ccgtataaaa 780
   tgccgtactt ggatttgcac tggtcttgtg gatgagatta ctcctccgtc aacggttttt 840
   gcagtgtaca atcacctcaa atgcccaaag gaaatttcgg tattcagata ttttgggcat 900
   gaacatatgc caggaagcgt tgaaatcaag ctgaggatac ttatggatga gctgaatccg 960
   taa 963
<210> 14
   <211> 320
   <212> PRT
   <213> Clostridium thermocellum ATCC 27405
<400> 14
   Met Ala Gln Leu Tyr Asp Met Pro Leu Glu Glu Leu Lys Lys Tyr Lys
   1               5                   10                  15
   Pro Ala Leu Thr Lys Gln Lys Asp Phe Asp Glu Phe Trp Glu Lys Ser
               20                  25                  30
   Leu Lys Glu Leu Ala Glu Ile Pro Leu Lys Tyr Gln Leu Ile Pro Tyr
           35                  40                  45
   Asp Phe Pro Ala Arg Arg Val Lys Val Phe Arg Val Glu Tyr Leu Gly
       50                  55                  60
   Phe Lys Gly Ala Asn Ile Glu Gly Trp Leu Ala Val Pro Glu Gly Glu
   65                  70                  75                  80
   Gly Leu Tyr Pro Gly Leu Val Gln Phe His Gly Tyr Asn Trp Ala Met
                   85                  90                  95
   Asp Gly Cys Val Pro Asp Val Val Asn Trp Ala Leu Asn Gly Tyr Ala
               100                 105                 110
   Ala Phe Leu Met Leu Val Arg Gly Gln Gln Gly Arg Ser Val Asp Asn
           115                 120                 125
   Ile Val Pro Gly Ser Gly His Ala Leu Gly Trp Met Ser Lys Gly Ile
       130                 135                 140
   Leu Ser Pro Glu Glu Tyr Tyr Tyr Arg Gly Val Tyr Met Asp Ala Val
   145                 150                 155                 160
   Arg Ala Val Glu Ile Leu Ala Ser Leu Pro Cys Val Asp Glu Ser Arg
                   165                 170                 175
   Ile Gly Val Thr Gly Gly Ser Gln Gly Gly Gly Leu Ala Leu Ala Val
               180                 185                 190
   Ala Ala Leu Ser Gly Ile Pro Lys Val Ala Ala Val His Tyr Pro Phe
           195                 200                 205
   Leu Ala His Phe Glu Arg Ala Ile Asp Val Ala Pro Asp Gly Pro Tyr
       210                 215                 220
   Leu Glu Ile Asn Glu Tyr Leu Arg Arg Asn Ser Gly Glu Glu Ile Glu
   225                 230                 235                 240
   Arg Gln Val Lys Lys Thr Leu Ser Tyr Phe Asp Ile Met Asn Leu Ala
   245 250 255
   Pro Arg Ile Lys Cys Arg Thr Trp Ile Cys Thr Gly Leu Val Asp Glu
   260 265 270
   Ile Thr Pro Pro Ser Thr Val Phe Ala Val Tyr Asn His Leu Lys Cys
   275 280 285
   Pro Lys Glu Ile Ser Val Phe Arg Tyr Phe Gly His Glu His Met Pro
   290 295 300
   Gly Ser Val Glu Ile Lys Leu Arg Ile Leu Met Asp Glu Leu Asn Pro
   305 310 315 320
<210> 15
   <211> 978
   <212> DNA
   <213> Thermotoga neapolitana
<400> 15
   atggccttct tcgatatgcc ccttgaggaa ctgaaaaagt accggcctga aaggtacgag 60
   gagaaagatt tcgatgagtt ctggagggaa acacttaaag aaagcgaagg attccctctg 120
   gatcccgtct ttgaaaaggt ggactttcat ctcaaaacgg ttgaaacgta cgatgttact 180
   ttctctggat acagggggca gagaataaag ggctggcttc ttgttccgaa gttggcggaa 240
   gaaaagcttc catgcgtcgt gcagtacata ggttacaatg gtggaagggg ttttccacac 300
   gactggctgt tctggccgtc aatgggttac atctgttttg tcatggacac cagggggcag 360
   ggaagcggct ggatgaaggg agacacaccg gattaccctg agggtccagt cgatccacag 420
   taccccggat tcatgacgag gggcattctg gatccgggaa cctattacta caggcgagtc 480
   ttcgtggatg cggtcagggc ggtggaagca gccatttcct tcccgagagt ggattccagg 540
   aaggtggtgg tggccggagg cagtcagggt gggggaatcg cccttgcggt gagtgccctg 600
   tcgaacaggg tgaaggctct gctctgcgat gtgccgtttc tgtgccactt cagaagggcc 660
   gtgcaacttg tcgacacaca cccatacgtg gagatcacca acttcctcaa aacccacagg 720
   gacaaagagg agattgtttt cagaacactt tcctacttcg atggtgtgaa ctttgcagca 780
   agggcaaagg tgcccgccct gttttccgtt gggctcatgg acaccatctg tcctccctcg 840
   acggtcttcg ccgcttacaa ccactacgcc ggtccaaagg agatcagaat ctatccgtac 900
   aacaaccacg aaggtggagg ttctttccag gcaattgagc aggtgaaatt cttgaagaga 960
   ctatttgagg aaggctag 978
<210> 16
   <211> 325
   <212> PRT
   <213> Thermotoga neapolitana
<400> 16
   Met Ala Phe Phe Asp Met Pro Leu Glu Glu Leu Lys Lys Tyr Arg Pro
   1 5 10 15
   Glu Arg Tyr Glu Glu Lys Asp Phe Asp Glu Phe Trp Arg Glu Thr Leu
   20 25 30
   Lys Glu Ser Glu Gly Phe Pro Leu Asp Pro Val Phe Glu Lys Val Asp
   35 40 45
   Phe His Leu Lys Thr Val Glu Thr Tyr Asp Val Thr Phe Ser Gly Tyr
   50 55 60
   Arg Gly Gln Arg Ile Lys Gly Trp Leu Leu Val Pro Lys Leu Ala Glu
   65 70 75 80
   Glu Lys Leu Pro Cys Val Val Gln Tyr Ile Gly Tyr Asn Gly Gly Arg
   85 90 95
   Gly Phe Pro His Asp Trp Leu Phe Trp Pro Ser Met Gly Tyr Ile Cys
   100 105 110
   Phe Val Met Asp Thr Arg Gly Gln Gly Ser Gly Trp Met Lys Gly Asp
   115 120 125
   Thr Pro Asp Tyr Pro Glu Gly Pro Val Asp Pro Gln Tyr Pro Gly Phe
   130 135 140
   Met Thr Arg Gly Ile Leu Asp Pro Gly Thr Tyr Tyr Tyr Arg Arg Val
   145 150 155 160
   Phe Val Asp Ala Val Arg Ala Val Glu Ala Ala Ile Ser Phe Pro Arg
   165 170 175
   Val Asp Ser Arg Lys Val Val Val Ala Gly Gly Ser Gln Gly Gly Gly
   180 185 190
   Ile Ala Leu Ala Val Ser Ala Leu Ser Asn Arg Val Lys Ala Leu Leu
   195 200 205
   Cys Asp Val Pro Phe Leu Cys His Phe Arg Arg Ala Val Gln Leu Val
   210 215 220
   Asp Thr His Pro Tyr Val Glu Ile Thr Asn Phe Leu Lys Thr His Arg
   225 230 235 240
   Asp Lys Glu Glu Ile Val Phe Arg Thr Leu Ser Tyr Phe Asp Gly Val
                   245                 250                 255
   Asn Phe Ala Ala Arg Ala Lys Val Pro Ala Leu Phe Ser Val Gly Leu
               260                 265                 270
   Met Asp Thr Ile Cys Pro Pro Ser Thr Val Phe Ala Ala Tyr Asn His
           275                 280                 285
   Tyr Ala Gly Pro Lys Glu Ile Arg Ile Tyr Pro Tyr Asn Asn His Glu
       290                 295                 300
   Gly Gly Gly Ser Phe Gln Ala Ile Glu Gln Val Lys Phe Leu Lys Arg
   305                 310                 315                 320
   Leu Phe Glu Glu Gly
                   325
<210> 17
   <211> 978
   <212> DNA
   <213> Thermotoga maritima MSB8
<400> 17
   atggccttct tcgatttacc actcgaagaa ctgaagaaat atcgtccaga gcggtacgaa      60
   gagaaagact tcgatgagtt ctgggaagag acactcgcag agagcgaaaa gttcccctta     120
   gaccccgtct tcgagaggat ggagtctcac ctcaaaacag tcgaagcgta cgatgtcacc     180
   ttctccggat acaggggaca gaggatcaaa gggtggctcc ttgttccaaa actggaagaa     240
   gaaaaacttc cctgcgttgt gcagtacata ggatacaacg gtggaagagg attccctcac     300
   gactggctgt tctggccttc tatgggttac atatgtttcg tcatggatac tcgaggtcag     360
   ggaagcggct ggctgaaagg agacacaccg gattaccctg agggtcccgt tgaccctcag     420
   tatccaggat tcatgacaag aggaatactg gatcccagaa cttactacta cagacgagtc     480
   ttcacggacg ctgtcagagc cgttgaagct gctgcttctt ttcctcaggt agatcaagaa     540
   agaatcgtga tagctggagg cagtcagggt ggcggaatag cccttgcggt gagcgctctc     600
   tcaaagaaag caaaggctct tctgtgcgat gtgccgtttc tgtgtcactt cagaagagca     660
   gtacagcttg tggatacgca tccatacgcg gagatcacga actttctaaa gacccacaga     720
   gacaaggaag aaatcgtgtt caggactctt tcctatttcg atggagtgaa cttcgcagcc     780
   agagcgaaga tccctgcgct gttttctgtg ggtctcatgg acaacatttg tcctccttca     840
   acggttttcg ctgcctacaa ttactacgct ggaccgaagg aaatcagaat ctatccgtac     900
   aacaaccacg agggaggagg ctctttccaa gcggttgaac aggtgaaatt cttgaaaaaa     960
   ctatttgaga aaggctaa                                                   978
<210> 18
   <211> 325
   <212> PRT
   <213> Thermotoga maritima MSB8
<400> 18
   Met Ala Phe Phe Asp Leu Pro Leu Glu Glu Leu Lys Lys Tyr Arg Pro
   1 5 10 15
   Glu Arg Tyr Glu Glu Lys Asp Phe Asp Glu Phe Trp Glu Glu Thr Leu
   20 25 30
   Ala Glu Ser Glu Lys Phe Pro Leu Asp Pro Val Phe Glu Arg Met Glu
   35 40 45
   Ser His Leu Lys Thr Val Glu Ala Tyr Asp Val Thr Phe Ser Gly Tyr
   50 55 60
   Arg Gly Gln Arg Ile Lys Gly Trp Leu Leu Val Pro Lys Leu Glu Glu
   65 70 75 80
   Glu Lys Leu Pro Cys Val Val Gln Tyr Ile Gly Tyr Asn Gly Gly Arg
   85 90 95
   Gly Phe Pro His Asp Trp Leu Phe Trp Pro Ser Met Gly Tyr Ile Cys
   100 105 110
   Phe Val Met Asp Thr Arg Gly Gln Gly Ser Gly Trp Leu Lys Gly Asp
   115 120 125
   Thr Pro Asp Tyr Pro Glu Gly Pro Val Asp Pro Gln Tyr Pro Gly Phe
   130 135 140
   Met Thr Arg Gly Ile Leu Asp Pro Arg Thr Tyr Tyr Tyr Arg Arg Val
   145 150 155 160
   Phe Thr Asp Ala Val Arg Ala Val Glu Ala Ala Ala Ser Phe Pro Gln
   165 170 175
   Val Asp Gln Glu Arg Ile Val Ile Ala Gly Gly Ser Gln Gly Gly Gly
   180 185 190
   Ile Ala Leu Ala Val Ser Ala Leu Ser Lys Lys Ala Lys Ala Leu Leu
   195 200 205
   Cys Asp Val Pro Phe Leu Cys His Phe Arg Arg Ala Val Gln Leu Val
   210 215 220
   Asp Thr His Pro Tyr Ala Glu Ile Thr Asn Phe Leu Lys Thr His Arg
   225 230 235 240
   Asp Lys Glu Glu Ile Val Phe Arg Thr Leu Ser Tyr Phe Asp Gly Val
   245 250 255
   Asn Phe Ala Ala Arg Ala Lys Ile Pro Ala Leu Phe Ser Val Gly Leu
   260 265 270
   Met Asp Asn Ile Cys Pro Pro Ser Thr Val Phe Ala Ala Tyr Asn Tyr
   275 280 285
   Tyr Ala Gly Pro Lys Glu Ile Arg Ile Tyr Pro Tyr Asn Asn His Glu
   290 295 300
   Gly Gly Gly Ser Phe Gln Ala Val Glu Gln Val Lys Phe Leu Lys Lys
   305 310 315 320
   Leu Phe Glu Lys Gly
   325
<210> 19
   <211> 963
   <212> DNA
   <213> Thermoanaerobacterium sp.
<400> 19
   atgggacttt tcgacatgcc attacaaaaa cttagagaat acactggtac aaatccatgc 60
   cctgaagatt tcgatgagta ttggaatagg gctttagatg agatgaggtc agttgatcct 120
   aaaattgaat tgaaagaaag tagctttcaa gtatcctttg cagaatgcta tgacttgtac 180
   tttacaggtg ttcgtggtgc cagaattcat gcaaagtata taaaacctaa gacagaaggg 240
   aaacatccag cgttgataag atttcatgga tattcgtcaa attcaggcga ctggaacgac 300
   aaattaaatt acgtggcggc aggcttcacc gttgtggcta tggatgtaag aggtcaagga 360
   gggcagtctc aagatgttgg cggtgtaact gggaatactt taaatgggca tattataaga 420
   gggctagacg atgatgctga taatatgctt ttcaggcata ttttcttaga cactgcccaa 480
   ttggctggaa tagttatgaa catgccagaa gttgatgaag atagagtggg agtcatggga 540
   ccttctcaag gcggagggct gtcgttggcg tgtgctgcat tggagccaag ggtacgcaaa 600
   gtagtatctg aatatccttt tttatctgac tacaagagag tttgggactt agaccttgca 660
   aaaaacgcct atcaagagat tacggactat ttcaggcttt ttgacccaag gcatgaaagg 720
   gagaatgagg tatttacaaa gcttggatat atagacgtta aaaaccttgc gaaaaggata 780
   aaaggcgatg tcttaatgtg cgttgggctt atggaccaag tatgtccgcc atcaactgtt 840
   tttgcagcct acaacaacat acagtcaaaa aaagatataa aagtgtatcc tgattatgga 900
   catgaaccta tgagaggatt tggagattta gcgatgcagt ttatgttgga actatattca 960
   taa 963
<210> 20
   <211> 320
   <212> PRT
   <213> Thermoanaerobacterium sp.
<400> 20
   Met Gly Leu Phe Asp Met Pro Leu Gln Lys Leu Arg Glu Tyr Thr Gly
   1               5                   10                  15
   Thr Asn Pro Cys Pro Glu Asp Phe Asp Glu Tyr Trp Asn Arg Ala Leu
               20                  25                  30
   Asp Glu Met Arg Ser Val Asp Pro Lys Ile Glu Leu Lys Glu Ser Ser
           35                  40                  45
   Phe Gln Val Ser Phe Ala Glu Cys Tyr Asp Leu Tyr Phe Thr Gly Val
       50                  55                  60
   Arg Gly Ala Arg Ile His Ala Lys Tyr Ile Lys Pro Lys Thr Glu Gly
   65                  70                  75                  80
   Lys His Pro Ala Leu Ile Arg Phe His Gly Tyr Ser Ser Asn Ser Gly
                   85                  90                  95
   Asp Trp Asn Asp Lys Leu Asn Tyr Val Ala Ala Gly Phe Thr Val Val
               100                 105                 110
   Ala Met Asp Val Arg Gly Gln Gly Gly Gln Ser Gln Asp Val Gly Gly
           115                 120                 125
   Val Thr Gly Asn Thr Leu Asn Gly His Ile Ile Arg Gly Leu Asp Asp
       130                 135                 140
   Asp Ala Asp Asn Met Leu Phe Arg His Ile Phe Leu Asp Thr Ala Gln
   145                 150                 155                 160
   Leu Ala Gly Ile Val Met Asn Met Pro Glu Val Asp Glu Asp Arg Val
                   165                 170                 175
   Gly Val Met Gly Pro Ser Gln Gly Gly Gly Leu Ser Leu Ala Cys Ala
               180                 185                 190
   Ala Leu Glu Pro Arg Val Arg Lys Val Val Ser Glu Tyr Pro Phe Leu
           195                 200                 205
   Ser Asp Tyr Lys Arg Val Trp Asp Leu Asp Leu Ala Lys Asn Ala Tyr
       210                 215                 220
   Gln Glu Ile Thr Asp Tyr Phe Arg Leu Phe Asp Pro Arg His Glu Arg
   225                 230                 235                 240
   Glu Asn Glu Val Phe Thr Lys Leu Gly Tyr Ile Asp Val Lys Asn Leu
                   245                 250                 255
   Ala Lys Arg Ile Lys Gly Asp Val Leu Met Cys Val Gly Leu Met Asp
               260                 265                 270
   Gln Val Cys Pro Pro Ser Thr Val Phe Ala Ala Tyr Asn Asn Ile Gln
           275                 280                 285
   Ser Lys Lys Asp Ile Lys Val Tyr Pro Asp Tyr Gly His Glu Pro Met
       290                 295                 300
   Arg Gly Phe Gly Asp Leu Ala Met Gln Phe Met Leu Glu Leu Tyr Ser
   305                 310                 315                 320
<210> 21
   <211> 1023
   <212> DNA
   <213> Bacillus sp. NRRL B-14911
<400> 21
   atgaggacgg ttcctgctcc tgtttttttg gagaggagtg gggagatgaa cctttttgat 60
   atgccccttg aggagctgca gcattacaag cctgcccaga ccaggcagga tgattttgag 120
   tcattctgga aaaagcggat tgaggagaac agtcaatatc cgctgaatat agaagtaatg 180
   gagcgggttt atccggttcc gggagtgaga gtatatgata tttattttga cgggttccgg 240
   aattcccgca tccatggggt gtatgttact ccagaaactc cgggagcgga cactcctgcg 300
   gcagtgattt ttcacggcta taactggaac acgctgcagc cgcattacag cttcaagcac 360
   gtgattcagg ggattcctgt actgatggtg gaggtgcggg gacaaaatct cttgtctcca 420
   gatagaaatc attatgggaa tggaggtccg ggaggctgga tgacactcgg cgtgatggat 480
   cccgatcaat attattacag cctggtatat atggactgct tccgcagcat tgatgctgtc 540
   agggaactgt cgaggaagag aagtgtgttt gtggaaggcg gaagccaggg aggtgcactg 600
   gcgattgccg cagccgccct gcaggatgac atcctgcttg cactcgccga catccctttt 660
   ctcacccatt tcaagcgttc cgtggagctt tcctcggatg gaccgtatca ggagatttcc 720
   cactacttca aagttcatga tcctcttcat caaacggaag agcaggtata tcagacgctc 780
   agctatgtgg actgcatgaa catggccagc atggttgaat gtccagtcct tctttcagcc 840
   ggtctggaag acatcgtttg tcccccgtcc agtgcatttg cactgttcaa ccatctcggc 900
   gggccaaaag aaatacgggc ctatccggaa tacgcccatg aagtaccggc tgtccatgaa 960
   gaggaaaagc tgaagtttat atcttcaagg ctaaaaaata gagaaaagag gtgccggcca 1020
   tga 1023
<210> 22
   <211> 340
   <212> PRT
   <213> Bacillus sp. NRRL B-14911
<400> 22
   Met Arg Thr Val Pro Ala Pro Val Phe Leu Glu Arg Ser Gly Glu Met
   1               5                   10                  15
   Asn Leu Phe Asp Met Pro Leu Glu Glu Leu Gln His Tyr Lys Pro Ala
               20                  25                  30
   Gln Thr Arg Gln Asp Asp Phe Glu Ser Phe Trp Lys Lys Arg Ile Glu
           35                  40                  45
   Glu Asn Ser Gln Tyr Pro Leu Asn Ile Glu Val Met Glu Arg Val Tyr
       50                  55                  60
   Pro Val Pro Gly Val Arg Val Tyr Asp Ile Tyr Phe Asp Gly Phe Arg
   65                  70                  75                  80
   Asn Ser Arg Ile His Gly Val Tyr Val Thr Pro Glu Thr Pro Gly Ala
                   85                  90                  95
   Asp Thr Pro Ala Ala Val Ile Phe His Gly Tyr Asn Trp Asn Thr Leu
               100                 105                 110
   Gln Pro His Tyr Ser Phe Lys His Val Ile Gln Gly Ile Pro Val Leu
           115                 120                 125
   Met Val Glu Val Arg Gly Gln Asn Leu Leu Ser Pro Asp Arg Asn His
       130                 135                 140
   Tyr Gly Asn Gly Gly Pro Gly Gly Trp Met Thr Leu Gly Val Met Asp
   145                 150                 155                 160
   Pro Asp Gln Tyr Tyr Tyr Ser Leu Val Tyr Met Asp Cys Phe Arg Ser
                   165                 170                 175
   Ile Asp Ala Val Arg Glu Leu Ser Arg Lys Arg Ser Val Phe Val Glu
               180                 185                 190
   Gly Gly Ser Gln Gly Gly Ala Leu Ala Ile Ala Ala Ala Ala Leu Gln
           195                 200                 205
   Asp Asp Ile Leu Leu Ala Leu Ala Asp Ile Pro Phe Leu Thr His Phe
       210                 215                 220
   Lys Arg Ser Val Glu Leu Ser Ser Asp Gly Pro Tyr Gln Glu Ile Ser
   225 230 235 240
   His Tyr Phe Lys Val His Asp Pro Leu His Gln Thr Glu Glu Gln Val
   245 250 255
   Tyr Gln Thr Leu Ser Tyr Val Asp Cys Met Asn Met Ala Ser Met Val
   260 265 270
   Glu Cys Pro Val Leu Leu Ser Ala Gly Leu Glu Asp Ile Val Cys Pro
   275 280 285
   Pro Ser Ser Ala Phe Ala Leu Phe Asn His Leu Gly Gly Pro Lys Glu
   290 295 300
   Ile Arg Ala Tyr Pro Glu Tyr Ala His Glu Val Pro Ala Val His Glu
   305 310 315 320
   Glu Glu Lys Leu Lys Phe Ile Ser Ser Arg Leu Lys Asn Arg Glu Lys
   325 330 335
   Arg Cys Arg Pro
   340
<210> 23
   <211> 960
   <212> DNA
   <213> Bacillus halodurans C-125
<400> 23
   ttagagatca gataaaaatt gaaaaatccg atcacgatgg cctggcaaat cttcgtgagc      60
   aaagtctgga tataactcga tactttttgt cgtcgtgagt ttgttataca tggcaaattg     120
   tgtagacggc gggcaaaccg tatccattaa cccaacagca agtaagactt ctccctttac     180
   gagtggagca agatgctgaa tatcaatata gcctagcttc gtaaagattt cagcctcacg     240
   tcggtgctgt ggatcaaagc gacgaaaata cgtttgcaat tcgtcataag ctttctcggc     300
   taaatccatc tcccatacgc gttggtaatc gctaaggaaa ggataaacag gagctacctt     360
   tttaattttc ggttccaaag ccgcacaagc aatcgctaag gcccctcctt gtgaccaacc     420
   tgtcactgcc acgcgctctt catcgacttc aggaaggttc atcacaatgt tggcaagctg     480
   agccgtatca agaaacacat gacggaacaa taattgatca gcattatcat cgagtccgcg     540
   tattatatga ccggaatgag tattcccctt cacgcctcct gtgtcttcag acaagcctcc     600
   ttgcccgcga acgtccattg caagaacaga atatccgagg gctgcgtaat gaagtaaacc     660
   cgtccattcc cccgcattca tcgtatatcc gtgaaaatga ataaccgccg ggtgtgtccc     720
   gctcgtgtgt cttgggcgca cgtattttgc gtgaattcta gcacccctaa cccctgtaaa     780
   atataggtgg aagcattctg catacgtggt ttgaaaatca ctcggtatga gctctacgtt     840
   tggatttacc tttctcatct cttgtaaagc acgatcccaa tactcagtaa agtcatctgg     900
   ctttggatta cgtcccatgt actcttttaa ttcggttaac ggcatgtcta ttagtggcat     960
<210> 24
   <211> 319
   <212> PRT
   <213> Bacillus halodurans C-125
<400> 24
   Met Pro Leu Ile Asp Met Pro Leu Thr Glu Leu Lys Glu Tyr Met Gly
   1               5                   10                  15
   Arg Asn Pro Lys Pro Asp Asp Phe Thr Glu Tyr Trp Asp Arg Ala Leu
               20                  25                  30
   Gln Glu Met Arg Lys Val Asn Pro Asn Val Glu Leu Ile Pro Ser Asp
           35                  40                  45
   Phe Gln Thr Thr Tyr Ala Glu Cys Phe His Leu Tyr Phe Thr Gly Val
       50                  55                  60
   Arg Gly Ala Arg Ile His Ala Lys Tyr Val Arg Pro Arg His Thr Ser
   65                  70                  75                  80
   Gly Thr His Pro Ala Val Ile His Phe His Gly Tyr Thr Met Asn Ala
                   85                  90                  95
   Gly Glu Trp Thr Gly Leu Leu His Tyr Ala Ala Leu Gly Tyr Ser Val
               100                 105                 110
   Leu Ala Met Asp Val Arg Gly Gln Gly Gly Leu Ser Glu Asp Thr Gly
           115                 120                 125
   Gly Val Lys Gly Asn Thr His Ser Gly His Ile Ile Arg Gly Leu Asp
       130                 135                 140
   Asp Asn Ala Asp Gln Leu Leu Phe Arg His Val Phe Leu Asp Thr Ala
   145                 150                 155                 160
   Gln Leu Ala Asn Ile Val Met Asn Leu Pro Glu Val Asp Glu Glu Arg
                   165                 170                 175
   Val Ala Val Thr Gly Trp Ser Gln Gly Gly Ala Leu Ala Ile Ala Cys
               180                 185                 190
   Ala Ala Leu Glu Pro Lys Ile Lys Lys Val Ala Pro Val Tyr Pro Phe
           195                 200                 205
   Leu Ser Asp Tyr Gln Arg Val Trp Glu Met Asp Leu Ala Glu Lys Ala
   210 215 220
   Tyr Asp Glu Leu Gln Thr Tyr Phe Arg Arg Phe Asp Pro Gln His Arg
   225 230 235 240
   Arg Glu Ala Glu Ile Phe Thr Lys Leu Gly Tyr Ile Asp Ile Gln His
   245 250 255
   Leu Ala Pro Leu Val Lys Gly Glu Val Leu Leu Ala Val Gly Leu Met
   260 265 270
   Asp Thr Val Cys Pro Pro Ser Thr Gln Phe Ala Met Tyr Asn Lys Leu
   275 280 285
   Thr Thr Thr Lys Ser Ile Glu Leu Tyr Pro Asp Phe Ala His Glu Asp
   290 295 300
   Leu Pro Gly His Arg Asp Arg Ile Phe Gln Phe Leu Ser Asp Leu
   305 310 315
<210> 25
   <211> 954
   <212> DNA
   <213> Bacillus clausii KSM-K16
<400> 25
   atgccattag tcgatatgcc gttgcgcgag ttgttagctt atgaaggaat aaaccctaaa 60
   ccagcagatt ttgaccaata ctggaaccgg gccaaaacgg aaattgaagc gattgatccc 120
   gaagtcactc tagtcgaatc ttctttccag tgttcgtttg caaactgtta ccatttctat 180
   tatcgaagcg ctggaaatgc aaaaatccat gcgaaatacg tacagccaaa agcaggggag 240
   aagacgccag cagtttttat gttccatggg tatggggggc gttcagccga atggagcagc 300
   ttgttaaatt atgtagcggc gggtttttct gttttctata tggacgtgcg tggacaaggt 360
   ggaacttcag aggatcctgg gggcgtaagg gggaatacat ataggggcca cattattcgc 420
   ggcctcgatg ccgggccaga cgcacttttt taccgcagcg ttttcttgga caccgtccaa 480
   ttggttcgtg ctgctaaaac attgcctcac atcgataaaa cacggcttat ggccacaggg 540
   tggtcgcaag ggggcgcctt aacgcttgcc tgtgctgccc ttgttcctga aatcaagcgt 600
   cttgctccag tatacccgtt tttaagcgat tacaagcgag tgtggcaaat ggatttagcg 660
   gttcgttcgt ataaagaatt ggctgattat ttccgttcat acgatccgca acataaacgc 720
   catggcgaaa tttttgaacg ccttggctac atcgatgtcc agcatcttgc tgaccggatt 780
   caaggagatg tcctaatggg agttggttta atggatacag aatgcccgcc gtctacccaa 840
   tttgctgctt ataataaaat aaaggctaaa aaatcgtatg agctctatcc tgattttggc 900
   catgagcacc ttccaggaat gaacgatcat atttttcgct ttttcactag ttga 954
<210> 26
   <211> 317
   <212> PRT
   <213> Bacillus clausii KSM-K16
<400> 26
   Met Pro Leu Val Asp Met Pro Leu Arg Glu Leu Leu Ala Tyr Glu Gly
   1               5                   10                  15
   Ile Asn Pro Lys Pro Ala Asp Phe Asp Gln Tyr Trp Asn Arg Ala Lys
               20                  25                  30
   Thr Glu Ile Glu Ala Ile Asp Pro Glu Val Thr Leu Val Glu Ser Ser
           35                  40                  45
   Phe Gln Cys Ser Phe Ala Asn Cys Tyr His Phe Tyr Tyr Arg Ser Ala
       50                  55                  60
   Gly Asn Ala Lys Ile His Ala Lys Tyr Val Gln Pro Lys Ala Gly Glu
   65                  70                  75                  80
   Lys Thr Pro Ala Val Phe Met Phe His Gly Tyr Gly Gly Arg Ser Ala
                   85                  90                  95
   Glu Trp Ser Ser Leu Leu Asn Tyr Val Ala Ala Gly Phe Ser Val Phe
               100                 105                 110
   Tyr Met Asp Val Arg Gly Gln Gly Gly Thr Ser Glu Asp Pro Gly Gly
           115                 120                 125
   Val Arg Gly Asn Thr Tyr Arg Gly His Ile Ile Arg Gly Leu Asp Ala
       130                 135                 140
   Gly Pro Asp Ala Leu Phe Tyr Arg Ser Val Phe Leu Asp Thr Val Gln
   145                 150                 155                 160
   Leu Val Arg Ala Ala Lys Thr Leu Pro His Ile Asp Lys Thr Arg Leu
                   165                 170                 175
   Met Ala Thr Gly Trp Ser Gln Gly Gly Ala Leu Thr Leu Ala Cys Ala
               180                 185                 190
   Ala Leu Val Pro Glu Ile Lys Arg Leu Ala Pro Val Tyr Pro Phe Leu
           195                 200                 205
   Ser Asp Tyr Lys Arg Val Trp Gln Met Asp Leu Ala Val Arg Ser Tyr
       210                 215                 220
   Lys Glu Leu Ala Asp Tyr Phe Arg Ser Tyr Asp Pro Gln His Lys Arg
   225 230 235 240
   His Gly Glu Ile Phe Glu Arg Leu Gly Tyr Ile Asp Val Gln His Leu
   245 250 255
   Ala Asp Arg Ile Gln Gly Asp Val Leu Met Gly Val Gly Leu Met Asp
   260 265 270
   Thr Glu Cys Pro Pro Ser Thr Gln Phe Ala Ala Tyr Asn Lys Ile Lys
   275 280 285
   Ala Lys Lys Ser Tyr Glu Leu Tyr Pro Asp Phe Gly His Glu His Leu
   290 295 300
   Pro Gly Met Asn Asp His Ile Phe Arg Phe Phe Thr Ser
   305 310 315
<210> 27
   <211> 49
   <212> DNA
   <213> artificial sequence
<220>
   <223> Primer
<400> 27
   taactgcagt aaggaggaat aggacatgca actattcgat ctgccgctc 49
<210> 28
   <211> 35
   <212> DNA
   <213> artificial sequence
<220>
   <223> Primer
<400> 28
   tgatctagat tatcagcctt taagatgctg cttaa 35
<210> 29
   <211> 994
   <212> DNA
   <213> artificial sequence
<220>
   <223> Synthetic construct
<400> 29
   taactgcagt aaggaggaat aggacatgca actattcgat ctgccgctcg accaattgca 60
   aacatataag cctgaaaaaa cagcaccgaa agatttttct gagttttgga aattgtcttt 120
   ggaggaactt gcaaaagtcc aagcagaacc tgatttacag ccggttgact atcctgctga 180
   cggagtaaaa gtgtaccgtc tcacatataa aagcttcgga aacgcccgca ttaccggatg 240
   gtacgcggtg cctgacaagc aaggcccgca tccggcgatc gtgaaatatc atggctacaa 300
   tgcaagctat gatggtgaga ttcatgaaat ggtaaactgg gcactccatg gctacgccgc 360
   attcggcatg cttgtccgcg gccagcagag cagcgaggat acgagtattt cactgcacgg 420
   tcacgctttg ggctggatga cgaaaggaat tcttgataaa gatacatact attaccgcgg 480
   tgtttatttg gacgccgtcc gcgcgcttga ggtcatcagc agcttcgacg aggttgacga 540
   aacaaggatc ggtgtgacag gaggaagcca aggcggaggt ttaaccattg ccgcagcagc 600
   gctgtcagac attccaaaag ccgcggttgc cgattatcct tatttaagca acttcgaacg 660
   ggccattgat gtggcgcttg aacagccgta ccttgaaatc aattccttct tcagaagaaa 720
   tggcagcccg gaaacagaag tgcaggcgat gaagacactt tcatatttcg atattatgaa 780
   tctcgctgac cgagtgaagg tgcctgtcct gatgtcaatc ggcctgattg acaaggtcac 840
   gccgccgtcc accgtgtttg ccgcctacaa tcatttggaa acagagaaag agctgaaggt 900
   gtaccgctac ttcggacatg agtatatccc tgcttttcaa acggaaaaac ttgctttctt 960
   taagcagcat cttaaaggct gataatctag atca 994
<210> 30
   <211> 994
   <212> DNA
   <213> artificial sequence
<220>
   <223> Synthetic construct
<400> 30
   taactgcagt aaggaggaat aggacatgca actattcgat ctgccgctcg accaattgca      60
   aacatataag cctgaaaaaa cagcaccgaa agatttttct gagttttgga aattgtcttt     120
   ggaggaactt gcaaaagtcc aagcagaacc tgatttacag ccggttgact atcctgctga     180
   cggagtaaaa gtgtaccgtc tcacatataa aagcttcgga aacgcccgca ttaccggatg     240
   gtacgcggtg cctgacaagg aaggcccgca tccggcgatc gtgaaatatc atggctacaa     300
   tgcaagctat gatggtgaga ttcatgaaat ggtaaactgg gcactccatg gctacgccac     360
   attcggcatg cttgtccgcg gccagcagag cagcgaggat acgagtattt caccgcacgg     420
   tcacgctttg ggctggatga cgaaaggaat tcttgataaa gatacatact attaccgcgg     480
   tgtttatttg gacgccgtcc gcgcgcttga ggtcatcagc agcttcgacg aggttgacga     540
   aacaaggatc ggtgtgacag gaggaagcca aggcggaggt ttaaccattg ccgcagcagc     600
   gctgtcagac attccaaaag ccgcggttgc cgattatcct tatttaagca acttcgaacg     660
   ggccattgat gtggcgcttg aacagccgta ccttgaaatc aattccttct tcagaagaaa     720
   tggcagcccg gaaacagaag tgcaggcgat gaagacactt tcatatttcg atattatgaa     780
   tctcgctgac cgagtgaagg tgcctgtcct gatgtcaatc ggcctgattg acaaggtcac     840
   gccgccgtcc accgtgtttg ccgcctacaa tcatttggaa acaaagaaag agctgaaggt     900
   gtaccgctac ttcggacatg agtatatccc tgcttttcaa actgaaaaac ttgctttctt     960
   taagcagcat cttaaaggct gataatctag atca                                 994
<210> 31
   <211> 960
   <212> DNA
   <213> Bacillus subtilis ATCC 29233
<220>
   <221> CDS
   <222> (1)..(960)
<400> 31
   atg caa cta ttc gat ctg ccg ctc gac caa ttg caa aca tat aag cct        48
   Met Gln Leu Phe Asp Leu Pro Leu Asp Gln Leu Gln Thr Tyr Lys Pro
   1               5                   10                  15
   gaa aaa aca gca ccg aaa gat ttt tct gag ttt tgg aaa ttg tct ttg        96
   Glu Lys Thr Ala Pro Lys Asp Phe Ser Glu Phe Trp Lys Leu Ser Leu
               20                  25                  30
   gag gaa ctt gca aaa gtc caa gca gaa cct gat cta cag ccg gtt gac       144
   Glu Glu Leu Ala Lys Val Gln Ala Glu Pro Asp Leu Gln Pro Val Asp
           35                  40                  45
   tat cct gct gac gga gta aaa gtg tac cgt ctc aca tat aaa agc ttc       192
   Tyr Pro Ala Asp Gly Val Lys Val Tyr Arg Leu Thr Tyr Lys Ser Phe
       50                  55                  60
   gga aac gcc cgc att acc gga tgg tac gcg gtg cct gac aag caa ggc       240
   Gly Asn Ala Arg Ile Thr Gly Trp Tyr Ala Val Pro Asp Lys Gln Gly
   65                  70                  75                  80
   ccg cat ccg gcg atc gtg aaa tat cat ggc tac aat gca agc tat gat       288
   Pro His Pro Ala Ile Val Lys Tyr His Gly Tyr Asn Ala Ser Tyr Asp
                   85                  90                  95
   ggt gag att cat gaa atg gta aac tgg gca ctc cat ggc tac gcc gca       336
   Gly Glu Ile His Glu Met Val Asn Trp Ala Leu His Gly Tyr Ala Ala
               100                 105                 110
   ttc ggc atg ctt gtc cgc ggc cag cag agc agc gag gat acg agt att       384
   Phe Gly Met Leu Val Arg Gly Gln Gln Ser Ser Glu Asp Thr Ser Ile
           115                 120                 125
   tca ccg cac ggt cac gct ttg ggc tgg atg acg aaa gga att ctt gat       432
   Ser Pro His Gly His Ala Leu Gly Trp Met Thr Lys Gly Ile Leu Asp
       130                 135                 140
   aaa gat aca tac tat tac cgc ggt gtt tat ttg gac gcc gtc cgc gcg       480
   Lys Asp Thr Tyr Tyr Tyr Arg Gly Val Tyr Leu Asp Ala Val Arg Ala
   145                 150                 155                 160
   ctt gag gtc atc agc agc ttc gac gag gtt gac gaa aca agg atc ggt       528
   Leu Glu Val Ile Ser Ser Phe Asp Glu Val Asp Glu Thr Arg Ile Gly
                   165                 170                 175
   gtg aca gga gga agc caa ggc gga ggt tta acc att gcc gca gca gcg       576
   Val Thr Gly Gly Ser Gln Gly Gly Gly Leu Thr Ile Ala Ala Ala Ala
               180                 185                 190
   ctg tca gac att cca aaa gcc gcg gtt gcc gat tat cct tat tta agc       624
   Leu Ser Asp Ile Pro Lys Ala Ala Val Ala Asp Tyr Pro Tyr Leu Ser
           195                 200                 205
   aac ttc gaa cgg gcc att gat gtg gcg ctt gaa cag ccg tac ctt gaa      672
   Asn Phe Glu Arg Ala Ile Asp Val Ala Leu Glu Gln Pro Tyr Leu Glu
       210                 215                 220
   atc aat tcc ttc ttc aga aga aat ggc agc ccg gaa aca gaa gtg cag      720
   Ile Asn Ser Phe Phe Arg Arg Asn Gly Ser Pro Glu Thr Glu Val Gln
   225                 230                 235                 240
   gcg atg aag aca ctt tca tat ttc gat att atg aat ctc gct gac cga      768
   Ala Met Lys Thr Leu Ser Tyr Phe Asp Ile Met Asn Leu Ala Asp Arg
                   245                 250                 255
   gtg aag gtg cct gtc ctg atg tca atc ggc ctg att gac aag gtc acg      816
   Val Lys Val Pro Val Leu Met Ser Ile Gly Leu Ile Asp Lys Val Thr
               260                 265                 270
   ccg cca tcc acc gtg ttt gcc gcc tac aat cat ttg gaa aca gag aaa      864
   Pro Pro Ser Thr Val Phe Ala Ala Tyr Asn His Leu Glu Thr Glu Lys
           275                 280                 285
   gag ctg aag gtg tac cgc tac ttc gga cat gag tat atc cct gct ttt      912
   Glu Leu Lys Val Tyr Arg Tyr Phe Gly His Glu Tyr Ile Pro Ala Phe
       290                 295                 300
   caa acg gaa aaa ctt gct ttc ttt aag cag cat ctt aaa ggc tga taa      960
   Gln Thr Glu Lys Leu Ala Phe Phe Lys Gln His Leu Lys Gly
   305                 310                 315
<210> 32
   <211> 318
   <212> PRT
   <213> Bacillus subtilis ATCC 29233
<400> 32
   Met Gln Leu Phe Asp Leu Pro Leu Asp Gln Leu Gln Thr Tyr Lys Pro
   1               5                   10                  15
   Glu Lys Thr Ala Pro Lys Asp Phe Ser Glu Phe Trp Lys Leu Ser Leu
               20                  25                  30
   Glu Glu Leu Ala Lys Val Gln Ala Glu Pro Asp Leu Gln Pro Val Asp
           35                  40                  45
   Tyr Pro Ala Asp Gly Val Lys Val Tyr Arg Leu Thr Tyr Lys Ser Phe
       50                  55                  60
   Gly Asn Ala Arg Ile Thr Gly Trp Tyr Ala Val Pro Asp Lys Gln Gly
   65                  70                  75                  80
   Pro His Pro Ala Ile Val Lys Tyr His Gly Tyr Asn Ala Ser Tyr Asp
                   85                  90                  95
   Gly Glu Ile His Glu Met Val Asn Trp Ala Leu His Gly Tyr Ala Ala
               100                 105                 110
   Phe Gly Met Leu Val Arg Gly Gln Gln Ser Ser Glu Asp Thr Ser Ile
   115 120 125
   Ser Pro His Gly His Ala Leu Gly Trp Met Thr Lys Gly Ile Leu Asp
   130 135 140
   Lys Asp Thr Tyr Tyr Tyr Arg Gly Val Tyr Leu Asp Ala Val Arg Ala
   145 150 155 160
   Leu Glu Val Ile Ser Ser Phe Asp Glu Val Asp Glu Thr Arg Ile Gly
   165 170 175
   Val Thr Gly Gly Ser Gln Gly Gly Gly Leu Thr Ile Ala Ala Ala Ala
   180 185 190
   Leu Ser Asp Ile Pro Lys Ala Ala Val Ala Asp Tyr Pro Tyr Leu Ser
   195 200 205
   Asn Phe Glu Arg Ala Ile Asp Val Ala Leu Glu Gln Pro Tyr Leu Glu
   210 215 220
   Ile Asn Ser Phe Phe Arg Arg Asn Gly Ser Pro Glu Thr Glu Val Gln
   225 230 235 240
   Ala Met Lys Thr Leu Ser Tyr Phe Asp Ile Met Asn Leu Ala Asp Arg
   245 250 255
   Val Lys Val Pro Val Leu Met Ser Ile Gly Leu Ile Asp Lys Val Thr
   260 265 270
   Pro Pro Ser Thr Val Phe Ala Ala Tyr Asn His Leu Glu Thr Glu Lys
   275 280 285
   Glu Leu Lys Val Tyr Arg Tyr Phe Gly His Glu Tyr Ile Pro Ala Phe
   290 295 300
   Gln Thr Glu Lys Leu Ala Phe Phe Lys Gln His Leu Lys Gly
   305 310 315
<210> 33
   <211> 24
   <212> DNA
   <213> artificial sequence
<220>
   <223> Primer
<400> 33
   atgcagcagc cttatgatgt gccg
<210> 34
   <211> 25
   <212> DNA
   <213> artificial sequence
<220>
   <223> Primer
<400> 34
   ttatttcaga tgctttctca gaaac 25
<210> 35
   <211> 27
   <212> DNA
   <213> artificial sequence
<220>
   <223> Primer
<400> 35
   atggcacaat tatatgatat gcctttg 27
<210> 36
   <211> 28
   <212> DNA
   <213> artificial sequence
<220>
   <223> Primer
<400> 36
   ttacggattc agctcatcca taagtatc 28
<210> 37
   <211> 24
   <212> DNA
   <213> artificial sequence
<220>
   <223> Primer
<400> 37
   atgcagctgt ttgacctgag cctg 24
<210> 38
   <211> 27
   <212> DNA
   <213> artificial sequence
<220>
   <223> Primer
<400> 38
   ttaggtggac agcagcaggt gcttttg 27
<210> 39
   <211> 24
   <212> DNA
   <213> artificial sequence
<220>
   <223> Primer
<400> 39
   atggctttct ttgacatgcc gctg 24
<210> 40
   <211> 27
   <212> DNA
   <213> artificial sequence
<220>
   <223> Primer
<400> 40
   ttagccttct tcgaacaggc gtttcag 27
<210> 41
   <211> 978
   <212> DNA
   <213> artificial sequence
<220>
   <223> Synthetic construct
<400> 41
   atggctttct ttgacatgcc gctggaagaa ctgaaaaagt accgtccgga acgttacgag      60
   gaaaaagact ttgacgaatt ttggcgcgaa accctgaaag aatccgaggg tttcccactg     120
   gacccggtat ttgaaaaagt tgacttccac ctgaagaccg tcgaaactta cgacgtcacc     180
   ttcagcggtt atcgtggcca gcgtatcaaa ggttggctgc tggtaccgaa actggcggaa     240
   gagaaactgc cgtgtgttgt tcagtacatt ggttacaacg gtggccgtgg tttcccgcac     300
   gactggctgt tctggccgtc tatgggttac atctgcttcg ttatggacac ccgtggtcag     360
   ggtagcggtt ggatgaaggg tgatactccg gactacccgg aaggtccggt ggacccgcag     420
   tacccgggct tcatgacgcg cggcatcctg gatcctggca cctattacta ccgtcgtgtg     480
   tttgtcgatg ccgtgcgcgc cgttgaagcc gctatcagct tcccacgcgt cgattctcgt     540
   aaagtggtag ttgctggtgg ctctcaaggt ggcggcattg cactggcagt ttccgcgctg     600
   tccaaccgtg ttaaagccct gctgtgcgat gttccgttcc tgtgccactt ccgtcgtgcg     660
   gtacagctgg tggacaccca cccgtacgta gaaattacga acttcctgaa aacccatcgt     720
   gataaagaag agatcgtatt ccgtaccctg tcttactttg atggcgttaa ttttgcggct     780
   cgtgcaaaag taccggcgct gttcagcgta ggtctgatgg acactatttg tccgccgtct     840
   accgtattcg cagcctacaa ccactacgct ggtccgaaag aaatccgcat ctacccgtac     900
   aacaaccacg aaggtggtgg ttctttccag gcaatcgaac aggttaaatt cctgaaacgc     960
   ctgttcgaag aaggctaa                                                   978
<210> 42
   <211> 795
   <212> DNA
   <213> artificial sequence
<220>
   <223> Primer
<400> 42
   atgattgaac aagatggatt gcacgcaggt tctccggccg cttgggtgga gaggctattc 60
   ggctatgact gggcacaaca gacaatcggc tgctctgatg ccgccgtgtt ccggctgtca 120
   gcgcaggggc gcccggttct ttttgtcaag accgacctgt ccggtgccct gaatgaactg 180
   caggacgagg cagcgcggct atcgtggctg gccacgacgg gcgttccttg cgcagctgtg 240
   ctcgacgttg tcactgaagc gggaagggac tggctgctat tgggcgaagt gccggggcag 300
   gatctcctgt catctcacct tgctcctgcc gagaaagtat ccatcatggc tgatgcaatg 360
   cggcggctgc atacgcttga tccggctacc tgcccattcg accaccaagc gaaacatcgc 420
   atcgagcgag cacgtactcg gatggaagcc ggtcttgtcg atcaggatga tctggacgaa 480
   gagcatcagg ggctcgcgcc agccgaactg ttcgccaggc tcaaggcgcg catgcccgac 540
   ggcgaggatc tcgtcgtgac ccatggcgat gcctgcttgc cgaatatcat ggtggaaaat 600
   ggccgctttt ctggattcat cgactgtggc cggctgggtg tggcggaccg ctatcaggac 660
   atagcgttgg ctacccgtga tattgctgaa gagcttggcg gcgaatgggc tgaccgcttc 720
   ctcgtgcttt acggtatcgc cgctcccgat tcgcagcgca tcgccttcta tcgccttctt 780
   gacgagttct tctaa 795
<210> 43
   <211> 3434
   <212> DNA
   <213> artificial sequence
<220>
   <223> Plasmid pKDl3
<400> 43
   agattgcagc attacacgtc ttgagcgatt gtgtaggctg gagctgcttc gaagttccta 60
   tactttctag agaataggaa cttcggaata ggaacttcaa gatcccctta ttagaagaac 120
   tcgtcaagaa ggcgatagaa ggcgatgcgc tgcgaatcgg gagcggcgat accgtaaagc 180
   acgaggaagc ggtcagccca ttcgccgcca agctcttcag caatatcacg ggtagccaac 240
   gctatgtcct gatagcggtc cgccacaccc agccggccac agtcgatgaa tccagaaaag 300
   cggccatttt ccaccatgat attcggcaag caggcatcgc catgggtcac gacgagatcc 360
   tcgccgtcgg gcatgcgcgc cttgagcctg gcgaacagtt cggctggcgc gagcccctga 420
   tgctcttcgt ccagatcatc ctgatcgaca agaccggctt ccatccgagt acgtgctcgc 480
   tcgatgcgat gtttcgcttg gtggtcgaat gggcaggtag ccggatcaag cgtatgcagc 540
   cgccgcattg catcagccat gatggatact ttctcggcag gagcaaggtg agatgacagg 600
   agatcctgcc ccggcacttc gcccaatagc agccagtccc ttcccgcttc agtgacaacg 660
   tcgagcacag ctgcgcaagg aacgcccgtc gtggccagcc acgatagccg cgctgcctcg 720
   tcctgcagtt cattcagggc accggacagg tcggtcttga caaaaagaac cgggcgcccc 780
   tgcgctgaca gccggaacac ggcggcatca gagcagccga ttgtctgttg tgcccagtca 840
   tagccgaata gcctctccac ccaagcggcc ggagaacctg cgtgcaatcc atcttgttca 900
   atcatgcgaa acgatcctca tcctgtctct tgatcagatc ttgatcccct gcgccatcag 960
   atccttggcg gcaagaaagc catccagttt actttgcagg gcttcccaac cttaccagag 1020
   ggcgccccag ctggcaattc cggttcgctt gctgtccata aaaccgccca gtctagctat 1080
   cgccatgtaa gcccactgca agctacctgc tttctctttg cgcttgcgtt ttcccttgtc 1140
   cagatagccc agtagctgac attcatccgg ggtcagcacc gtttctgcgg actggctttc 1200
   tacgtgttcc gcttccttta gcagcccttg cgccctgagt gcttgcggca gcgtgagctt 1260
   caaaagcgct ctgaagttcc tatactttct agagaatagg aacttcgaac tgcaggtcga 1320
   cggatccccg gaattaattc tcatgtttga cagcttatca ctgatcagtg aattaatggc 1380
   gatgacgcat cctcacgata atatccgggt aggcgcaatc actttcgtct ctactccgtt 1440
   acaaagcgag gctgggtatt tcccggcctt tctgttatcc gaaatccact gaaagcacag 1500
   cggctggctg aggagataaa taataaacga ggggctgtat gcacaaagca tcttctgttg 1560
   agttaagaac gagtatcgag atggcacata gccttgctca aattggaatc aggtttgtgc 1620
   caataccagt agaaacagac gaagaagcta gctttgcact ggattgcgag gctttgccat 1680
   ggctaattcc catgtcagcc gttaagtgtt cctgtgtcac tgaaaattgc tttgagaggc 1740
   tctaagggct tctcagtgcg ttacatccct ggcttgttgt ccacaaccgt taaaccttaa 1800
   aagctttaaa agccttatat attctttttt ttcttataaa acttaaaacc ttagaggcta 1860
   tttaagttgc tgatttatat taattttatt gttcaaacat gagagcttag tacgtgaaac 1920
   atgagagctt agtacgttag ccatgagagc ttagtacgtt agccatgagg gtttagttcg 1980
   ttaaacatga gagcttagta cgttaaacat gagagcttag tacgtgaaac atgagagctt 2040
   agtacgtact atcaacaggt tgaactgcgg atcttgcggc cgcaaaaatt aaaaatgaag 2100
   ttttaaatca atctaaagta tatatgagta aacttggtct gacagttacc aatgcttaat 2160
   cagtgaggca cctatctcag cgatctgtct atttcgttca tccatagttg cctgactccc 2220
   cgtcgtgtag ataactacga tacgggaggg cttaccatct ggccccagtg ctgcaatgat 2280
   accgcgagac ccacgctcac cggctccaga tttatcagca ataaaccagc cagccggaag 2340
   ggccgagcgc agaagtggtc ctgcaacttt atccgcctcc atccagtcta ttaattgttg 2400
   ccgggaagct agagtaagta gttcgccagt taatagtttg cgcaacgttg ttgccattgc 2460
   tacaggcatc gtggtgtcac gctcgtcgtt tggtatggct tcattcagct ccggttccca 2520
   acgatcaagg cgagttacat gatcccccat gttgtgcaaa aaagcggtta gctccttcgg   2580
   tcctccgatc gttgtcagaa gtaagttggc cgcagtgtta tcactcatgg ttatggcagc   2640
   actgcataat tctcttactg tcatgccatc cgtaagatgc ttttctgtga ctggtgagta   2700
   ctcaaccaag tcattctgag aatagtgtat gcggcgaccg agttgctctt gcccggcgtc   2760
   aatacgggat aataccgcgc cacatagcag aactttaaaa gtgctcatca ttggaaaacg   2820
   ttcttcgggg cgaaaactct caaggatctt accgctgttg agatccagtt cgatgtaacc   2880
   cactcgtgca cccaactgat cttcagcatc ttttactttc accagcgttt ctgggtgagc   2940
   aaaaacagga aggcaaaatg ccgcaaaaaa gggaataagg gcgacacgga aatgttgaat   3000
   actcatactc ttcctttttc aatattattg aagcatttat cagggttatt gtctcatgag   3060
   cggatacata tttgaatgta tttagaaaaa taaacaaata ggggttccgc gcacatttcc   3120
   ccgaaaagtg ccacctgcat cgatggcccc ccgatggtag tgtggggtct ccccatgcga   3180
   gagtagggaa ctgccaggca tcaaataaaa cgaaaggctc agtcgaaaga ctgggccttt   3240
   cgttttatct gttgtttgtc ggtgaacgct ctcctgagta ggacaaatcc gccgggagcg   3300
   gatttgaacg ttgcgaagca acggcccgga gggtggcggg caggacgccc gccataaact   3360
   gccaggcatc aaattaagca gaaggccatc ctgacggatg gcctttttgc gtggccagtg   3420
   ccaagcttgc atgc                                                     3434
<210> 44
   <211> 80
   <212> DNA
   <213> artificial sequence
<220>
   <223> Primer
<400> 44
   atgagcacgt cagacgatat ccataacacc acagccactg gcaaatgccc gttccatcag     60
   gtgtaggctg gagctgcttc                                                 80
<210> 45
   <211> 82
   <212> DNA
   <213> artificial sequence
<220>
   <223> Primer
<400> 45
   taacagcagg tcgaaacggt cgaggttcat cactttcacc catgccgcca cgaagtcttt      60
   attccgggga tccgtcgacc tg                                               82
<210> 46
   <211> 1424
   <212> DNA
   <213> artificial sequence
<220>
   <223> Synthetic construct
<400> 46
   taacagcagg tcgaaacggt cgaggttcat cactttcacc catgccgcca cgaagtcttt 60
   attccgggga tccgtcgacc tgcagttcga agttcctatt ctctagaaag tataggaact 120
   tcagagcgct tttgaagctc acgctgccgc aagcactcag ggcgcaaggg ctgctaaagg 180
   aagcggaaca cgtagaaagc cagtccgcag aaacggtgct gaccccggat gaatgtcagc 240
   tactgggcta tctggacaag ggaaaacgca agcgcaaaga gaaagcaggt agcttgcagt 300
   gggcttacat ggcgatagct agactgggcg gttttatgga cagcaagcga accggaattg 360
   ccagctgggg cgccctctgg taaggttggg aagccctgca aagtaaactg gatggctttc 420
   ttgccgccaa ggatctgatg gcgcagggga tcaagatctg atcaagagac aggatgagga 480
   tcgtttcgca tgattgaaca agatggattg cacgcaggtt ctccggccgc ttgggtggag 540
   aggctattcg gctatgactg ggcacaacag acaatcggct gctctgatgc cgccgtgttc 600
   cggctgtcag cgcaggggcg cccggttctt tttgtcaaga ccgacctgtc cggtgccctg 660
   aatgaactgc aggacgaggc agcgcggcta tcgtggctgg ccacgacggg cgttccttgc 720
   gcagctgtgc tcgacgttgt cactgaagcg ggaagggact ggctgctatt gggcgaagtg 780
   ccggggcagg atctcctgtc atctcacctt gctcctgccg agaaagtatc catcatggct 840
   gatgcaatgc ggcggctgca tacgcttgat ccggctacct gcccattcga ccaccaagcg 900
   aaacatcgca tcgagcgagc acgtactcgg atggaagccg gtcttgtcga tcaggatgat 960
   ctggacgaag agcatcaggg gctcgcgcca gccgaactgt tcgccaggct caaggcgcgc 1020
   atgcccgacg gcgaggatct cgtcgtgacc catggcgatg cctgcttgcc gaatatcatg 1080
   gtggaaaatg gccgcttttc tggattcatc gactgtggcc ggctgggtgt ggcggaccgc 1140
   tatcaggaca tagcgttggc tacccgtgat attgctgaag agcttggcgg cgaatgggct 1200
   gaccgcttcc tcgtgcttta cggtatcgcc gctcccgatt cgcagcgcat cgccttctat 1260
   cgccttcttg acgagttctt ctaataaggg gatcttgaag ttcctattcc gaagttccta 1320
   ttctctagaa agtataggaa cttcgaagca gctccagcct acacctgatg gaacgggcat 1380
   ttgccagtgg ctgtggtgtt atggatatcg tctgacgtgc tcat 1424
<210> 47
   <211> 2181
   <212> DNA
   <213> Escherichia coli
<220>
   <221> CDS
   <222> (1)..(2181)
<400> 47
   atg agc acg tca gac gat atc cat aac acc aca gcc act ggc aaa tgc 48
   Met Ser Thr Ser Asp Asp Ile His Asn Thr Thr Ala Thr Gly Lys Cys
   1 5 10 15
   ccg ttc cat cag ggc ggt cac gac cag agt gcg ggg gcg ggc aca acc 96
   Pro Phe His Gln Gly Gly His Asp Gln Ser Ala Gly Ala Gly Thr Thr
   20 25 30
   act cgc gac tgg tgg cca aat caa ctt cgt gtt gac ctg tta aac caa 144
   Thr Arg Asp Trp Trp Pro Asn Gln Leu Arg Val Asp Leu Leu Asn Gln
   35 40 45
   cat tct aat cgt tct aac cca ctg ggt gag gac ttt gac tac cgc aaa 192
   His Ser Asn Arg Ser Asn Pro Leu Gly Glu Asp Phe Asp Tyr Arg Lys
   50 55 60
   gaa ttc agc aaa tta gat tac tac ggc ctg aaa aaa gat ctg aaa gcc 240
   Glu Phe Ser Lys Leu Asp Tyr Tyr Gly Leu Lys Lys Asp Leu Lys Ala
   65 70 75 80
   ctg ttg aca gaa tct caa ccg tgg tgg cca gcc gac tgg ggc agt tac 288
   Leu Leu Thr Glu Ser Gln Pro Trp Trp Pro Ala Asp Trp Gly Ser Tyr
   85 90 95
   gcc ggt ctg ttt att cgt atg gcc tgg cac ggc gcg ggg act tac cgt 336
   Ala Gly Leu Phe Ile Arg Met Ala Trp His Gly Ala Gly Thr Tyr Arg
   100 105 110
   tca atc gat gga cgc ggt ggc gcg ggt cgt ggt cag caa cgt ttt gca 384
   Ser Ile Asp Gly Arg Gly Gly Ala Gly Arg Gly Gln Gln Arg Phe Ala
   115 120 125
   ccg ctg aac tcc tgg ccg gat aac gta agc ctc gat aaa gcg cgt cgc 432
   Pro Leu Asn Ser Trp Pro Asp Asn Val Ser Leu Asp Lys Ala Arg Arg
   130 135 140
   ctg ttg tgg cca atc aaa cag aaa tat ggt cag aaa atc tcc tgg gcc 480
   Leu Leu Trp Pro Ile Lys Gln Lys Tyr Gly Gln Lys Ile Ser Trp Ala
   145 150 155 160
   gac ctg ttt atc ctc gcg ggt aac gtg gcg cta gaa aac tcc ggc ttc 528
   Asp Leu Phe Ile Leu Ala Gly Asn Val Ala Leu Glu Asn Ser Gly Phe
   165 170 175
   cgt acc ttc ggt ttt ggt gcc ggt cgt gaa gac gtc tgg gaa ccg gat 576
   Arg Thr Phe Gly Phe Gly Ala Gly Arg Glu Asp Val Trp Glu Pro Asp
   180 185 190
   ctg gat gtt aac tgg ggt gat gaa aaa gcc tgg ctg act cac cgt cat 624
   Leu Asp Val Asn Trp Gly Asp Glu Lys Ala Trp Leu Thr His Arg His
   195 200 205
   ccg gaa gcg ctg gcg aaa gca ccg ctg ggt gca acc gag atg ggt ctg 672
   Pro Glu Ala Leu Ala Lys Ala Pro Leu Gly Ala Thr Glu Met Gly Leu
   210 215 220
   att tac gtt aac ccg gaa ggc ccg gat cac agc ggc gaa ccg ctt tct 720
   Ile Tyr Val Asn Pro Glu Gly Pro Asp His Ser Gly Glu Pro Leu Ser
   225 230 235 240
   gcg gca gca gct atc cgc gcg acc ttc ggc aac atg ggc atg aac gac 768
   Ala Ala Ala Ala Ile Arg Ala Thr Phe Gly Asn Met Gly Met Asn Asp
   245 250 255
   gaa gaa acc gtg gcg ctg att gcg ggt ggt cat acg ctg ggt aaa acc 816
   Glu Glu Thr Val Ala Leu Ile Ala Gly Gly His Thr Leu Gly Lys Thr
               260                 265                 270
   cac ggt gcc ggt ccg aca tca aat gta ggt cct gat cca gaa gct gca      864
   His Gly Ala Gly Pro Thr Ser Asn Val Gly Pro Asp Pro Glu Ala Ala
           275                 280                 285
   ccg att gaa gaa caa ggt tta ggt tgg gcg agc act tac ggc agc ggc      912
   Pro Ile Glu Glu Gln Gly Leu Gly Trp Ala Ser Thr Tyr Gly Ser Gly
       290                 295                 300
   gtt ggc gca gat gcc att acc tct ggt ctg gaa gta gtc tgg acc cag      960
   Val Gly Ala Asp Ala Ile Thr Ser Gly Leu Glu Val Val Trp Thr Gln
   305                 310                 315                 320
   acg ccg acc cag tgg agc aac tat ttc ttc gag aac ctg ttc aag tat     1008
   Thr Pro Thr Gln Trp Ser Asn Tyr Phe Phe Glu Asn Leu Phe Lys Tyr
                   325                 330                 335
   gag tgg gta cag acc cgc agc ccg gct ggc gca atc cag ttc gaa gcg     1056
   Glu Trp Val Gln Thr Arg Ser Pro Ala Gly Ala Ile Gln Phe Glu Ala
               340                 345                 350
   gta gac gca ccg gaa att atc ccg gat ccg ttt gat ccg tcg aag aaa     1104
   Val Asp Ala Pro Glu Ile Ile Pro Asp Pro Phe Asp Pro Ser Lys Lys
           355                 360                 365
   cgt aaa ccg aca atg ctg gtg acc gac ctg acg ctg cgt ttt gat cct     1152
   Arg Lys Pro Thr Met Leu Val Thr Asp Leu Thr Leu Arg Phe Asp Pro
       370                 375                 380
   gag ttc gag aag atc tct cgt cgt ttc ctc aac gat ccg cag gcg ttc     1200
   Glu Phe Glu Lys Ile Ser Arg Arg Phe Leu Asn Asp Pro Gln Ala Phe
   385                 390                 395                 400
   aac gaa gcc ttt gcc cgt gcc tgg ttc aaa ctg acg cac agg gat atg     1248
   Asn Glu Ala Phe Ala Arg Ala Trp Phe Lys Leu Thr His Arg Asp Met
                   405                 410                 415
   ggg ccg aaa tct cgc tac atc ggg ccg gaa gtg ccg aaa gaa gat ctg     1296
   Gly Pro Lys Ser Arg Tyr Ile Gly Pro Glu Val Pro Lys Glu Asp Leu
               420                 425                 430
   atc tgg caa gat ccg ctg ccg cag ccg atc tac aac ccg acc gag cag     1344
   Ile Trp Gln Asp Pro Leu Pro Gln Pro Ile Tyr Asn Pro Thr Glu Gln
           435                 440                 445
   gac att atc gat ctg aaa ttc gcg att gcg gat tct ggt ctg tct gtt     1392
   Asp Ile Ile Asp Leu Lys Phe Ala Ile Ala Asp Ser Gly Leu Ser Val
       450                 455                 460
   agt gag ctg gta tcg gtg gcc tgg gca tct gct tct acc ttc cgt ggt     1440
   Ser Glu Leu Val Ser Val Ala Trp Ala Ser Ala Ser Thr Phe Arg Gly
   465                 470                 475                 480
   ggc gac aaa cgc ggt ggt gcc aac ggt gcg cgt ctg gca tta atg ccg     1488
   Gly Asp Lys Arg Gly Gly Ala Asn Gly Ala Arg Leu Ala Leu Met Pro
                   485                 490                 495
   cag cgc gac tgg gat gtg aac gcc gca gcc gtt cgt gct ctg cct gtt     1536
   Gln Arg Asp Trp Asp Val Asn Ala Ala Ala Val Arg Ala Leu Pro Val
               500                 505                 510
   ctg gag aaa atc cag aaa gag tct ggt aaa gcc tcg ctg gcg gat atc     1584
   Leu Glu Lys Ile Gln Lys Glu Ser Gly Lys Ala Ser Leu Ala Asp Ile
   515 520 525
   ata gtg ctg gct ggt gtg gtt ggt gtt gag aaa gcc gca agc gcc gca 1632
   Ile Val Leu Ala Gly Val Val Gly Val Glu Lys Ala Ala Ser Ala Ala
   530 535 540
   ggt ttg agc att cat gta ccg ttt gcg ccg ggt cgc gtt gat gcg cgt 1680
   Gly Leu Ser Ile His Val Pro Phe Ala Pro Gly Arg Val Asp Ala Arg
   545 550 555 560
   cag gat cag act gac att gag atg ttt gag ctg ctg gag cca att gct 1728
   Gln Asp Gln Thr Asp Ile Glu Met Phe Glu Leu Leu Glu Pro Ile Ala
   565 570 575
   gac ggt ttc cgt aac tat cgc gct cgt ctg gac gtt tcc acc acc gag 1776
   Asp Gly Phe Arg Asn Tyr Arg Ala Arg Leu Asp Val Ser Thr Thr Glu
   580 585 590
   tca ctg ctg atc gac aaa gca cag caa ctg acg ctg acc gcg ccg gaa 1824
   Ser Leu Leu Ile Asp Lys Ala Gln Gln Leu Thr Leu Thr Ala Pro Glu
   595 600 605
   atg act gcg ctg gtg ggc ggc atg cgt gta ctg ggt gcc aac ttc gat 1872
   Met Thr Ala Leu Val Gly Gly Met Arg Val Leu Gly Ala Asn Phe Asp
   610 615 620
   ggc agc aaa aac ggc gtc ttc act gac cgc gtt ggc gta ttg agc aat 1920
   Gly Ser Lys Asn Gly Val Phe Thr Asp Arg Val Gly Val Leu Ser Asn
   625 630 635 640
   gac ttc ttc gtg aac ttg ctg gat atg cgt tac gag tgg aaa gcg acc 1968
   Asp Phe Phe Val Asn Leu Leu Asp Met Arg Tyr Glu Trp Lys Ala Thr
   645 650 655
   gac gaa tcg aaa gag ctg ttc gaa ggc cgt gac cgt gaa acc ggc gaa 2016
   Asp Glu Ser Lys Glu Leu Phe Glu Gly Arg Asp Arg Glu Thr Gly Glu
   660 665 670
   gtg aaa ttt acg gcc agc cgt gcg gat ctg gtg ttt ggt tct aac tcc 2064
   Val Lys Phe Thr Ala Ser Arg Ala Asp Leu Val Phe Gly Ser Asn Ser
   675 680 685
   gtc ctg cgt gcg gtg gcg gaa gtt tac gcc agt agc gat gcc cac gag 2112
   Val Leu Arg Ala Val Ala Glu Val Tyr Ala Ser Ser Asp Ala His Glu
   690 695 700
   aag ttt gtt aaa gac ttc gtg gcg gca tgg gtg aaa gtg atg aac ctc 2160
   Lys Phe Val Lys Asp Phe Val Ala Ala Trp Val Lys Val Met Asn Leu
   705 710 715 720
   gac cgt ttc gac ctg ctg taa 2181
   Asp Arg Phe Asp Leu Leu
   725
<210> 48
   <211> 726
   <212> PRT
   <213> Escherichia coli
<400> 48
   Met Ser Thr Ser Asp Asp Ile His Asn Thr Thr Ala Thr Gly Lys Cys
   1               5                   10                  15
   Pro Phe His Gln Gly Gly His Asp Gln Ser Ala Gly Ala Gly Thr Thr
               20                  25                  30
   Thr Arg Asp Trp Trp Pro Asn Gln Leu Arg Val Asp Leu Leu Asn Gln
           35                  40                  45
   His Ser Asn Arg Ser Asn Pro Leu Gly Glu Asp Phe Asp Tyr Arg Lys
       50                  55                  60
   Glu Phe Ser Lys Leu Asp Tyr Tyr Gly Leu Lys Lys Asp Leu Lys Ala
   65                  70                  75                  80
   Leu Leu Thr Glu Ser Gln Pro Trp Trp Pro Ala Asp Trp Gly Ser Tyr
                   85                  90                  95
   Ala Gly Leu Phe Ile Arg Met Ala Trp His Gly Ala Gly Thr Tyr Arg
               100                 105                 110
   Ser Ile Asp Gly Arg Gly Gly Ala Gly Arg Gly Gln Gln Arg Phe Ala
           115                 120                 125
   Pro Leu Asn Ser Trp Pro Asp Asn Val Ser Leu Asp Lys Ala Arg Arg
       130                 135                 140
   Leu Leu Trp Pro Ile Lys Gln Lys Tyr Gly Gln Lys Ile Ser Trp Ala
   145                 150                 155                 160
   Asp Leu Phe Ile Leu Ala Gly Asn Val Ala Leu Glu Asn Ser Gly Phe
                   165                 170                 175
   Arg Thr Phe Gly Phe Gly Ala Gly Arg Glu Asp Val Trp Glu Pro Asp
               180                 185                 190
   Leu Asp Val Asn Trp Gly Asp Glu Lys Ala Trp Leu Thr His Arg His
           195                 200                 205
   Pro Glu Ala Leu Ala Lys Ala Pro Leu Gly Ala Thr Glu Met Gly Leu
       210                 215                 220
   Ile Tyr Val Asn Pro Glu Gly Pro Asp His Ser Gly Glu Pro Leu Ser
   225                 230                 235                 240
   Ala Ala Ala Ala Ile Arg Ala Thr Phe Gly Asn Met Gly Met Asn Asp
                   245                 250                 255
   Glu Glu Thr Val Ala Leu Ile Ala Gly Gly His Thr Leu Gly Lys Thr
               260                 265                 270
   His Gly Ala Gly Pro Thr Ser Asn Val Gly Pro Asp Pro Glu Ala Ala
   275 280 285
   Pro Ile Glu Glu Gln Gly Leu Gly Trp Ala Ser Thr Tyr Gly Ser Gly
   290 295 300
   Val Gly Ala Asp Ala Ile Thr Ser Gly Leu Glu Val Val Trp Thr Gln
   305 310 315 320
   Thr Pro Thr Gln Trp Ser Asn Tyr Phe Phe Glu Asn Leu Phe Lys Tyr
   325 330 335
   Glu Trp Val Gln Thr Arg Ser Pro Ala Gly Ala Ile Gln Phe Glu Ala
   340 345 350
   Val Asp Ala Pro Glu Ile Ile Pro Asp Pro Phe Asp Pro Ser Lys Lys
   355 360 365
   Arg Lys Pro Thr Met Leu Val Thr Asp Leu Thr Leu Arg Phe Asp Pro
   370 375 380
   Glu Phe Glu Lys Ile Ser Arg Arg Phe Leu Asn Asp Pro Gln Ala Phe
   385 390 395 400
   Asn Glu Ala Phe Ala Arg Ala Trp Phe Lys Leu Thr His Arg Asp Met
   405 410 415
   Gly Pro Lys Ser Arg Tyr Ile Gly Pro Glu Val Pro Lys Glu Asp Leu
   420 425 430
   Ile Trp Gln Asp Pro Leu Pro Gln Pro Ile Tyr Asn Pro Thr Glu Gln
   435 440 445
   Asp Ile Ile Asp Leu Lys Phe Ala Ile Ala Asp Ser Gly Leu Ser Val
   450 455 460
   Ser Glu Leu Val Ser Val Ala Trp Ala Ser Ala Ser Thr Phe Arg Gly
   465 470 475 480
   Gly Asp Lys Arg Gly Gly Ala Asn Gly Ala Arg Leu Ala Leu Met Pro
   485 490 495
   Gln Arg Asp Trp Asp Val Asn Ala Ala Ala Val Arg Ala Leu Pro Val
   500 505 510
   Leu Glu Lys Ile Gln Lys Glu Ser Gly Lys Ala Ser Leu Ala Asp Ile
   515 520 525
   Ile Val Leu Ala Gly Val Val Gly Val Glu Lys Ala Ala Ser Ala Ala
   530 535 540
   Gly Leu Ser Ile His Val Pro Phe Ala Pro Gly Arg Val Asp Ala Arg
   545 550 555 560
   Gln Asp Gln Thr Asp Ile Glu Met Phe Glu Leu Leu Glu Pro Ile Ala
   565 570 575
   Asp Gly Phe Arg Asn Tyr Arg Ala Arg Leu Asp Val Ser Thr Thr Glu
   580 585 590
   Ser Leu Leu Ile Asp Lys Ala Gln Gln Leu Thr Leu Thr Ala Pro Glu
   595 600 605
   Met Thr Ala Leu Val Gly Gly Met Arg Val Leu Gly Ala Asn Phe Asp
   610 615 620
   Gly Ser Lys Asn Gly Val Phe Thr Asp Arg Val Gly Val Leu Ser Asn
   625 630 635 640
   Asp Phe Phe Val Asn Leu Leu Asp Met Arg Tyr Glu Trp Lys Ala Thr
   645 650 655
   Asp Glu Ser Lys Glu Leu Phe Glu Gly Arg Asp Arg Glu Thr Gly Glu
   660 665 670
   Val Lys Phe Thr Ala Ser Arg Ala Asp Leu Val Phe Gly Ser Asn Ser
   675 680 685
   Val Leu Arg Ala Val Ala Glu Val Tyr Ala Ser Ser Asp Ala His Glu
   690 695 700
   Lys Phe Val Lys Asp Phe Val Ala Ala Trp Val Lys Val Met Asn Leu
   705 710 715 720
   Asp Arg Phe Asp Leu Leu
   725
<210> 49
   <211> 6329
   <212> DNA
   <213> artificial sequence
<220>
   <223> Plasmid pKD46
<400> 49
   catcgattta ttatgacaac ttgacggcta catcattcac tttttcttca caaccggcac 60
   ggaactcgct cgggctggcc ccggtgcatt ttttaaatac ccgcgagaaa tagagttgat 120
   cgtcaaaacc aacattgcga ccgacggtgg cgataggcat ccgggtggtg ctcaaaagca 180
   gcttcgcctg gctgatacgt tggtcctcgc gccagcttaa gacgctaatc cctaactgct 240
   ggcggaaaag atgtgacaga cgcgacggcg acaagcaaac atgctgtgcg acgctggcga 300
   tatcaaaatt gctgtctgcc aggtgatcgc tgatgtactg acaagcctcg cgtacccgat 360
   tatccatcgg tggatggagc gactcgttaa tcgcttccat gcgccgcagt aacaattgct 420
   caagcagatt tatcgccagc agctccgaat agcgcccttc cccttgcccg gcgttaatga 480
   tttgcccaaa caggtcgctg aaatgcggct ggtgcgcttc atccgggcga aagaaccccg 540
   tattggcaaa tattgacggc cagttaagcc attcatgcca gtaggcgcgc ggacgaaagt 600
   aaacccactg gtgataccat tcgcgagcct ccggatgacg accgtagtga tgaatctctc 660
   ctggcgggaa cagcaaaata tcacccggtc ggcaaacaaa ttctcgtccc tgatttttca 720
   ccaccccctg accgcgaatg gtgagattga gaatataacc tttcattccc agcggtcggt 780
   cgataaaaaa atcgagataa ccgttggcct caatcggcgt taaacccgcc accagatggg 840
   cattaaacga gtatcccggc agcaggggat cattttgcgc ttcagccata cttttcatac 900
   tcccgccatt cagagaagaa accaattgtc catattgcat cagacattgc cgtcactgcg 960
   tcttttactg gctcttctcg ctaaccaaac cggtaacccc gcttattaaa agcattctgt 1020
   aacaaagcgg gaccaaagcc atgacaaaaa cgcgtaacaa aagtgtctat aatcacggca 1080
   gaaaagtcca cattgattat ttgcacggcg tcacactttg ctatgccata gcatttttat 1140
   ccataagatt agcggatcct acctgacgct ttttatcgca actctctact gtttctccat 1200
   acccgttttt ttgggaattc gagctctaag gaggttataa aaaatggata ttaatactga 1260
   aactgagatc aagcaaaagc attcactaac cccctttcct gttttcctaa tcagcccggc 1320
   atttcgcggg cgatattttc acagctattt caggagttca gccatgaacg cttattacat 1380
   tcaggatcgt cttgaggctc agagctgggc gcgtcactac cagcagctcg cccgtgaaga 1440
   gaaagaggca gaactggcag acgacatgga aaaaggcctg ccccagcacc tgtttgaatc 1500
   gctatgcatc gatcatttgc aacgccacgg ggccagcaaa aaatccatta cccgtgcgtt 1560
   tgatgacgat gttgagtttc aggagcgcat ggcagaacac atccggtaca tggttgaaac 1620
   cattgctcac caccaggttg atattgattc agaggtataa aacgaatgag tactgcactc 1680
   gcaacgctgg ctgggaagct ggctgaacgt gtcggcatgg attctgtcga cccacaggaa 1740
   ctgatcacca ctcttcgcca gacggcattt aaaggtgatg ccagcgatgc gcagttcatc 1800
   gcattactga tcgttgccaa ccagtacggc cttaatccgt ggacgaaaga aatttacgcc 1860
   tttcctgata agcagaatgg catcgttccg gtggtgggcg ttgatggctg gtcccgcatc 1920
   atcaatgaaa accagcagtt tgatggcatg gactttgagc aggacaatga atcctgtaca 1980
   tgccggattt accgcaagga ccgtaatcat ccgatctgcg ttaccgaatg gatggatgaa 2040
   tgccgccgcg aaccattcaa aactcgcgaa ggcagagaaa tcacggggcc gtggcagtcg 2100
   catcccaaac ggatgttacg tcataaagcc atgattcagt gtgcccgtct ggccttcgga 2160
   tttgctggta tctatgacaa ggatgaagcc gagcgcattg tcgaaaatac tgcatacact 2220
   gcagaacgtc agccggaacg cgacatcact ccggttaacg atgaaaccat gcaggagatt 2280
   aacactctgc tgatcgccct ggataaaaca tgggatgacg acttattgcc gctctgttcc 2340
   cagatatttc gccgcgacat tcgtgcatcg tcagaactga cacaggccga agcagtaaaa 2400
   gctcttggat tcctgaaaca gaaagccgca gagcagaagg tggcagcatg acaccggaca 2460
   ttatcctgca gcgtaccggg atcgatgtga gagctgtcga acagggggat gatgcgtggc 2520
   acaaattacg gctcggcgtc atcaccgctt cagaagttca caacgtgata gcaaaacccc 2580
   gctccggaaa gaagtggcct gacatgaaaa tgtcctactt ccacaccctg cttgctgagg 2640
   tttgcaccgg tgtggctccg gaagttaacg ctaaagcact ggcctgggga aaacagtacg 2700
   agaacgacgc cagaaccctg tttgaattca cttccggcgt gaatgttact gaatccccga 2760
   tcatctatcg cgacgaaagt atgcgtaccg cctgctctcc cgatggttta tgcagtgacg 2820
   gcaacggcct tgaactgaaa tgcccgttta cctcccggga tttcatgaag ttccggctcg 2880
   gtggtttcga ggccataaag tcagcttaca tggcccaggt gcagtacagc atgtgggtga 2940
   cgcgaaaaaa tgcctggtac tttgccaact atgacccgcg tatgaagcgt gaaggcctgc 3000
   attatgtcgt gattgagcgg gatgaaaagt acatggcgag ttttgacgag atcgtgccgg 3060
   agttcatcga aaaaatggac gaggcactgg ctgaaattgg ttttgtattt ggggagcaat 3120
   ggcgatgacg catcctcacg ataatatccg ggtaggcgca atcactttcg tctactccgt 3180
   tacaaagcga ggctgggtat ttcccggcct ttctgttatc cgaaatccac tgaaagcaca 3240
   gcggctggct gaggagataa ataataaacg aggggctgta tgcacaaagc atcttctgtt 3300
   gagttaagaa cgagtatcga gatggcacat agccttgctc aaattggaat caggtttgtg 3360
   ccaataccag tagaaacaga cgaagaatcc atgggtatgg acagttttcc ctttgatatg 3420
   taacggtgaa cagttgttct acttttgttt gttagtcttg atgcttcact gatagataca 3480
   agagccataa gaacctcaga tccttccgta tttagccagt atgttctcta gtgtggttcg 3540
   ttgtttttgc gtgagccatg agaacgaacc attgagatca tacttacttt gcatgtcact 3600
   caaaaatttt gcctcaaaac tggtgagctg aatttttgca gttaaagcat cgtgtagtgt 3660
   ttttcttagt ccgttacgta ggtaggaatc tgatgtaatg gttgttggta ttttgtcacc 3720
   attcattttt atctggttgt tctcaagttc ggttacgaga tccatttgtc tatctagttc 3780
   aacttggaaa atcaacgtat cagtcgggcg gcctcgctta tcaaccacca atttcatatt 3840
   gctgtaagtg tttaaatctt tacttattgg tttcaaaacc cattggttaa gccttttaaa 3900
   ctcatggtag ttattttcaa gcattaacat gaacttaaat tcatcaaggc taatctctat 3960
   atttgccttg tgagttttct tttgtgttag ttcttttaat aaccactcat aaatcctcat 4020
   agagtatttg ttttcaaaag acttaacatg ttccagatta tattttatga atttttttaa 4080
   ctggaaaaga taaggcaata tctcttcact aaaaactaat tctaattttt cgcttgagaa 4140
   cttggcatag tttgtccact ggaaaatctc aaagccttta accaaaggat tcctgatttc 4200
   cacagttctc gtcatcagct ctctggttgc tttagctaat acaccataag cattttccct 4260
   actgatgttc atcatctgag cgtattggtt ataagtgaac gataccgtcc gttctttcct 4320
   tgtagggttt tcaatcgtgg ggttgagtag tgccacacag cataaaatta gcttggtttc 4380
   atgctccgtt aagtcatagc gactaatcgc tagttcattt gctttgaaaa caactaattc 4440
   agacatacat ctcaattggt ctaggtgatt ttaatcacta taccaattga gatgggctag 4500
   tcaatgataa ttactagtcc ttttcctttg agttgtgggt atctgtaaat tctgctagac 4560
   ctttgctgga aaacttgtaa attctgctag accctctgta aattccgcta gacctttgtg 4620
   tgtttttttt gtttatattc aagtggttat aatttataga ataaagaaag aataaaaaaa 4680
   gataaaaaga atagatccca gccctgtgta taactcacta ctttagtcag ttccgcagta 4740
   ttacaaaagg atgtcgcaaa cgctgtttgc tcctctacaa aacagacctt aaaaccctaa 4800
   aggcttaagt agcaccctcg caagctcggt tgcggccgca atcgggcaaa tcgctgaata 4860
   ttccttttgt ctccgaccat caggcacctg agtcgctgtc tttttcgtga cattcagttc 4920
   gctgcgctca cggctctggc agtgaatggg ggtaaatggc actacaggcg ccttttatgg 4980
   attcatgcaa ggaaactacc cataatacaa gaaaagcccg tcacgggctt ctcagggcgt 5040
   tttatggcgg gtctgctatg tggtgctatc tgactttttg ctgttcagca gttcctgccc 5100
   tctgattttc cagtctgacc acttcggatt atcccgtgac aggtcattca gactggctaa 5160
   tgcacccagt aaggcagcgg tatcatcaac ggggtctgac gctcagtgga acgaaaactc 5220
   acgttaaggg attttggtca tgagattatc aaaaaggatc ttcacctaga tccttttaaa 5280
   ttaaaaatga agttttaaat caatctaaag tatatatgag taaacttggt ctgacagtta 5340
   ccaatgctta atcagtgagg cacctatctc agcgatctgt ctatttcgtt catccatagt 5400
   tgcctgactc cccgtcgtgt agataactac gatacgggag ggcttaccat ctggccccag 5460
   tgctgcaatg ataccgcgag acccacgctc accggctcca gatttatcag caataaacca 5520
   gccagccgga agggccgagc gcagaagtgg tcctgcaact ttatccgcct ccatccagtc 5580
   tattaattgt tgccgggaag ctagagtaag tagttcgcca gttaatagtt tgcgcaacgt 5640
   tgttgccatt gctacaggca tcgtggtgtc acgctcgtcg tttggtatgg cttcattcag 5700
   ctccggttcc caacgatcaa ggcgagttac atgatccccc atgttgtgca aaaaagcggt 5760
   tagctccttc ggtcctccga tcgttgtcag aagtaagttg gccgcagtgt tatcactcat 5820
   ggttatggca gcactgcata attctcttac tgtcatgcca tccgtaagat gcttttctgt 5880
   gactggtgag tactcaacca agtcattctg agaatagtgt atgcggcgac cgagttgctc 5940
   ttgcccggcg tcaatacggg ataataccgc gccacatagc agaactttaa aagtgctcat 6000
   cattggaaaa cgttcttcgg ggcgaaaact ctcaaggatc ttaccgctgt tgagatccag 6060
   ttcgatgtaa cccactcgtg cacccaactg atcttcagca tcttttactt tcaccagcgt 6120
   ttctgggtga gcaaaaacag gaaggcaaaa tgccgcaaaa aagggaataa gggcgacacg 6180
   gaaatgttga atactcatac tcttcctttt tcaatattat tgaagcattt atcagggtta 6240
   ttgtctcatg agcggataca tatttgaatg tatttagaaa aataaacaaa taggggttcc 6300
   gcgcacattt ccccgaaaag tgccacctg 6329
<210> 50
   <211> 25
   <212> DNA
   <213> artificial sequence
<220>
   <223> Primer
<400> 50
   aacaatatgt aagatctcaa ctatc 25
<210> 51
   <211> 25
   <212> DNA
   <213> artificial sequence
<220>
   <223> Primer
<400> 51
   cagacatgag agatccagtg tgtag 25
<210> 52
   <211> 9332
   <212> DNA
   <213> artificial sequence
<220>
   <223> Plasmid pCP20
<400> 52
   gagacacaac gtggctttgt tgaataaatc gaacttttgc tgagttgaag gatcagatca      60
   cgcatcttcc cgacaacgca gaccgttccg tggcaaagca aaagttcaaa atcaccaact     120
   ggtccaccta caacaaagct ctcatcaacc gtggctccct cactttctgg ctggatgatg     180
   gggcgattca ggcctggtat gagtcagcaa caccttcttc acgaggcaga cctcagcgcc     240
   acaggtgcgg ttgctggcgc taaccgtttt tatcaggctc tgggaggcag aataaatgat     300
   catatcgtca attattacct ccacggggag agcctgagca aactggcctc aggcatttga     360
   gaagcacacg gtcacactgc ttccggtagt caataaaccg gtaaaccagc aatagacata     420
   agcggctatt taacgaccct gccctgaacc gacgaccggg tcgaatttgc tttcgaattt     480
   ctgccattca tccgcttatt atcacttatt caggcgtagc aaccaggcgt ttaagggcac     540
   caataactgc cttaaaaaaa ttacgccccg ccctgccact catcgcagta ctgttgtaat     600
   tcattaagca ttctgccgac atggaagcca tcacaaacgg catgatgaac ctgaatcgcc 660
   agcggcatca gcaccttgtc gccttgcgta taatatttgc ccatggtgaa aacgggggcg 720
   aagaagttgt ccatattggc cacgtttaaa tcaaaactgg tgaaactcac ccagggattg 780
   gctgagacga aaaacatatt ctcaataaac cctttaggga aataggccag gttttcaccg 840
   taacacgcca catcttgcga atatatgtgt agaaactgcc ggaaatcgtc gtggtattca 900
   ctccagagcg atgaaaacgt ttcagtttgc tcatggaaaa cggtgtaaca agggtgaaca 960
   ctatcccata tcaccagctc accgtctttc attgccatac ggaattccgg atgagcattc 1020
   atcaggcggg caagaatgtg aataaaggcc ggataaaact tgtgcttatt tttctttacg 1080
   gtctttaaaa aggccgtaat atccagctga acggtctggt tataggtaca ttgagcaact 1140
   gactgaaatg cctcaaaatg ttctttacga tgccattggg atatatcaac ggtggtatat 1200
   ccagtgattt ttttctccat tttagcttcc ttagctcctg aaaatctcga taactcaaaa 1260
   aatacgcccg gtagtgatct tatttcatta tggtgaaagt tggaacctct tacgtgccga 1320
   tcaacgtctc attttcgcca aaagttggcc cagggcttcc cggtatcaac agggacacca 1380
   ggatttattt attctgcgaa gtgatcttcc gtcacaggta tttattcggc gcaaagtgcg 1440
   tcgggtgatg ctgccaactt actgatttag tgtatgatgg tgtttttgag gtgctccagt 1500
   ggcttctgtt tctatcagct gtccctcctg ttcagctact gacggggtgg tgcgtaacgg 1560
   caaaagcacc gccggacatc agcgcttgtt tcggcgtggg tatggtggca ggccccgtgg 1620
   ccgggggact gttgggcgcc tgtagtgcca tttaccccca ttcactgcca gagccgtgag 1680
   cgcagcgaac tgaatgtcac gaaaaagaca gcgactcagg tgcctgatgg tcggagacaa 1740
   aaggaatatt cagcgatttg cccgagcttg cgagggtgct acttaagcct ttagggtttt 1800
   aaggtctgtt ttgtagagga gcaaacagcg tttgcgacat ccttttgtaa tactgcggaa 1860
   ctgactaaag tagtgagtta tacacagggc tgggatctat tctttttatc tttttttatt 1920
   ctttctttat tctataaatt ataaccactt gaatataaac aaaaaaaaca cacaaaggtc 1980
   tagcggaatt tacagagggt ctagcagaat ttacaagttt tccagcaaag gtctagcaga 2040
   atttacagat acccacaact caaaggaaaa ggactagtaa ttatcattga ctagcccatc 2100
   tcaattggta tagtgattaa aatcacctag accaattgag atgtatgtct gaattagttg 2160
   ttttcaaagc aaatgaacta gcgattagtc gctatgactt aacggagcat gaaaccaagc 2220
   taattttatg ctgtgtggca ctactcaacc ccacgattga aaaccctaca aggaaagaac 2280
   ggacggtatc gttcacttat aaccaatacg ttcagatgat gaacatcagt agggaaaatg 2340
   cttatggtgt attagctaaa gcaaccagag agctgatgac gagaactgtg gaaatcagga 2400
   atcctttggt taaaggcttt gagattttcc agtggacaaa ctatgccaag ttctcaagcg 2460
   aaaaattaga attagttttt agtgaagaga tattgcctta tcttttccag ttaaaaaaat 2520
   tcataaaata taatctggaa catgttaagt cttttgaaaa caaatactct atgaggattt 2580
   atgagtggtt attaaaagaa ctaacacaaa agaaaactca caaggcaaat atagagatta 2640
   gccttgatga atttaagttc atgttaatgc ttgaaaataa ctaccatgag tttaaaaggc 2700
   ttaaccaatg ggttttgaaa ccaataagta aagatttaaa cacttacagc aatatgaaat 2760
   tggtggttga taagcgaggc cgcccgactg atacgttgat tttccaagtt gaactagata 2820
   gacaaatgga tctcgtaacc gaacttgaga acaaccagat aaaaatgaat ggtgacaaaa 2880
   taccaacaac cattacatca gattcctacc tacataacgg actaagaaaa acactacacg 2940
   atgctttaac tgcaaaaatt cagctcacca gttttgaggc aaaatttttg agtgacatgc 3000
   aaagtaagta tgatctcaat ggttcgttct catggctcac gcaaaaacaa cgaaccacac 3060
   tagagaacat actggctaaa tacggaagga tctgaggttc ttatggctct tgtatctatc 3120
   agtgaagcat caagactaac aaacaaaagt agaacaactg ttcaccgtta catatcaaag 3180
   ggaaaactgt ccatatgcac agatgaaaac ggtgtaaaaa agatagatac atcagagctt 3240
   ttacgagttt ttggtgcatt taaagctgtt caccatgaac agatcgacaa tgtaacagat 3300
   gaacagcatg taacacctaa tagaacaggt gaaaccagta aaacaaagca actagaacat 3360
   gaaattgaac acctgagaca acttgttaca gctcaacagt cacacataga cagcctgaaa 3420
   caggcgatgc tgcttatcga atcaaagctg ccgacaacac gggagccagt gacgcctccc 3480
   gtggggaaaa aatcatggca attctggaag aaatagcgcc tgtttcgttt caggcaggtt 3540
   atcagggagt gtcagcgtcc tgcggttctc cggggcgttc gggtcatgca gcccgtaatg 3600
   gtgatttacc agcgtctgcc aggcatcaat tctaggcctg tctgcgcggt cgtagtacgg 3660
   ctggaggcgt tttccggtct gtagctccat gttcggaatg acaaaattca gctcaagccg 3720
   tcccttgtcc tggtgctcca cccacaggat gctgtactga tttttttcga gaccgggcat 3780
   cagtacacgc tcaaagctcg ccatcacttt ttcacgtcct cccggcggca gctccttctc 3840
   cgcgaacgac agaacaccgg acgtgtattt cttcgcaaat ggcgtggcat cgatgagttc 3900
   ccggacttct tccggattac cctgaagcac cgttgcgcct tcgcggttac gctccctccc 3960
   cagcaggtaa tcaaccggac cactgccacc accttttccc ctggcatgaa atttaactat 4020
   catcccgcgc cccctgttcc ctgacagcca gacgcagccg gcgcagctca tccccgatgg 4080
   ccatcagtgc ggccaccacc tgaacccggt caccggaaga ccactgcccg ctgttcacct 4140
   tacgggctgt ctgattcagg ttatttccga tggcggccag ctgacgcagt aacggcggtg 4200
   ccagtgtcgg cagttttccg gaacgggcaa ccggctcccc caggcagacc cgccgcatcc 4260
   ataccgccag ttgtttaccc tcacagcgtt caagtaaccg ggcatgttca tcatcagtaa 4320
   cccgtattgt gagcatcctc tcgcgtttca tcggtatcat taccccatga acagaaatcc 4380
   cccttacacg gaggcatcag tgactaaacg ggtctgacgc tcagtggaac cgaaaactca 4440
   cgttaaggga ttttggtcat gagattatca aaaaggatct tcacctagat ccttttaaat 4500
   taaaaatgaa gttttaaatc aatctaaagt atatatgagt aaacttggtc tgacagttac 4560
   caatgcttaa tcagtgaggc acctatctca gcgatctgtc tatttcgttc atccatagtt 4620
   gcctgactcc ccgtcgtgta gataactacg atacgggagg gcttaccatc tggccccagt 4680
   gctgcaatga taccgcgaga cccacgctca ccggctccag atttatcagc aataaaccag 4740
   ccagccggaa gggccgagcg cagaagtggt cctgcaactt tatccgcctc catccagtct 4800
   attaattgtt gccgggaagc tagagtaagt agttcgccag ttaatagttt gcgcaacgtt 4860
   gttgccattg ctgcaggcat cgtggtgtca cgctcgtcgt ttggtatggc ttcattcagc 4920
   tccggttccc aacgatcaag gcgagttaca tgatccccca tgttgtgcaa aaaagcggtt 4980
   agctccttcg gtcctccgat cgttgtcaga agtaagttgg ccgcagtgtt atcactcatg 5040
   gttatggcag cactgcataa ttctcttact gtcatgccat ccgtaagatg cttttctgtg 5100
   actggtgagt actcaaccaa gtcattctga gaatagtgta tgcggcgacc gagttgctct 5160
   tgcccggcgt caacacggga taataccgcg ccacatagca gaactttaaa agtgctcatc 5220
   attggaaaac gttcttcggg gcgaaaactc tcaaggatct taccgctgtt gagatccagt 5280
   tcgatgtaac ccactcgtgc acccaactga tcttcagcat cttttacttt caccagcgtt 5340
   tctgggtgag caaaaacagg aaggcaaaat gccgcaaaaa agggaataag ggcgacacgg 5400
   aaatgttgaa tactcatact cttccttttt caatattatt gaagcattta tcagggttat 5460
   tgtctcatga gcggatacat atttgaatgt atttagaaaa ataaacaaat aggggttccg 5520
   cgcacatttc cccgaaaagt gccacctgac gtctaagaaa ccattattat catgacatta 5580
   acctataaaa ataggcgtat cacgaggccc tttcgtcttc aagaatttta taaaccgtgg 5640
   agcgggcaat actgagctga tgagcaattt ccgttgcacc agtgcccttc tgatgaagcg 5700
   tcagcacgac gttcctgtcc acggtacgcc tgcggccaaa tttgattcct ttcagctttg 5760
   cttcctgtcg gccctcattc gtgcgctcta ggatcctcta cgccggacgc atcgtggccg 5820
   gcatcaccgg cgctgaggtc tgcctcgtga agaaggtgtt gctgactcat accaggcctg 5880
   aatcgcccca tcatccagcc agaaagtgag ggagccacgg ttgatgagag ctttgttgta 5940
   ggtggaccag ttggtgattt tgaacttttg ctttgccacg gaacggtctg cgttgtcggg 6000
   aagatgcgtg atctgatcct tcaactcagc aaaagttcga tttattcaac aaagccgccg 6060
   tcccgtcaag tcagcgtaat gctctgccag tgttacaacc aattaaccaa ttctgattag 6120
   aaaaactcat cgagcatcaa atgaaactgc aatttattca tatcaggatt atcaatacca 6180
   tatttttgaa aaagccgttt ctgtaatgaa ggagaaaact caccgaggca gttccatagg 6240
   atggcaagat cctggtatcg gtctgcgatt ccgactcgtc caacatcaat acaacctatt 6300
   aatttcccct cgtcaaaaat aaggttatca agtgagaaat caccatgagt gacgactgaa 6360
   tccggtgaga atggcagaat aggaacttcg gaataggaac ttcaaagcgt ttccgaaaac 6420
   gagcgcttcc gaaaatgcaa cgcgagctgc gcacatacag ctcactgttc acgtcgcacc 6480
   tatatctgcg tgttgcctgt atatatatat acatgagaag aacggcatag tgcgtgttta 6540
   tgcttaaatg cgtacttata tgcgtctatt tatgtaggat gaaaggtagt ctagtacctc 6600
   ctgtgatatt atcccattcc atgcggggta tcgtatgctt ccttcagcac taccctttag 6660
   ctgttctata tgctgccact cctcaattgg attagtctca tccttcaatg ctatcatttc 6720
   ctttgatatt ggatcatatg catagtaccg agaaactagt gcgaagtagt gatcaggtat 6780
   tgctgttatc tgatgagtat acgttgtcct ggccacggca gaagcacgct tatcgctcca 6840
   atttcccaca acattagtca actccgttag gcccttcatt gaaagaaatg aggtcatcaa 6900
   atgtcttcca atgtgagatt ttgggccatt ttttatagca aagattgaat aaggcgcatt 6960
   tttcttcaaa gctttattgt acgatctgac taagttatct tttaataatt ggtattcctg 7020
   tttattgctt gaagaattgc cggtcctatt tactcgtttt aggactggtt cagaattcct 7080
   caaaaattca tccaaatata caagtggatc gatcctaccc cttgcgctaa agaagtatat 7140
   gtgcctacta acgcttgtct ttgtctctgt cactaaacac tggattatta ctcccagata 7200
   cttattttgg actaatttaa atgatttcgg atcaacgttc ttaatatcgc tgaatcttcc 7260
   acaattgatg aaagtagcta ggaagaggaa ttggtataaa gtttttgttt ttgtaaatct 7320
   cgaagtatac tcaaacgaat ttagtatttt ctcagtgatc tcccagatgc tttcaccctc 7380
   acttagaagt gctttaagca tttttttact gtggctattt cccttatctg cttcttccga 7440
   tgattcgaac tgtaattgca aactacttac aatatcagtg atatcagatt gatgtttttg 7500
   tccatagtaa ggaataattg taaattccca agcaggaatc aatttcttta atgaggcttc 7560
   cagaattgtt gctttttgcg tcttgtattt aaactggagt gatttattga caatatcgaa 7620
   actcagcgaa ttgcttatga tagtattata gctcatgaat gtggctctct tgattgctgt 7680
   tccgttatgt gtaatcatcc aacataaata ggttagttca gcagcacata atgctatttt 7740
   ctcacctgaa ggtctttcaa acctttccac aaactgacga acaagcacct taggtggtgt 7800
   tttacataat atatcaaatt gtggcataca acctccttag tacatgcaac cattatcacc 7860
   gccagaggta aaatagtcaa cacgcacggt gttagatatt tatcccttgc ggtgatagat 7920
   ttaacgtatg agcacaaaaa agaaaccatt aacacaagag cagcttgagg acgcacgtcg 7980
   ccttaaagca atttatgaaa aaaagaaaaa tgaacttggc ttatcccagg aatctgtcgc 8040
   agacaagatg gggatggggc agtcaggcgt tggtgcttta tttaatggca tcaatgcatt 8100
   aaatgcttat aacgccgcat tgcttacaaa aattctcaaa gttagcgttg aagaatttag 8160
   cccttcaatc gccagagaaa tctacgagat gtatgaagcg gttagtatgc agccgtcact 8220
   tagaagtgag tatgagtacc ctgttttttc tcatgttcag gcagggatgt tctcacctaa 8280
   gcttagaacc tttaccaaag gtgatgcgga gagatgggta agcacaacca aaaaagccag 8340
   tgattctgca ttctggcttg aggttgaagg taattccatg accgcaccaa caggctccaa 8400
   gccaagcttt cctgacggaa tgttaattct cgttgaccct gagcaggctg ttgagccagg 8460
   tgatttctgc atagccagac ttgggggtga tgagtttacc ttcaagaaac tgatcaggga 8520
   tagcggtcag gtgtttttac aaccactaaa cccacagtac ccaatgatcc catgcaatga 8580
   gagttgttcc gttgtgggga aagttatcgc tagtcagtgg cctgaagaga cgtttggctg 8640
   atcggcaagg tgttctggtc ggcgcatagc tgataacaat tgagcaagaa tctgcatttc 8700
   tttccagact tgttcaacag gccagccatt acgctcgtca tcaaaatcac tcgcatcaac 8760
   caaaccgtta ttcattcgtg attgcgcctg agcgagacga aatacgcgat cgctgttaaa 8820
   aggacaatta caaacaggaa tcgaatgcaa ccggcgcagg aacactgcca gcgcatcaac 8880
   aatattttca cctgaatcag gatattcttc taatacctgg aatgctgttt tcccggggat 8940
   cgcagtggtg agtaaccatg catcatcagg agtacggata aaatgcttga tggtcggaag 9000
   aggcataaat tccgtcagcc agtttagtct gaccatctca tctgtaacat cattggcaac 9060
   gctacctttg ccatgtttca gaaacaactc tggcgcatcg ggcttcccat acaatcgata 9120
   gattgtcgca cctgattgcc cgacattatc gcgagcccat ttatacccat ataaatcagc 9180
   atccatgttg gaatttaatc gcggcctcga gcaagacgtt tcccgttgaa tatggctcat 9240
   aacacccctt gtattactgt ttatgtaagc agacagtttt attgttcatg atgatatatt 9300
   tttatcttgt gcaatgtaac atcagagatt tt 9332
<210> 53
   <211> 80
   <212> DNA
   <213> artificial sequence
<220>
   <223> Primer
<400> 53
   atgtcgcaac ataacgaaaa gaacccacat cagcaccagt caccactaca cgattccagc 60
   gtgtaggctg gagctgcttc 80
<210> 54
   <211> 82
   <212> DNA
   <213> artificial sequence
<220>
   <223> Primer
<400> 54
   ttacgccggg attttgtcaa tcttaggaat gcgtgaccac acgcggtgtg ctgtcatcag 60
   attccgggga tccgtcgacc tg 82
<210> 55
   <211> 1424
   <212> DNA
   <213> artificial sequence
<220>
   <223> Synthetic construct
<400> 55
   ttacgccggg attttgtcaa tcttaggaat gcgtgaccac acgcggtgtg ctgtcatcag 60
   attccgggga tccgtcgacc tgcagttcga agttcctatt ctctagaaag tataggaact 120
   tcagagcgct tttgaagctc acgctgccgc aagcactcag ggcgcaaggg ctgctaaagg 180
   aagcggaaca cgtagaaagc cagtccgcag aaacggtgct gaccccggat gaatgtcagc 240
   tactgggcta tctggacaag ggaaaacgca agcgcaaaga gaaagcaggt agcttgcagt 300
   gggcttacat ggcgatagct agactgggcg gttttatgga cagcaagcga accggaattg 360
   ccagctgggg cgccctctgg taaggttggg aagccctgca aagtaaactg gatggctttc 420
   ttgccgccaa ggatctgatg gcgcagggga tcaagatctg atcaagagac aggatgagga 480
   tcgtttcgca tgattgaaca agatggattg cacgcaggtt ctccggccgc ttgggtggag 540
   aggctattcg gctatgactg ggcacaacag acaatcggct gctctgatgc cgccgtgttc 600
   cggctgtcag cgcaggggcg cccggttctt tttgtcaaga ccgacctgtc cggtgccctg 660
   aatgaactgc aggacgaggc agcgcggcta tcgtggctgg ccacgacggg cgttccttgc 720
   gcagctgtgc tcgacgttgt cactgaagcg ggaagggact ggctgctatt gggcgaagtg 780
   ccggggcagg atctcctgtc atctcacctt gctcctgccg agaaagtatc catcatggct 840
   gatgcaatgc ggcggctgca tacgcttgat ccggctacct gcccattcga ccaccaagcg 900
   aaacatcgca tcgagcgagc acgtactcgg atggaagccg gtcttgtcga tcaggatgat 960
   ctggacgaag agcatcaggg gctcgcgcca gccgaactgt tcgccaggct caaggcgcgc 1020
   atgcccgacg gcgaggatct cgtcgtgacc catggcgatg cctgcttgcc gaatatcatg 1080
   gtggaaaatg gccgcttttc tggattcatc gactgtggcc ggctgggtgt ggcggaccgc 1140
   tatcaggaca tagcgttggc tacccgtgat attgctgaag agcttggcgg cgaatgggct 1200
   gaccgcttcc tcgtgcttta cggtatcgcc gctcccgatt cgcagcgcat cgccttctat 1260
   cgccttcttg acgagttctt ctaataaggg gatcttgaag ttcctattcc gaagttccta 1320
   ttctctagaa agtataggaa cttcgaagca gctccagcct acacgctgga atcgtgtagt 1380
   ggtgactggt gctgatgtgg gttcttttcg ttatgttgcg acat 1424
<210> 56
   <211> 2262
   <212> DNA
   <213> Escherichia coli
<220>
   <221> CDS
   <222> (1)..(2262)
<400> 56
   atg tcg caa cat aac gaa aag aac cca cat cag cac cag tca cca cta 48
   Met Ser Gln His Asn Glu Lys Asn Pro His Gln His Gln Ser Pro Leu
   1 5 10 15
   cac gat tcc agc gaa gcg aaa ccg ggg atg gac tca ctg gca cct gag 96
   His Asp Ser Ser Glu Ala Lys Pro Gly Met Asp Ser Leu Ala Pro Glu
   20 25 30
   gac ggc tct cat cgt cca gcg gct gaa cca aca ccg cca ggt gca caa 144
   Asp Gly Ser His Arg Pro Ala Ala Glu Pro Thr Pro Pro Gly Ala Gln
   35 40 45
   cct acc gcc cca ggg agc ctg aaa gcc cct gat acg cgt aac gaa aaa 192
   Pro Thr Ala Pro Gly Ser Leu Lys Ala Pro Asp Thr Arg Asn Glu Lys
   50 55 60
   ctt aat tct ctg gaa gac gta cgc aaa ggc agt gaa aat tat gcg ctg 240
   Leu Asn Ser Leu Glu Asp Val Arg Lys Gly Ser Glu Asn Tyr Ala Leu
   65 70 75 80
   acc act aat cag ggc gtg cgc atc gcc gac gat caa aac tca ctg cgt 288
   Thr Thr Asn Gln Gly Val Arg Ile Ala Asp Asp Gln Asn Ser Leu Arg
   85 90 95
   gcc ggt agc cgt ggt cca acg ctg ctg gaa gat ttt att ctg cgc gag 336
   Ala Gly Ser Arg Gly Pro Thr Leu Leu Glu Asp Phe Ile Leu Arg Glu
   100 105 110
   aaa atc acc cac ttt gac cat gag cgc att ccg gaa cgt att gtt cat 384
   Lys Ile Thr His Phe Asp His Glu Arg Ile Pro Glu Arg Ile Val His
   115 120 125
   gca cgc gga tca gcc gct cac ggt tat ttc cag cca tat aaa agc tta 432
   Ala Arg Gly Ser Ala Ala His Gly Tyr Phe Gln Pro Tyr Lys Ser Leu
   130 135 140
   agc gat att acc aaa gcg gat ttc ctc tca gat ccg aac aaa atc acc 480
   Ser Asp Ile Thr Lys Ala Asp Phe Leu Ser Asp Pro Asn Lys Ile Thr
   145 150 155 160
   cca gta ttt gta cgt ttc tct acc gtt cag ggt ggt gct ggc tct gct 528
   Pro Val Phe Val Arg Phe Ser Thr Val Gln Gly Gly Ala Gly Ser Ala
   165 170 175
   gat acc gtg cgt gat atc cgt ggc ttt gcc acc aag ttc tat acc gaa 576
   Asp Thr Val Arg Asp Ile Arg Gly Phe Ala Thr Lys Phe Tyr Thr Glu
   180 185 190
   gag ggt att ttt gac ctc gtt ggc aat aac acg cca atc ttc ttt atc 624
   Glu Gly Ile Phe Asp Leu Val Gly Asn Asn Thr Pro Ile Phe Phe Ile
   195 200 205
   cag gat gcg cat aaa ttc ccc gat ttt gtt cat gcg gta aaa cca gaa 672
   Gln Asp Ala His Lys Phe Pro Asp Phe Val His Ala Val Lys Pro Glu
   210 215 220
   ccg cac tgg gca att cca caa ggg caa agt gcc cac gat act ttc tgg 720
   Pro His Trp Ala Ile Pro Gln Gly Gln Ser Ala His Asp Thr Phe Trp
   225 230 235 240
   gat tat gtt tct ctg caa cct gaa act ctg cac aac gtg atg tgg gcg 768
   Asp Tyr Val Ser Leu Gln Pro Glu Thr Leu His Asn Val Met Trp Ala
   245 250 255
   atg tcg gat cgc ggc atc ccc cgc agt tac cgc acc atg gaa ggc ttc      816
   Met Ser Asp Arg Gly Ile Pro Arg Ser Tyr Arg Thr Met Glu Gly Phe
               260                 265                 270
   ggt att cac acc ttc cgc ctg att aat gcc gaa ggg aag gca acg ttt      864
   Gly Ile His Thr Phe Arg Leu Ile Asn Ala Glu Gly Lys Ala Thr Phe
           275                 280                 285
   gta cgt ttc cac tgg aaa cca ctg gca ggt aaa gcc tca ctc gtt tgg      912
   Val Arg Phe His Trp Lys Pro Leu Ala Gly Lys Ala Ser Leu Val Trp
       290                 295                 300
   gat gaa gca caa aaa ctc acc gga cgt gac ccg gac ttc cac cgc cgc      960
   Asp Glu Ala Gln Lys Leu Thr Gly Arg Asp Pro Asp Phe His Arg Arg
   305                 310                 315                 320
   gag ttg tgg gaa gcc att gaa gca ggc gat ttt ccg gaa tac gaa ctg     1008
   Glu Leu Trp Glu Ala Ile Glu Ala Gly Asp Phe Pro Glu Tyr Glu Leu
                   325                 330                 335
   ggc ttc cag ttg att cct gaa gaa gat gaa ttc aag ttc gac ttc gat     1056
   Gly Phe Gln Leu Ile Pro Glu Glu Asp Glu Phe Lys Phe Asp Phe Asp
               340                 345                 350
   ctt ctc gat cca acc aaa ctt atc ccg gaa gaa ctg gtg ccc gtt cag     1104
   Leu Leu Asp Pro Thr Lys Leu Ile Pro Glu Glu Leu Val Pro Val Gln
           355                 360                 365
   cgt gtc ggc aaa atg gtg ctc aat cgc aac ccg gat aac ttc ttt gct     1152
   Arg Val Gly Lys Met Val Leu Asn Arg Asn Pro Asp Asn Phe Phe Ala
       370                 375                 380
   gaa aac gaa cag gcg gct ttc cat cct ggg cat atc gtg ccg gga ctg     1200
   Glu Asn Glu Gln Ala Ala Phe His Pro Gly His Ile Val Pro Gly Leu
   385                 390                 395                 400
   gac ttc acc aac gat ccg ctg ttg cag gga cgt ttg ttc tcc tat acc     1248
   Asp Phe Thr Asn Asp Pro Leu Leu Gln Gly Arg Leu Phe Ser Tyr Thr
                   405                 410                 415
   gat aca caa atc agt cgt ctt ggt ggg ccg aat ttc cat gag att ccg     1296
   Asp Thr Gln Ile Ser Arg Leu Gly Gly Pro Asn Phe His Glu Ile Pro
               420                 425                 430
   att aac cgt ccg acc tgc cct tac cat aat ttc cag cgt gac ggc atg     1344
   Ile Asn Arg Pro Thr Cys Pro Tyr His Asn Phe Gln Arg Asp Gly Met
           435                 440                 445
   cat cgc atg ggg atc gac act aac ccg gcg aat tac gaa ccg aac tcg     1392
   His Arg Met Gly Ile Asp Thr Asn Pro Ala Asn Tyr Glu Pro Asn Ser
       450                 455                 460
   att aac gat aac tgg ccg cgc gaa aca ccg ccg ggg ccg aaa cgc ggc     1440
   Ile Asn Asp Asn Trp Pro Arg Glu Thr Pro Pro Gly Pro Lys Arg Gly
   465                 470                 475                 480
   ggt ttt gaa tca tac cag gag cgc gtg gaa ggc aat aaa gtt cgc gag     1488
   Gly Phe Glu Ser Tyr Gln Glu Arg Val Glu Gly Asn Lys Val Arg Glu
                   485                 490                 495
   cgc agc cca tcg ttt ggc gaa tat tat tcc cat ccg cgt ctg ttc tgg     1536
   Arg Ser Pro Ser Phe Gly Glu Tyr Tyr Ser His Pro Arg Leu Phe Trp
               500                 505                 510
   cta agt cag acg cca ttt gag cag cgc cat att gtc gat ggt ttc agt     1584
   Leu Ser Gln Thr Pro Phe Glu Gln Arg His Ile Val Asp Gly Phe Ser
             515                 520                 525
   ttt gag tta agc aaa gtc gtt cgt ccg tat att cgt gag cgc gtt gtt      1632
   Phe Glu Leu Ser Lys Val Val Arg Pro Tyr Ile Arg Glu Arg Val Val
        530                 535                 540
   gac cag ctg gcg cat att gat ctc act ctg gcc cag gcg gtg gcg aaa      1680
   Asp Gln Leu Ala His Ile Asp Leu Thr Leu Ala Gln Ala Val Ala Lys
   545                 550                 555                 560
   aat ctc ggt atc gaa ctg act gac gac cag ctg aat atc acc cca cct      1728
   Asn Leu Gly Ile Glu Leu Thr Asp Asp Gln Leu Asn Ile Thr Pro Pro
                   565                 570                 575
   ccg gac gtc aac ggt ctg aaa aag gat cca tcc tta agt ttg tac gcc      1776
   Pro Asp Val Asn Gly Leu Lys Lys Asp Pro Ser Leu Ser Leu Tyr Ala
               580                 585                 590
   att cct gac ggt gat gtg aaa ggt cgc gtg gta gcg att tta ctt aat      1824
   Ile Pro Asp Gly Asp Val Lys Gly Arg Val Val Ala Ile Leu Leu Asn
           595                 600                 605
   gat gaa gtg aga tcg gca gac ctt ctg gcc att ctc aag gcg ctg aag      1872
   Asp Glu Val Arg Ser Ala Asp Leu Leu Ala Ile Leu Lys Ala Leu Lys
       610                 615                 620
   gcc aaa ggc gtt cat gcc aaa ctg ctc tac tcc cga atg ggt gaa gtg      1920
   Ala Lys Gly Val His Ala Lys Leu Leu Tyr Ser Arg Met Gly Glu Val
   625                 630                 635                 640
   act gcg gat gac ggt acg gtg ttg cct ata gcc gct acc ttt gcc ggt      1968
   Thr Ala Asp Asp Gly Thr Val Leu Pro Ile Ala Ala Thr Phe Ala Gly
                   645                 650                 655
   gca cct tcg ctg acg gtc gat gcg gtc att gtc cct tgc ggc aat atc      2016
   Ala Pro Ser Leu Thr Val Asp Ala Val Ile Val Pro Cys Gly Asn Ile
               660                 665                 670
   gcg gat atc gct gac aac ggc gat gcc aac tac tac ctg atg gaa gcc      2064
   Ala Asp Ile Ala Asp Asn Gly Asp Ala Asn Tyr Tyr Leu Met Glu Ala
           675                 680                 685
   tac aaa cac ctt aaa ccg att gcg ctg gcg ggt gac gcg cgc aag ttt      2112
   Tyr Lys His Leu Lys Pro Ile Ala Leu Ala Gly Asp Ala Arg Lys Phe
       690                 695                 700
   aaa gca aca atc aag atc gct gac cag ggt gaa gaa ggg att gtg gaa      2160
   Lys Ala Thr Ile Lys Ile Ala Asp Gln Gly Glu Glu Gly Ile Val Glu
   705                 710                 715                 720
   gct gac agc gct gac ggt agt ttt atg gat gaa ctg cta acg ctg atg      2208
   Ala Asp Ser Ala Asp Gly Ser Phe Met Asp Glu Leu Leu Thr Leu Met
                   725                 730                 735
   gca gca cac cgc gtg tgg tca cgc att cct aag att gac aaa att cct      2256
   Ala Ala His Arg Val Trp Ser Arg Ile Pro Lys Ile Asp Lys Ile Pro
               740                 745                 750
   gcc tga                                                              2262
   Ala
<210> 57
   <211> 753
   <212> PRT
   <213> Escherichia coli
<400> 57
   Met Ser Gln His Asn Glu Lys Asn Pro His Gln His Gln Ser Pro Leu
   1               5                   10                  15
   His Asp Ser Ser Glu Ala Lys Pro Gly Met Asp Ser Leu Ala Pro Glu
               20                  25                  30
   Asp Gly Ser His Arg Pro Ala Ala Glu Pro Thr Pro Pro Gly Ala Gln
           35                  40                  45
   Pro Thr Ala Pro Gly Ser Leu Lys Ala Pro Asp Thr Arg Asn Glu Lys
       50                  55                  60
   Leu Asn Ser Leu Glu Asp Val Arg Lys Gly Ser Glu Asn Tyr Ala Leu
   65                  70                  75                  80
   Thr Thr Asn Gln Gly Val Arg Ile Ala Asp Asp Gln Asn Ser Leu Arg
                   85                  90                  95
   Ala Gly Ser Arg Gly Pro Thr Leu Leu Glu Asp Phe Ile Leu Arg Glu
               100                 105                 110
   Lys Ile Thr His Phe Asp His Glu Arg Ile Pro Glu Arg Ile Val His
           115                 120                 125
   Ala Arg Gly Ser Ala Ala His Gly Tyr Phe Gln Pro Tyr Lys Ser Leu
       130                 135                 140
   Ser Asp Ile Thr Lys Ala Asp Phe Leu Ser Asp Pro Asn Lys Ile Thr
   145                 150                 155                 160
   Pro Val Phe Val Arg Phe Ser Thr Val Gln Gly Gly Ala Gly Ser Ala
                   165                 170                 175
   Asp Thr Val Arg Asp Ile Arg Gly Phe Ala Thr Lys Phe Tyr Thr Glu
               180                 185                 190
   Glu Gly Ile Phe Asp Leu Val Gly Asn Asn Thr Pro Ile Phe Phe Ile
           195                 200                 205
   Gln Asp Ala His Lys Phe Pro Asp Phe Val His Ala Val Lys Pro Glu
       210                 215                 220
   Pro His Trp Ala Ile Pro Gln Gly Gln Ser Ala His Asp Thr Phe Trp
   225                 230                 235                 240
   Asp Tyr Val Ser Leu Gln Pro Glu Thr Leu His Asn Val Met Trp Ala
                   245                 250                 255
   Met Ser Asp Arg Gly Ile Pro Arg Ser Tyr Arg Thr Met Glu Gly Phe
               260                 265                 270
   Gly Ile His Thr Phe Arg Leu Ile Asn Ala Glu Gly Lys Ala Thr Phe
           275                 280                 285
   Val Arg Phe His Trp Lys Pro Leu Ala Gly Lys Ala Ser Leu Val Trp
       290                 295                 300
   Asp Glu Ala Gln Lys Leu Thr Gly Arg Asp Pro Asp Phe His Arg Arg
   305                 310                 315                 320
   Glu Leu Trp Glu Ala Ile Glu Ala Gly Asp Phe Pro Glu Tyr Glu Leu
                   325                 330                 335
   Gly Phe Gln Leu Ile Pro Glu Glu Asp Glu Phe Lys Phe Asp Phe Asp
               340                 345                 350
   Leu Leu Asp Pro Thr Lys Leu Ile Pro Glu Glu Leu Val Pro Val Gln
           355                 360                 365
   Arg Val Gly Lys Met Val Leu Asn Arg Asn Pro Asp Asn Phe Phe Ala
       370                 375                 380
   Glu Asn Glu Gln Ala Ala Phe His Pro Gly His Ile Val Pro Gly Leu
   385                 390                 395                 400
   Asp Phe Thr Asn Asp Pro Leu Leu Gln Gly Arg Leu Phe Ser Tyr Thr
                   405                 410                 415
   Asp Thr Gln Ile Ser Arg Leu Gly Gly Pro Asn Phe His Glu Ile Pro
               420                 425                 430
   Ile Asn Arg Pro Thr Cys Pro Tyr His Asn Phe Gln Arg Asp Gly Met
           435                 440                 445
   His Arg Met Gly Ile Asp Thr Asn Pro Ala Asn Tyr Glu Pro Asn Ser
       450                 455                 460
   Ile Asn Asp Asn Trp Pro Arg Glu Thr Pro Pro Gly Pro Lys Arg Gly
   465                 470                 475                 480
   Gly Phe Glu Ser Tyr Gln Glu Arg Val Glu Gly Asn Lys Val Arg Glu
                   485                 490                 495
   Arg Ser Pro Ser Phe Gly Glu Tyr Tyr Ser His Pro Arg Leu Phe Trp
   500 505 510
   Leu Ser Gln Thr Pro Phe Glu Gln Arg His Ile Val Asp Gly Phe Ser
   515 520 525
   Phe Glu Leu Ser Lys Val Val Arg Pro Tyr Ile Arg Glu Arg Val Val
   530 535 540
   Asp Gln Leu Ala His Ile Asp Leu Thr Leu Ala Gln Ala Val Ala Lys
   545 550 555 560
   Asn Leu Gly Ile Glu Leu Thr Asp Asp Gln Leu Asn Ile Thr Pro Pro
   565 570 575
   Pro Asp Val Asn Gly Leu Lys Lys Asp Pro Ser Leu Ser Leu Tyr Ala
   580 585 590
   Ile Pro Asp Gly Asp Val Lys Gly Arg Val Val Ala Ile Leu Leu Asn
   595 600 605
   Asp Glu Val Arg Ser Ala Asp Leu Leu Ala Ile Leu Lys Ala Leu Lys
   610 615 620
   Ala Lys Gly Val His Ala Lys Leu Leu Tyr Ser Arg Met Gly Glu Val
   625 630 635 640
   Thr Ala Asp Asp Gly Thr Val Leu Pro Ile Ala Ala Thr Phe Ala Gly
   645 650 655
   Ala Pro Ser Leu Thr Val Asp Ala Val Ile Val Pro Cys Gly Asn Ile
   660 665 670
   Ala Asp Ile Ala Asp Asn Gly Asp Ala Asn Tyr Tyr Leu Met Glu Ala
   675 680 685
   Tyr Lys His Leu Lys Pro Ile Ala Leu Ala Gly Asp Ala Arg Lys Phe
   690 695 700
   Lys Ala Thr Ile Lys Ile Ala Asp Gln Gly Glu Glu Gly Ile Val Glu
   705 710 715 720
   Ala Asp Ser Ala Asp Gly Ser Phe Met Asp Glu Leu Leu Thr Leu Met
   725 730 735
   Ala Ala His Arg Val Trp Ser Arg Ile Pro Lys Ile Asp Lys Ile Pro
   740 745 750
   Ala
<210> 58
   <211> 25
   <212> DNA
   <213> artificial sequence
<220>
   <223> Primer
<400> 58
   gatctgactg gtggtctata gttag 25
<210> 59
   <211> 25
   <212> DNA
   <213> artificial sequence
<220>
   <223> Primer
<400> 59
   gtagttatca tgatgtgtaa gtaag 25
<210> 60
   <211> 963
   <212> DNA
   <213> artificial sequence
<220>
   <223> Synthetic construct
<400> 60
   atgcagctgt ttgacctgag cctggaagaa ctgaaaaagt ataaaccgaa aaagaccgcc    60
   cgtcctgact tctctgattt ctggaagaaa tctctggaag aactgcgtca ggtagaagct   120
   gaaccgaccc tggaaagcta cgactatcca gtaaagggcg tgaaagtgta ccgtctgact   180
   taccagtctt tcggtcactc taagattgaa ggtttctacg ctgtaccgga ccaaactggt   240
   ccgcatccgg cgctggttcg tttccatggc tacaatgctt cttatgatgg cggtattcac   300
   gacatcgtca attgggctct gcacggctac gcaactttcg gcatgctggt ccgtggccag   360
   ggtggcagcg aagataccag cgtcactcca ggcggccatg cactgggttg gatgaccaaa   420
   ggtattctga gcaaagacac ctactactac cgcggcgtct acctggatgc ggtacgtgct   480
   ctggaagtca ttcagtcttt cccggaagtc gacgaacacc gtatcggtgt aattggtggc   540
   tctcagggtg gcgccctggc catcgcggca gcggcactgt ccgatatccc gaaggtggtg   600
   gtggcggatt acccgtacct gtctaacttc gaacgtgcgg ttgacgtggc tctggaacag   660
   ccgtacctgg agatcaactc ttacttccgc cgtaacagcg atccgaaagt ggaggagaaa   720
   gcgttcgaaa ccctgagcta cttcgatctg atcaacctgg caggctgggt gaaacagccg   780
   actctgatgg ctattggtct gatcgataag atcaccccgc catccactgt cttcgcggct   840
   tacaaccacc tggaaactga taaagatctg aaagtatacc gttacttcgg ccacgagttt   900
   atccctgcat tccagaccga gaaactgtct ttcctgcaaa agcacctgct gctgtccacc   960
   taa                                                                 963
<210> 61
   <211> 182
   <212> PRT
   <213> Bacillus subtilis ATCC 31954
<400> 61
   Arg Gly Gln Gln Ser Ser Glu Asp Thr Ser Ile Ser Leu His Gly His
   1 5 10 15
   Ala Leu Gly Trp Met Thr Lys Gly Ile Leu Asp Lys Asp Thr Tyr Tyr
   20 25 30
   Tyr Arg Gly Val Tyr Leu Asp Ala Val Arg Ala Leu Glu Val Ile Ser
   35 40 45
   Ser Phe Asp Glu Val Asp Glu Thr Arg Ile Gly Val Thr Gly Gly Ser
   50 55 60
   Gln Gly Gly Gly Leu Thr Ile Ala Ala Ala Ala Leu Ser Asp Ile Pro
   65 70 75 80
   Lys Ala Ala Val Ala Asp Tyr Pro Tyr Leu Ser Asn Phe Glu Arg Ala
   85 90 95
   Ile Asp Val Ala Leu Glu Gln Pro Tyr Leu Glu Ile Asn Ser Phe Phe
   100 105 110
   Arg Arg Asn Gly Ser Pro Glu Thr Glu Val Gln Ala Met Lys Thr Leu
   115 120 125
   Ser Tyr Phe Asp Ile Met Asn Leu Ala Asp Arg Val Lys Val Pro Val
   130 135 140
   Leu Met Ser Ile Gly Leu Ile Asp Lys Val Thr Pro Pro Ser Thr Val
   145 150 155 160
   Phe Ala Ala Tyr Asn His Leu Glu Thr Glu Lys Glu Leu Lys Val Tyr
   165 170 175
   Arg Tyr Phe Gly His Glu
   180
<210> 62
   <211> 53
   <212> DNA
   <213> artificial sequence
<220>
   <223> Primer
<400> 62
   taactgcagt aaggaggaat aggacatgcc tctggttgat atgcctctgc gtg 53
<210> 63
   <211> 39
   <212> DNA
   <213> artificial sequence
<220>
   <223> Primer
<400> 63
   tgatctagat taggaggtga agaagcggaa gatctgatc 39
<210> 64
   <211> 988
   <212> DNA
   <213> artificial sequence
<220>
   <223> PCR amplification product
<400> 64
   taactgcagt aaggaggaat aggacatgcc tctggttgat atgcctctgc gtgaactgct     60
   ggcttatgaa ggcatcaacc caaaacctgc tgacttcgat cagtattgga accgcgctaa    120
   aaccgaaatt gaggctatcg atcctgaagt aactctggta gagtcctcct tccagtgctc    180
   cttcgctaac tgctaccatt tctattatcg ttccgcgggc aacgctaaaa tccacgcgaa    240
   gtacgtacag ccaaaagcgg gtgaaaaaac tccggcagtc ttcatgtttc acggctacgg    300
   tggtcgttcc gctgaatggt cctctctgct gaactacgtt gctgctggtt tcagcgtctt    360
   ctacatggat gttcgtggcc agggcggtac ctccgaggac ccgggtggcg tacgtggtaa    420
   cacctatcgt ggtcatatca tccgtggcct ggacgcgggt ccggatgcgc tgttctaccg    480
   ttccgtgttc ctggacacgg tacagctggt gcgcgctgca aaaaccctgc cgcacattga    540
   caagacccgt ctgatggcca ccggctggag ccagggtggc gcactgactc tggcgtgtgc    600
   agcgctggta ccggaaatca aacgtctggc gccggtctac ccgttcctgt ctgactacaa    660
   acgcgtatgg cagatggacc tggctgttcg ttcctacaaa gaactggcgg actatttccg    720
   ctcctatgat ccgcagcata aacgccacgg tgaaattttc gaacgcctgg gttatatcga    780
   cgttcagcac ctggctgatc gtattcaggg cgacgttctg atgggtgtgg gcctgatgga    840
   caccgaatgc ccgccgagca cccaatttgc ggcgtacaac aagattaaag ctaagaaaag    900
   ctacgaactg tacccggact ttggtcatga gcatctgcct ggtatgaacg atcacatctt    960
   ccgcttcttc acctcctaat ctagatca                                       988
<210> 65
   <211> 951
   <212> DNA
   <213> artificial sequence
<220>
   <223> Synthetic gene - codon optimized
<400> 65
   atgcctctgg ttgatatgcc tctgcgtgaa ctgctggctt atgaaggcat caacccaaaa 60
   cctgctgact tcgatcagta ttggaaccgc gctaaaaccg aaattgaggc tatcgatcct 120
   gaagtaactc tggtagagtc ctccttccag tgctccttcg ctaactgcta ccatttctat 180
   tatcgttccg cgggcaacgc taaaatccac gcgaagtacg tacagccaaa agcgggtgaa 240
   aaaactccgg cagtcttcat gtttcacggc tacggtggtc gttccgctga atggtcctct 300
   ctgctgaact acgttgctgc tggtttcagc gtcttctaca tggatgttcg tggccagggc 360
   ggtacctccg aggacccggg tggcgtacgt ggtaacacct atcgtggtca tatcatccgt 420
   ggcctggacg cgggtccgga tgcgctgttc taccgttccg tgttcctgga cacggtacag 480
   ctggtgcgcg ctgcaaaaac cctgccgcac attgacaaga cccgtctgat ggccaccggc 540
   tggagccagg gtggcgcact gactctggcg tgtgcagcgc tggtaccgga aatcaaacgt 600
   ctggcgccgg tctacccgtt cctgtctgac tacaaacgcg tatggcagat ggacctggct 660
   gttcgttcct acaaagaact ggcggactat ttccgctcct atgatccgca gcataaacgc 720
   cacggtgaaa ttttcgaacg cctgggttat atcgacgttc agcacctggc tgatcgtatt 780
   cagggcgacg ttctgatggg tgtgggcctg atggacaccg aatgcccgcc gagcacccaa 840
   tttgcggcgt acaacaagat taaagctaag aaaagctacg aactgtaccc ggactttggt 900
   catgagcatc tgcctggtat gaacgatcac atcttccgct tcttcacctc c 951
<210> 66
   <211> 52
   <212> DNA
   <213> artificial sequence
<220>
   <223> Primer
<400> 66
   taactgcagt aaggaggaat aggacatggg tctgttcgat atgccactgc aa 52
<210> 67
   <211> 36
   <212> DNA
   <213> artificial sequence
<220>
   <223> Primer
<400> 67
   tgatctagat taagaataca gttccagcat gaactg 36
<210> 68
   <211> 997
   <212> DNA
   <213> artificial sequence
<220>
   <223> PCR amplification product
<400> 68
   taactgcagt aaggaggaat aggacatggg tctgttcgat atgccactgc aaaaactgcg      60
   tgaatatacc ggtaccaacc catgtcctga ggatttcgat gaatactggg atcgcgcact     120
   ggacgaaatg cgtagcgttg atcctaaaat caagatgaag aagagctcct ttcaagttcc     180
   gttcgcggaa tgttacgatc tgtattttac cggcgttcgt ggtgcccgca ttcacgcgaa     240
   atacattcgt ccgaaaaccg aaggcaaaca cccggcgctg attcgcttcc atggttactc     300
   cagcaactct ggtgattgga acgacaagct gaactacgtt gcggctggtt ttaccgtagt     360
   agcgatggac gctcgtggcc agggtggcca atctcaggac gtcggcggtg ttaatggcaa     420
   caccctgaac ggtcacatca tccgtggcct ggacgatgat gcagataaca tgctgttccg     480
   tcatattttc ctggacaccg cgcagctggc tggtatcgtt atgaacatgc cggaaatcga     540
   tgaggaccgc gtagctgtta tgggtccgtc ccagggcggc ggtctgtccc tggcgtgtgc     600
   ggctctggaa cctaaaatcc gtaaagtagt gtccgaatat ccgttcctga gcgactacaa     660
   gcgtgtgtgg gatctggatc tggccaaaaa tgcgtaccaa gaaatcactg actatttccg     720
   tctgttcgac ccacgccacg aacgtgagaa cgaggttttt actaaactgg gttacattga     780
   cgtaaagaac ctggcgaaac gtatcaaagg tgatgttctg atgtgcgtgg gcctgatgga     840
   tcaggtctgc ccgccgagca ccgtatttgc agcatacaac aacatccagt ccaagaagga     900
   catcaaagtc tacccggact atggtcacga accgatgcgt ggcttcggtg acctggctat     960
   gcagttcatg ctggaactgt attcttaatc tagatca                              997
<210> 69
   <211> 960
   <212> DNA
   <213> artificial sequence
<220>
   <223> Synthetic gene - codon optimized
<400> 69
   atgggtctgt tcgatatgcc actgcaaaaa ctgcgtgaat ataccggtac caacccatgt 60
   cctgaggatt tcgatgaata ctgggatcgc gcactggacg aaatgcgtag cgttgatcct 120
   aaaatcaaga tgaagaagag ctcctttcaa gttccgttcg cggaatgtta cgatctgtat 180
   tttaccggcg ttcgtggtgc ccgcattcac gcgaaataca ttcgtccgaa aaccgaaggc 240
   aaacacccgg cgctgattcg cttccatggt tactccagca actctggtga ttggaacgac 300
   aagctgaact acgttgcggc tggttttacc gtagtagcga tggacgctcg tggccagggt 360
   ggccaatctc aggacgtcgg cggtgttaat ggcaacaccc tgaacggtca catcatccgt 420
   ggcctggacg atgatgcaga taacatgctg ttccgtcata ttttcctgga caccgcgcag 480
   ctggctggta tcgttatgaa catgccggaa atcgatgagg accgcgtagc tgttatgggt 540
   ccgtcccagg gcggcggtct gtccctggcg tgtgcggctc tggaacctaa aatccgtaaa 600
   gtagtgtccg aatatccgtt cctgagcgac tacaagcgtg tgtgggatct ggatctggcc 660
   aaaaatgcgt accaagaaat cactgactat ttccgtctgt tcgacccacg ccacgaacgt 720
   gagaacgagg tttttactaa actgggttac attgacgtaa agaacctggc gaaacgtatc 780
   aaaggtgatg ttctgatgtg cgtgggcctg atggatcagg tctgcccgcc gagcaccgta 840
   tttgcagcat acaacaacat ccagtccaag aaggacatca aagtctaccc ggactatggt 900
   cacgaaccga tgcgtggctt cggtgacctg gctatgcagt tcatgctgga actgtattct 960
<210> 70
   <211> 320
   <212> PRT
   <213> Thermoanaerobacterium saccharolyticum
<400> 70
   Met Gly Leu Phe Asp Met Pro Leu Gln Lys Leu Arg Glu Tyr Thr Gly
   1               5                   10                  15
   Thr Asn Pro Cys Pro Glu Asp Phe Asp Glu Tyr Trp Asp Arg Ala Leu
               20                  25                  30
   Asp Glu Met Arg Ser Val Asp Pro Lys Ile Lys Met Lys Lys Ser Ser
           35                  40                  45
   Phe Gln Val Pro Phe Ala Glu Cys Tyr Asp Leu Tyr Phe Thr Gly Val
       50                  55                  60
   Arg Gly Ala Arg Ile His Ala Lys Tyr Ile Arg Pro Lys Thr Glu Gly
   65                  70                  75                  80
   Lys His Pro Ala Leu Ile Arg Phe His Gly Tyr Ser Ser Asn Ser Gly
                   85                  90                  95
   Asp Trp Asn Asp Lys Leu Asn Tyr Val Ala Ala Gly Phe Thr Val Val
               100                 105                 110
   Ala Met Asp Ala Arg Gly Gln Gly Gly Gln Ser Gln Asp Val Gly Gly
           115                 120                 125
   Val Asn Gly Asn Thr Leu Asn Gly His Ile Ile Arg Gly Leu Asp Asp
       130                 135                 140
   Asp Ala Asp Asn Met Leu Phe Arg His Ile Phe Leu Asp Thr Ala Gln
   145 150 155 160
   Leu Ala Gly Ile Val Met Asn Met Pro Glu Ile Asp Glu Asp Arg Val
   165 170 175
   Ala Val Met Gly Pro Ser Gln Gly Gly Gly Leu Ser Leu Ala Cys Ala
   180 185 190
   Ala Leu Glu Pro Lys Ile Arg Lys Val Val Ser Glu Tyr Pro Phe Leu
   195 200 205
   Ser Asp Tyr Lys Arg Val Trp Asp Leu Asp Leu Ala Lys Asn Ala Tyr
   210 215 220
   Gln Glu Ile Thr Asp Tyr Phe Arg Leu Phe Asp Pro Arg His Glu Arg
   225 230 235 240
   Glu Asn Glu Val Phe Thr Lys Leu Gly Tyr Ile Asp Val Lys Asn Leu
   245 250 255
   Ala Lys Arg Ile Lys Gly Asp Val Leu Met Cys Val Gly Leu Met Asp
   260 265 270
   Gln Val Cys Pro Pro Ser Thr Val Phe Ala Ala Tyr Asn Asn Ile Gln
   275 280 285
   Ser Lys Lys Asp Ile Lys Val Tyr Pro Asp Tyr Gly His Glu Pro Met
   290 295 300
   Arg Gly Phe Gly Asp Leu Ala Met Gln Phe Met Leu Glu Leu Tyr Ser
   305 310 315 320
<210> 71
   <211> 49
   <212> DNA
   <213> artificial sequence
<220>
   <223> Primer
<400> 71
   taactgcagt aaggaggaat aggacatggg gttcttcgac ctgcctctg 49
<210> 72
   <211> 38
   <212> DNA
   <213> artificial sequence
<220>
   <223> Primer
<400> 72
   tgatctagat tagcccttct caaacagttt ctttcagg 38
<210> 73
   <211> 1012
   <212> DNA
   <213> artificial sequence
<220>
   <223> PCR amplification product
<400> 73
   taactgcagt aaggaggaat aggacatggc gttcttcgac ctgcctctgg aagaactgaa 60
   gaaataccgt ccagagcgtt acgaagagaa ggacttcgac gagttctggg aggaaactct 120
   ggcggagagc gaaaagtttc cgctggaccc agtgttcgag cgtatggaat ctcacctgaa 180
   aaccgtggag gcatatgacg ttactttttc tggttaccgt ggccagcgta tcaaaggctg 240
   gctgctggtt ccgaaactgg aggaagaaaa actgccgtgc gtagttcagt acatcggtta 300
   caacggtggc cgtggctttc cgcacgattg gctgttctgg ccgtctatgg gctacatttg 360
   cttcgtcatg gatactcgtg gtcagggttc cggctggctg aaaggcgata ctccggatta 420
   tccggagggc ccggtagacc cgcagtaccc tggcttcatg acgcgtggta ttctggatcc 480
   gcgtacctat tactatcgcc gcgtttttac cgatgcagtt cgtgccgtag aggccgcggc 540
   ttctttccct caggttgacc aggagcgtat tgttatcgct ggtggctccc agggtggcgg 600
   catcgccctg gcggtatctg cgctgagcaa gaaagctaag gcactgctgt gtgacgtccc 660
   gttcctgtgt cacttccgtc gcgctgttca gctggtagat acccatccgt acgcggagat 720
   tactaacttc ctgaaaactc accgcgacaa agaagaaatc gttttccgca ccctgtccta 780
   tttcgacggc gttaacttcg cggctcgtgc aaaaattccg gcactgttct ctgttggtct 840
   gatggacaac atctgccctc cttctaccgt tttcgcggca tataactatt atgcgggtcc 900
   gaaagaaatc cgtatctatc cgtacaacaa ccacgaaggc ggtggtagct ttcaggctgt 960
   tgaacaagtg aaattcctga agaaactgtt tgagaagggc taatctagat ca 1012
<210> 74
   <211> 975
   <212> DNA
   <213> artificial sequence
<220>
   <223> Synthetic gene - codon optimized
<400> 74
   atggcgttct tcgacctgcc tctggaagaa ctgaagaaat accgtccaga gcgttacgaa    60
   gagaaggact tcgacgagtt ctgggaggaa actctggcgg agagcgaaaa gtttccgctg   120
   gacccagtgt tcgagcgtat ggaatctcac ctgaaaaccg tggaggcata tgacgttact   180
   ttttctggtt accgtggcca gcgtatcaaa ggctggctgc tggttccgaa actggaggaa   240
   gaaaaactgc cgtgcgtagt tcagtacatc ggttacaacg gtggccgtgg ctttccgcac   300
   gattggctgt tctggccgtc tatgggctac atttgcttcg tcatggatac tcgtggtcag   360
   ggttccggct ggctgaaagg cgatactccg gattatccgg agggcccggt agacccgcag   420
   taccctggct tcatgacgcg tggtattctg gatccgcgta cctattacta tcgccgcgtt   480
   tttaccgatg cagttcgtgc cgtagaggcc gcggcttctt tccctcaggt tgaccaggag   540
   cgtattgtta tcgctggtgg ctcccagggt ggcggcatcg ccctggcggt atctgcgctg   600
   agcaagaaag ctaaggcact gctgtgtgac gtcccgttcc tgtgtcactt ccgtcgcgct   660
   gttcagctgg tagataccca tccgtacgcg gagattacta acttcctgaa aactcaccgc   720
   gacaaagaag aaatcgtttt ccgcaccctg tcctatttcg acggcgttaa cttcgcggct   780
   cgtgcaaaaa ttccggcact gttctctgtt ggtctgatgg acaacatctg ccctccttct   840
   accgttttcg cggcatataa ctattatgcg ggtccgaaag aaatccgtat ctatccgtac   900
   aacaaccacg aaggcggtgg tagctttcag gctgttgaac aagtgaaatt cctgaagaaa   960
   ctgtttgaga agggc                                                    975

## Description

### FIELD OF THE INVENTION

This invention relates to the field of peracid biosynthesis and *in situ* enzyme catalysis. Specifically, a process is provided to produce peracids using the perhydrolysis activity of enzymes identified structurally as belonging to the CE-7 family of carbohydrate esterases, including cephalosporin acetyl hydrolases (CAHs; E.C. 3.1.1.41) and acetyl xylan esterases (AXEs; E.C.3.1.1.72). The enzymatic process produces percarboxylic acids from carboxylic acid ester substrates.

### BACKGROUND OF THE INVENTION

Peracid compositions have been reported to be effective antimicrobial agents. Methods to clean, disinfect, and/or sanitize hard surfaces, meat products, living plant tissues, and medical devices against undesirable microbial growth have been described (U.S. patent 6,545,047; U.S. patent 6,183,807; U.S. patent 6,518,307; U.S. patent application publication 20030026846; and U.S. patent 5,683,724). Peracids have also been reported to be useful in preparing bleaching compositions for laundry detergent applications (U.S. patent 3,974,082; U.S. patent 5,296,161; and U.S. patent 5,364,554).

Peracids can be prepared by the chemical reaction of a carboxylic acid and hydrogen peroxide (see Organic Peroxides, Daniel Swem, ed., Vol. 1, pp 313-516; Wiley Interscience, New York, 1971). The reaction is usually catalyzed by a strong inorganic acid, such as concentrated sulfuric acid. The reaction of hydrogen peroxide with a carboxylic acid is an equilibrium reaction, and the production of peracid is favored by the use of an excess concentration of peroxide and/or carboxylic acid, or by the removal of water. There are several disadvantages to the chemical reaction for,peracid production: a) the high concentration of carboxylic acid used to favor production of peracid can result in an undesirable odor when using the peracid-containing solution, 2) the peracid is oftentimes unstable in solution over time, and the concentration of peracid in the solution decreases during storage prior to use, and 3) the formulation is often strongly acidic due to the use of a concentrated sulfuric acid as catalyst.

One way to overcome the disadvantages of the chemical production of peracids is to employ an enzyme catalyst in place of a strong acid catalyst. The use of an enzyme catalyst allows for the rapid production of peracid at the time of use and/or application, avoiding problems associated with storage of peracid solutions and variations in peracid concentrations over time. The high concentrations of carboxylic acids typically used to produce peracid via the direct chemical reaction with hydrogen peroxide are not required for enzymatic production of peracid, where the enzyme-catalyzed reaction can use a carboxylic acid ester as substrate at a much lower concentration than is typically used in the chemical reaction. The enzyme reaction can be performed across a broad range of pH, dependent on enzyme activity and stability at a given pH, and on the substrate specificity for perhydrolysis at a given pH.

Esterases, lipases, and some proteases have the ability catalyze the hydrolysis of alkyl esters to produce the corresponding carboxylic acids (Formula 1).

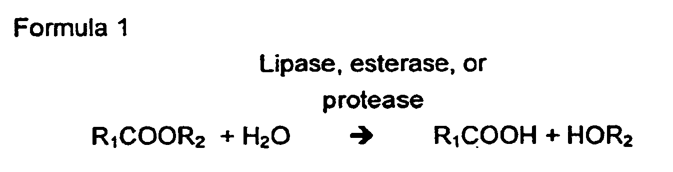

Some esterases, lipases, and proteases also exhibit perhydrolysis activity, catalyzing the synthesis of peracids from alkyl esters (Formula 2).

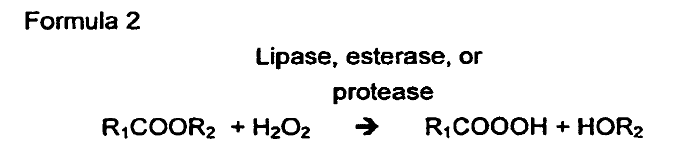

O. Kirk et al. (Biocatalysis, 11:65-77 (1994)) investigated the ability of hydrolases (lipases, esterases, and proteases) to catalyze perhydrolysis of acyl substrates with hydrogen peroxide to form peroxycarboxylic acids, and reported that perhydrolysis proceeds with a very low efficiency in aqueous systems. Furthermore, they found that lipases and esterases degraded percarboxylic acid to the corresponding carboxylic acid and hydrogen peroxide. They also found that proteases neither degraded nor catalyzed perhydrolysis of carboxylic acid esters in water. The authors concluded that esterases, lipases and proteases are, in general, not suitable for catalyzing perhydrolysis of simple esters, such as methyl octanoate and trioctanoin, in an aqueous environment.

U.S. patent 3,974,082 describes the production of bleaching compositions for laundry detergent applications by contacting the material to be bleached with an aqueous solution containing an oxygen-releasing inorganic peroxygen compound, an acyl alkyl ester, and an esterase or lipase capable of hydrolyzing the ester.

U.S. patent 5,364,554 describes an activated oxidant system for *in situ* generation of peracid in aqueous solution using a protease enzyme, a source of hydrogen peroxide, and an ester substrate that is preferably chemically non-perhydrolyzable. A method of bleaching and a method of forming peracid are also disclosed.

U.S. patent 5,296,161 describes production of peracid in an aqueous solution comprising one or more specific esterases and lipases, a source of hydrogen peroxide, and a functionalized ester substrate suitable for use in a bleaching composition. However, the concentration of peracid produced was generally insufficient for use in many commercial disinfectant applications.

WO2006119060 describes a process to produce a concentrated aqueous peracid solution in situ using at least one enzyme having perhydrolase activity in the presence of hydrogen peroxide (at a concentration of at least 500 mM) under neutral to acidic reaction conditions from suitable carboxylic acid esters (including glycerides) and/or amides substrates. The concentrated peracid solution produced is said to be sufficient for use in a variety of disinfection and/or bleaching applications.

WO2007/070609 describes method for producing peroxycarboxylic acids from carboxylic acid esters. More specifically, carboxylic acid esters are reacted with an inorganic peroxide, such as hydrogen peroxide, in the presence of an enzyme catalyst having perhydrolysis activity derived from *Bacillus sp.* to produce peroxycarboxylic acids.

Most known methods for preparing peracids from the corresponding carboxylic acid esters using enzyme catalysts do not produce and accumulate a peracid at a sufficiently-high concentration to be efficacious for disinfection in a variety of applications. Several protease and lipase combinations have recently been reported to generate peracids (*e.g*., peracetic acid) *in situ* at concentrations suitable for use as a disinfectant and/or commercial bleaching agent (see co-owned U.S. Patent Applications Nos. 11/413,246 and 11/588,523). However, there remains a need to identify additional perhydrolase catalysts capable of producing peracids *in situ.*

U.S. patent 4,444,886 describes a strain of *Bacillus subtilis* (ATCC 31954™) having ester hydrolase activity (described as a "diacetinase") that has high specificity for hydrolyzing glycerol esters having acyl groups having 2 to 8 carbon atoms. U.S. patent 4,444,886 does not describe, discuss or predict that the ester hydrolase activity of this strain has perhydrolase activity towards carboxylic acid esters, including glycerol esters.

The problem to be solved is to provide a process to enzymatically produce peracids *in situ* at concentrations suitable for use in a variety of disinfectant applications and/or bleaching applications. Preferably, the substrates used to produce the peracid compositions should be relatively non-toxic and inexpensive, such as carboxylic acid esters, especially mono-, di-, and triacylglycerols, where the acyl group has 1- 8 carbon atoms, as well as acetylated sugars.

### SUMMARY OF THE INVENTION

The stated problems have been solved by the discovery that enzymes belonging to the structural family of CE-7 esterases (e.g., cephalosporin C deacetylases [CAHs] and acetyl xylan esterases [AXEs]) exhibit significant perhydrolysis activity for converting the present carboxylic acid esters (in the presence of an inorganic source of peroxygen such as hydrogen peroxide) into peracids at concentrations sufficient for use as a disinfectant and/or bleaching agent. The system achieves efficiency by producing the peracid in high concentrations without requiring a high concentration of peroxygen.

Specific examples of perhydrolases are exemplified from *Bacillus subtilis* (ATCC 31954™), *B. subtilis* BE1010 (Payne and Jackson, J. Bacteriol. 173:2278-2282 (1991)), *B. subtilis* ATCC 6633™ (U.S. 6,465,233), *B. subtilis* ATCC 29233™; *B. licheniformis* ATCC 14580™ (Rey *et al., Genome Biol.,* 5(10):article 77 (2004)), *Clostridium thermocellum* ATCC 27405™ (Copeland *et al.,* GENBANK® ZP_00504991, *B. pumilus* PS213 (Degrassi et al., Microbiology, 146:1585-1591 (2000)), and *Thermotoga neapolitana* (GENBANK® AAB70869.1).

Each of the present perhydrolases described herein share conserved structural features (i.e. a conserved signature motif) as well as superior perhydrolysis activity when compared to other α/β-hydrolases, (such as commercially available lipases; see comparative Examples 26 and 28), making this unique family of enzymes particularly suitable for generating peracids *in situ* at concentrations sufficient for use as a disinfectant and/or bleaching agent. Suitable perhydrolases useful in the present process can be identified by a conserved signature motif found within the CE-7 family of carbohydrate esterases.

In one aspect of the invention, a process is provided, including a process for producing a peroxycarboxylic acid from a carboxylic acid ester comprising
a) providing a set of reaction components, said components comprising:
   1) a carboxylic acid ester selected from the group consisting of:
      i) esters having the structure 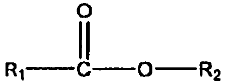 wherein R₁= C1 to C7 straight chain or branched chain alkyl optionally substituted with an hydroxyl or a C1 to C4 alkoxy group and R₂= C1 to C10 straight chain or branched chain alkyl, alkenyl, alkynyl, aryl, alkylaryl, alkylheteroaryl, heteroaryl, (CH₂CH₂-O)ₙH or (CH₂CH(CH₃)-O)ₙH and n=1 to 10; and
      ii) glycerides having the structure 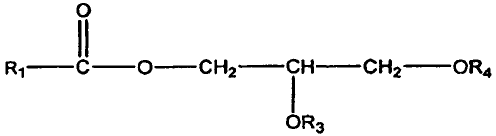 wherein R₁= C1 to C7 straight chain or branched chain alkyl optionally substituted with an hydroxyl or a C1 to C4 alkoxy group and R₃ and R₄ are individually H or R₁C(O); and
      iii) acetylated carbohydrates
   2) a source of peroxygen; and
   3) an enzyme catalyst having perhydrolysis activity, wherein said enzyme catalyst comprises an enzyme having at least 90% amino acid identity to an amino acid sequence of SEQ ID No: 16, 18, 8, 10, 12, 14, 20, 22, 24 or 26; and
b) combining said reaction components under suitable aqueous reaction conditions wherein said conditions comprise a pH range of about 2 to about 9 whereby a peroxycarboxylic acid is produced.

In another aspect of the invention, a process is provided, including a process to reduce a concentration of a microbial population on a hard surface or inanimate object using an enzymatically-produced peroxycarboxylic acid composition, said process comprising:
a) providing a set of reaction components, said components comprising:
   1. a substrate selected from the group consisting of:
      i) esters having the structure 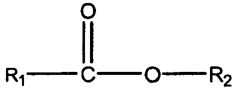 wherein R₁= C1 to C7 straight chain or branched chain alkyl optionally substituted with an hydroxyl or a C1 to C4 alkoxy group and R₂= C1 to C10 straight chain or branched chain alkyl, alkenyl, alkynyl, aryl, alkylaryl, alkylheteroaryl, heteroaryl, (CH₂CH₂-O)ₙH or (CH₂CH(CH₃)-O)ₙH and n=1 to 10; and
      ii) glycerides having the structure 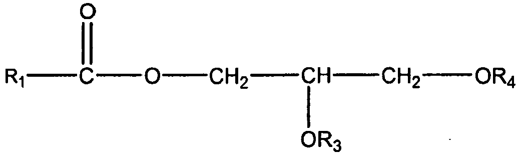 wherein R₁= C1 to C7 straight chain or branched chain alkyl optionally substituted with an hydroxyl or a C1 to C4 alkoxy group and R₃ and R₄ are individually H or R₁C(O);
      iii) acetylated carbohydrates;
   2) a source of peroxygen; and
   3) an enzyme catalyst having perhydrolysis activity, wherein said enzyme catalyst comprises: an enzyme having at least 90% amino acid identity to an amino acid sequence of SEQ ID No: 16, 18, 8, 10, 12, 14, 20, 22, 24, or 26;
b) combining said reaction components under suitable aqueous reaction conditions wherein said conditions, comprise a pH range of about 2 to about 9, whereby a peroxycarboxylic acid product is formed;
c) optionally diluting said peroxycarboxylic acid product; and
d) contacting said hard surface or inanimate object with the peroxycarboxylic acid produced in step b) or step c) whereby viable microorganisms on said surface or said inanimate object are reduced at least 3 log;
wherein said hard surface or inanimate object is not a living animal.

In another aspect of the invention, a system is provided, including a peroxycarboxylic acid generating system comprising:
a) a substrate selected from the group consisting of:
   1) esters having the structure 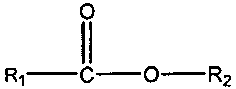 wherein R₁= C1 to C7 straight chain or branched chain alkyl optionally substituted with an hydroxyl or a C1 to C4 alkoxy group and R₂= C1 to C10 straight chain or branched chain alkyl, alkenyl, alkynyl, aryl, alkylaryl, alkylheteroaryl, heteroaryl, (CH₂CH₂-O)ₙH or (CH₂CH(CH₃)-O)ₙH and n=1 to 10; and
   2) glycerides having the structure 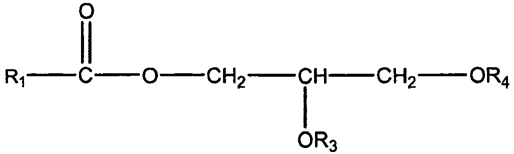 wherein R₁= C1 to C7 straight chain or branched chain alkyl optionally substituted with an hydroxyl or a C1 to C4 alkoxy group and R₃ and R₄ are individually H or R₁C(O); and
   3) acetylated carbohydrates; and
b) a source of peroxygen; and
c) an enzyme catalyst having perhydrolysis activity, wherein said enzyme catalyst comprises: an enzyme having at least 90% amino acid identity in an amino acid sequence of SED ID No: 16, 18, 8, 10, 12, 14, 20, 22, 24, or 26.

Also disclosed is a formulation, said formulation comprising:
a) a first mixture comprising an enzyme catalyst comprising a perhydrolase enzyme having a CE-7 signature motif and an carboxylic acid ester selected from the group consisting of monoacetin, diacetin, triacetin and mixtures thereof; said first mixture optionally comprising a further component selected from the group consisting of an inorganic or organic buffer, a corrosion inhibitor, a wetting agent, and combinations thereof; and
b) a second mixture comprising a source of peroxygen and water, said second mixture optionally further comprising a chelating agent.

Also disclosed is a formulation, said formulation comprising:
a) a first mixture comprising a enzyme catalyst comprising a perhydrolase enzyme having a CE-7 signature motif and an acetylated saccharide selected from the group consisting of acetylated monosaccharides, acetylated disaccharides, acetylated polysaccharides, and combinations thereof, said first mixture optionally further comprising an inorganic or organic buffer, a corrosion inhibitor, and a wetting agent; and
b) a second mixture comprising a source of peroxygen and water, said second mixture optionally comprising a chelating agent.

In other aspects the first and second mixtures above may be combined to provide for production of peroxycarboxylic acid. In a further aspect, the first and second mixtures above may be combined to form disinfectant formulations.

Also disclosed is a recombinant *Escherichia coli* cell comprising a disruption in *kat*E and a disruption in *kat*G with the proviso that the host cell is not *Escherichia coli* UM2. In another aspect, the recombinant *Escherichia coli* host cell comprising a disruption in *kat*E and a disruption in *kat*G, is derived from *Escherichia coli* strain MG1655. In another aspect, the *Escherichia coli* host cell or strain produces or comprises at least one enzyme having perhydrolase activity. In another aspect, said enzyme is capable of using or generating hydrogen peroxide.

The enzyme catalyst having perhydrolysis activity used in the present process may be in the form of non-viable whole cells, permeabilized whole cells, one or more cell components of a microbial cell extract, partially-purified enzyme, and purified enzyme. The enzyme catalyst may be unimmobilized or immobilized, including but not limited to: immobilization in or on an insoluble solid support, covalently attached to a soluble polymer (e.g., low-molecular weight polyethylene glycol (PEG)), and immobilized as soluble enzyme in a hollow-fiber cartridge.

In another aspect of the invention, a method is provided to reduce a concentration of a microbial population on a hard surface or inanimate object by contacting the peracid composition produced by the above processes with said hard surface or inanimate object, whereby the concentration of the viable microbial population is reduced at least 3-log, preferably at least 4-log, more preferably at least 5-log, and most preferably at least 6-log. In a further aspect, the peracid composition produced by the above methods may be optionally diluted to a desired efficacious concentration prior to contacting the surface or inanimate object to be treated.

### BRIEF DESCRIPTION OF THE FIGURE

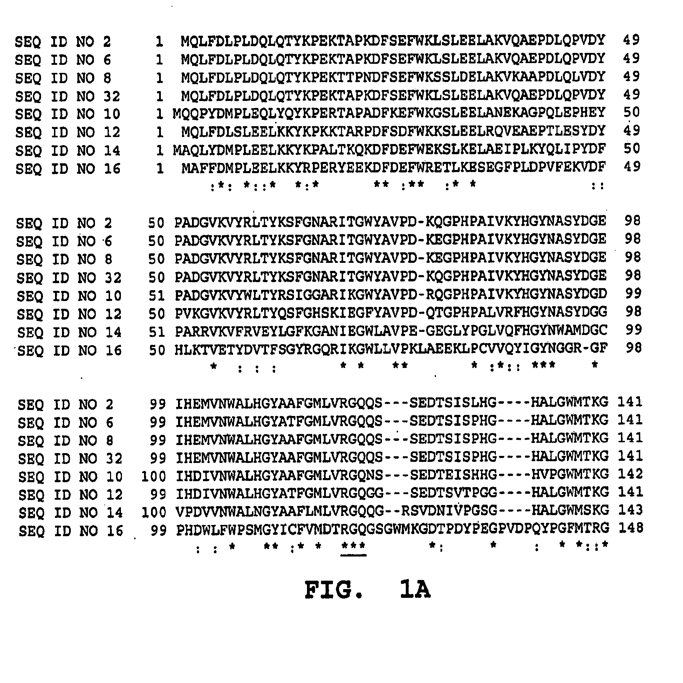

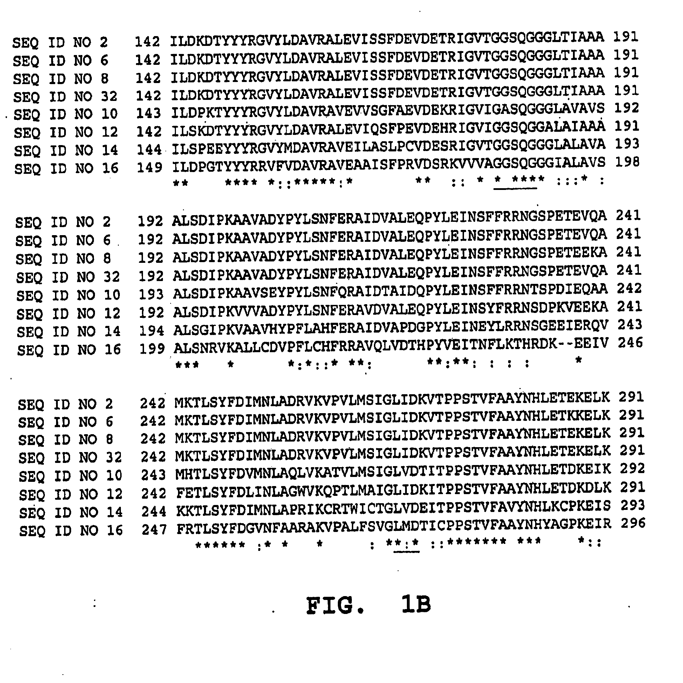

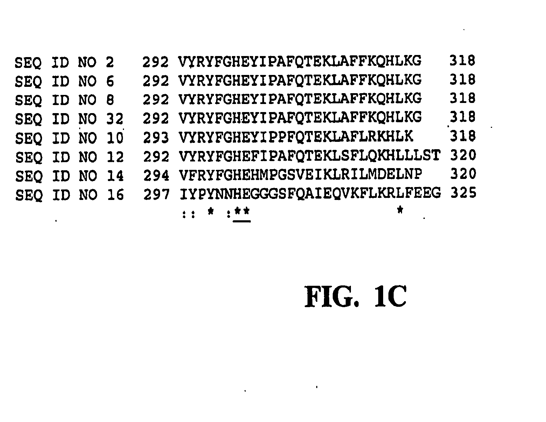

Figure 1 (panels a-c) is a CLUSTALW alignment of perhydrolases of the present invention. Each of the perhydrolases are structurally classified members of the carbohydrate esterase family 7 (CE-7) and share certain conserved domains. Several conserved motifs (underlined) have been identified that together form the signature motif for CE-7 carbohydrate esterases. An additional motif (LXD; amino acid residues 267-269 of SEQ ID NO: 2) is also underlined and may be used to further characterize the signature motif.

### BRIEF DESCRIPTION OF THE BIOLOGICAL SEQUENCES

The following sequences comply with 37 C.F.R. 1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (1998) and the sequence listing requirements of the European Patent Convention (EPC) and the Patent Cooperation Treaty (PCT) Rules 5.2 and 49.5(a-bis), and Section 208 and Annex C of the Administrative Instructions. The symbols and format used for nucleotide and amino acid sequence data comply with the rules set forth in 37 C. F. R. §1.822.

SEQ ID NO: 1 is the nucleic acid sequence of the cephalosporin C deacetylase (*cah*) coding region from *Bacillus subtilis* ATCC 31954™.

SEQ ID NO: 2 is the deduced amino acid sequence of the cephalosporin C deacetylase from *Bacillus subtilis* ATCC 31954™.

SEQ ID NOs: 3 and 4 are primers used to PCR amplify perhydrolase genes from *Bacillus subtilis* species for construction of pSW186, pSW187, pSW188, and pSW190.

SEQ ID NO: 5 is the nucleic acid sequence of the cephalosporin C deacetylase coding region from *B. subtilis subsp. subtilis str.* 168.

SEQ ID NO: 6 is the deduced amino acid sequence of the cephalosporin C deacetylase from *B. subtilis subsp. subtilis str.* 168, and is identical to the deduced amino acid sequence of the cephalosporin C deacetylase from *B. subtilis* BE1010.

SEQ ID NO: 7 is the nucleic acid sequence of the cephalosporin acetylesterase coding region from *B. subtilis* ATCC 6633™.

SEQ ID NO: 8 is the deduced amino acid sequence of the cephalosporin acetylesterase from *B*. *subtilis* ATCC 6633™.

SEQ ID NO: 9 is the nucleic acid sequence of the cephalosporin C deacetylase coding region from *B*. *licheniformis* ATCC 14580™.

SEQ ID NO: 10 is the deduced amino acid sequence of the cephalosporin C deacetylase from *B. licheniformis* ATCC 14580™.

SEQ ID NO: 11 is the nucleic acid sequence of the acetyl xylan esterase coding region from *B. pumilus* PS213.

SEQ ID NO: 12 is the deduced amino acid sequence of the acetyl xylan esterase from *B. pumilus* PS213.

SEQ ID NO: 13 is the nucleic acid sequence of the acetyl xylan esterase coding region from *Clostridium thermocellum* ATCC 27405™.

SEQ ID NO: 14 is the deduced amino acid sequence of the acetyl xylan esterase from *Clostridium thermocellum* ATCC 27405™.

SEQ ID NO: 15 is the nucleic acid sequence of the acetyl xylan esterase coding region from *Thermotoga neapolitana.*

SEQ ID NO: 16 is the deduced amino acid sequence of the acetyl xylan esterase from *Thermotoga neapolitana.*

SEQ ID NO: 17 is the nucleic acid sequence of the acetyl xylan esterase coding region from *Thermotoga maritima* MSB8.

SEQ ID NO: 18 is the deduced amino acid sequence of the acetyl xylan esterase from *Thermotoga maritima* MSB8.

SEQ ID NO: 19 is the nucleic acid sequence of the acetyl xylan esterase coding region from *Thermoanaerobacterium sp.* JW/SL YS485.

SEQ ID NO: 20 is the deduced amino acid sequence of the acetyl xylan esterase from *Thermoanaerobacterium sp.* JW/SL YS485.

SEQ ID NO: 21 is the nucleic acid sequence of the cephalosporin C deacetylase coding region from *Bacillus sp.* NRRL B-14911.

SEQ ID NO: 22 is the deduced amino acid sequence of the cephalosporin C deacetylase from *Bacillus sp.* NRRL B-14911.

SEQ ID NO: 23 is the nucleic acid sequence of the cephalosporin C deacetylase coding region from *Bacillus halodurans* C-125.

SEQ ID NO: 24 is the deduced amino acid sequence of the cephalosporin C deacetylase from *Bacillus halodurans* C-125.

SEQ ID NO: 25 is the nucleic acid sequence of the cephalosporin C deacetylase coding region from *Bacillus clausii* KSM-K16.

SEQ ID NO: 26 is the deduced amino acid sequence of the cephalosporin C deacetylase from *Bacillus clausii* KSM-K16.

SEQ ID NOs: 27 and 28 are primers used to PCR amplify perhydrolase genes from *Bacillus subtilis* species for construction of pSW194 and pSW189.

SEQ ID NO: 29 is the nucleic acid sequence of the PCR product cloned into pSW194.

SEQ ID NO: 30 is the nucleic acid sequence of the PCR product cloned into pSW189.

SEQ ID NO: 31 is the nucleic acid sequence of the *Bacillus subtilis* ATCC 29233™ cephalosporin C deacetylase (*cah*) gene cloned into pSW190.

SEQ-ID NO: 32 is the deduced amino acid sequence of the *Bacillus subtilis* ATCC 29233™ cephalosporin C deacetylase (CAH).

SEQ ID NOs: 33 and 34 are primers used to PCR amplify the *Bacillus licheniformis* ATCC 14580™ cephalosporin C deacetylase gene for construction of pSW191.

SEQ ID NOs: 35 and 36 are primers used to PCR amplify the *Clostridium thermocellum* ATCC 27405™ acetyl xylan esterase gene for construction of pSW193.

SEQ ID NOs: 37 and 38 are primers used to PCR amplify the *Bacillus pumilus* PS213 acetyl xylan esterase coding sequence (GENBANK® AJ249957) for construction of pSW195.

SEQ ID NOs: 39 and 40 are primers used to PCR amplify the *Thermotoga neapolitana* acetyl xylan esterase gene (GENBANK® 58632) for construction of pSW196.

SEQ ID NO: 41 is the nucleic acid sequence of the codon-optimized version of the *Thermotoga neapolitana* acetyl xylan esterase gene in plasmid pSW196.

SEQ ID NO: 42 is the nucleic acid sequence of the kanamycin resistance gene.

SEQ ID NO: 43 is the nucleic acid sequence of plasmid pKD13.

SEQ ID NOs: 44 and 45 are primers used to generate a PCR product encoding the kanamycin gene flanked by regions having homology to the *katG* catalase gene in *E. coli* MG1655. The product was used to disrupt the endogenous *katG* gene.

SEQ ID NO: 46 is the nucleic acid sequence of the PCR product encoding the kanamycin resistance gene flanked by regions having homology to the *katG* catalase gene in *E. coli* MG1655. The product was used to disrupt the endogenous *kat*G gene.

SEQ ID NO: 47 is the nucleic acid sequence of the *katG* catalase gene in *E*. *coli* MG1655.

SEQ ID NO: 48 is the deduced amino acid sequence of the KatG catalase in *E. coli* MG1655.

SEQ ID NO: 49 is the nucleic acid sequence of plasmid pKD46.

SEQ ID NOs: 50 and 51 are primers used to confirm the disruption of the *katG* gene.

SEQ ID NO: 52 is the nucleic acid sequence of plasmid pCP20.

SEQ ID NO: 53 and 54 are primers used to generate a PCR product encoding the kanamycin gene flanked by regions having homology to the *kat*E catalase gene in E. coli MG1655. The product was used to disrupt the endogenous *kat*E gene.

SEQ ID NO: 55 is the nucleic acid sequence of the PCR product encoding the kanamycin resistance gene flanked by regions having homology to the *katE* catalase gene in *E*. *coli* MG1655. The product was used to disrupt the endogenous *kat*E gene.

SEQ ID NO: 56 is the nucleic acid sequence of the *katE* catalase gene in *E. coli* MG1655.

SEQ ID NO: 57 is the deduced amino acid sequence of the KatE catalase in *E. coli* MG1655.

SEQ ID NOs: 58 and 59 are primers used to confirm disruption of the *katE* gene in the single knockout strain *E. coli* MG1655 Δ*kat*E, and in the double-knockout strain *E. coli* MG1655 *Δkat*G *Δkat*E, herein referred to as *E. coli* KLP18.

SEQ ID NO: 60 is the nucleic acid sequence of the codon optimized version of the *Bacillus pumilus* PS213 encoding the amino acid sequence SEQ ID NO: 12.

SEQ ID NO: 61 is the amino acid sequence of the region encompassing amino acids residues 118 through 299 of SEQ ID NO: 2.

### DETAILED DESCRIPTION OF THE INVENTION

The stated problems have been solved by the discovery that enzymes belonging to the CE-7 carbohydrate esterase family exhibit significant perhydrolysis activity for converting carboxylic acid ester substrates to peracids. This family of structurally related enzymes can be used to generate concentrations of peracids with high efficiency for disinfection and/or bleaching applications.

In this disclosure, a number of terms and abbreviations are used. The following definitions apply unless specifically stated otherwise.

As used herein, the term "comprising" means the presence of the stated features, integers, steps, or components as referred to in the claims, but that it does not preclude the presence or addition of one or more other features, integers, steps, components or groups thereof.

As used herein, the term "about" modifying the quantity of an ingredient or reactant of the invention or employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities.

As used herein, the term "peracid" is synonymous with peroxyacid, peroxycarboxylic acid, peroxy acid, percarboxylic acid and peroxoic acid.

As used herein, the term "peracetic acid" is abbreviated as "PAA" and is synonymous with peroxyacetic acid, ethaneperoxoic acid and all other synonyms of CAS Registry Number 79-21-0.

As used herein, the term "monoacetin" is synonymous with glycerol monoacetate, glycerin monoacetate, and glyceryl monoacetate.

As used herein, the term "diacetin" is synonymous with glycerol diacetate; glycerin diacetate, glyceryl diacetate, and all other synonyms of CAS Registry Number 25395-31-7.

As used herein, the term "triacetin" is synonymous with glycerin triacetate; glycerol triacetate; glyceryl triacetate, 1,2,3-triacetoxypropane, 1,2,3-propanetriol triacetate and all other synonyms of CAS Registry Number 102-76-1.

As used herein, the term "monobutyrin" is synonymous with glycerol monobutyrate, glycerin monobutyrate, and glyceryl monobutyrate.

As used herein, the term "dibutyrin" is synonymous with glycerol dibutyrate and glyceryl dibutyrate.

As used herein, the term "tributyrin" is synonymous with glycerol tributyrate, 1,2,3-tributyrylglycerol, and all other synonyms of CAS Registry Number 60-01-5.

As used herein, the term "monopropionin" is synonymous with glycerol monopropionate, glycerin monopropionate, and glyceryl monopropionate.

As used herein, the term "dipropionin" is synonymous with glycerol dipropionate and glyceryl dipropionate.

As used herein, the term "tripropionin" is synonymous with glyceryl tripropionate, glycerol tripropionate, 1,2,3-tripropionylglycerol, and all other synonyms of CAS Registry Number 139-45-7.

As used herein, the term "ethyl acetate" is synonymous with acetic ether, acetoxyethane, ethyl ethanoate, acetic acid ethyl ester, ethanoic acid ethyl ester, ethyl acetic ester and all other synonyms of CAS Registry Number 141-78-6.

As used herein, the term "ethyl lactate" is synonymous with lactic acid ethyl ester and all other synonyms of CAS Registry Number 97-64-3.

As used herein, the terms "acetylated sugar" and "acetylated saccharide" refer to mono-, di- and polysaccharides comprising at least one acetyl group. Examples include, but are not limited to glucose pentaacetate, xylose tetraacetate, acetylated xylan, acetylated xylan fragments, β-D-ribofuranose-1,2,3,5-tetraacetate, tri-O-acetyl-D-galactal, and tri-O-acetyl-glucal.

As used herein, the terms "suitable enzymatic reaction mixture", "components suitable for *in situ* generation of a peracid", "suitable reaction components", and "suitable aqueous reaction mixture" refer to the materials and water in which the reactants and enzyme catalyst come into contact. The components of the suitable aqueous reaction mixture are provided herein and those skilled in the art appreciate the range of component variations suitable for this process. In one embodiment, the suitable enzymatic reaction mixture produces peracid *in situ* upon combining the reaction components. As such, the reaction components may be provided as a multicomponent system wherein one or more of the reaction components remains separated until use. The design of systems and means for separating and combining multiple active components are known in the art and generally will depend upon the physical form of the individual reaction components. For example, multiple active fluids (liquid-liquid) systems typically use multichamber dispenser bottles or two-phase systems (U.S. Patent Application Pub. No. 2005/0139608; U.S. Patent 5,398,846; U.S. Patent 5,624,634; U.S. Patent 6,391,840; E.P. Patent 0807156B1; U.S. Patent Appln. Pub. No. 2005/0008526; and PCT Publication No. WO 00/11713A1) such as found in some bleaching applications wherein the desired bleaching agent is produced upon mixing the reactive fluids. Other forms of multicomponent systems used to generate peracid may include, but are not limited to those designed for one or more solid components or combinations of solid-liquid components, such as powders (e.g., many commercially available bleaching composition, U.S. Patent 5,116,575), multi-layered tablets (U.S. Patent 6,210,639), water dissolvable packets having multiple compartments (U.S. Patent 6,995,125) and solid agglomerates that react upon the addition of water (U.S. Patent 6,319,888). In one embodiment, a formulation is provided as two individual mixtures whereby a peroxycarboxylic acid disinfectant is generated upon combining the two mixtures. In another embodiment, a formulation is provided comprising:
a) a first mixture comprising:
   i) an enzyme catalyst having perhydrolase activity, said enzyme catalyst comprising an enzyme having a CE-7 signature motif; and
   ii) a carboxylic acid ester substrate, said first mixture optionally comprising a component selected from the group consisting of an inorganic or organic buffer, a corrosion inhibitor, a wetting agent, and combinations thereof; and
b) a second mixture comprising a source of peroxygen and water, said second mixture optionally comprising a chelating agent.

In another embodiment, the carboxylic acid ester in the first mixture is selected from the group consisting of monoacetin, diacetin, triacetin, and combinations thereof. In another embodiment, the carboxylic acid ester in the first mixture is an acetylated saccharide. In another embodiment, the enzyme catalyst in the first mixture is a particulate solid. In another embodiment, the first reaction mixture is a solid tablet or powder.

As used herein, the term "perhydrolysis" is defined as the reaction of a selected substrate with peroxide to form a peracid. Typically, inorganic peroxide is reacted with the selected substrate in the presence of a catalyst to produce the peracid. As used herein, the term "chemical perhydrolysis" includes perhydrolysis reactions in which a substrate (a peracid precursor) is combined with a source of hydrogen peroxide wherein peracid is formed in the absence of an enzyme catalyst.

As used herein, the term "perhydrolase activity" refers to the catalyst activity per unit mass (for example, milligram) of protein, dry cell weight, or immobilized catalyst weight.

As used herein, "one unit of enzyme activity" or "one unit of activity" or "U" is defined as the amount of perhydrolase activity required for the production of 1 µmol of peracid product per minute at a specified temperature.

As used herein, the terms "enzyme catalyst" and "perhydrolase catalyst" refer to a catalyst comprising an enzyme having perhydrolysis activity and may be in the form of a whole microbial cell, permeabilized microbial cell(s), one or more cell components of a microbial cell extract, partially purified enzyme, or purified enzyme. The enzyme catalyst may also be chemically modified (e.g., by pegylation or by reaction with crosslinking reagents). The perhydrolase catalyst may also be immobilized on a soluble or insoluble support using methods well-known to those skilled in the art; see for example, Immobilization of Enzymes and Cells; Gordon F. Bickerstaff, Editor; Humana Press, Totowa, NJ, USA; 1997. As described herein, all of the present enzymes having perhydrolysis activity are structurally members of the carbohydrate family esterase family 7 (CE-7 family) of enzymes (see Coutinho, P.M., Henrissat, B. "Carbohydrate-active enzymes: an integrated database approach" in Recent Advances in Carbohydrate Bioengineering, H.J. Gilbert, G. Davies, B. Henrissat and B. Svensson eds., (1999) The Royal Society of Chemistry, Cambridge, pp. 3-12.).

Members of the CE-7 family include cephalosporin C deacetylases (CAHs; E.C. 3.1.1.41) and acetyl xylan esterases (AXEs; E.C. 3.1.1.72). Members of the CE-7 esterase family share a conserved signature motif (Vincent et al., J. Mol. Biol., 330:593-606 (2003)). Perhydrolases comprising the CE-7 signature motif and/or a substantially similar structure are suitable for use in the present invention. Means to identify substantially similar biological molecules are well known in the art (e.g. sequence alignment protocols, nucleic acid hybridizations, presence of a conserved signature motif, etc.). In one aspect, the enzyme catalyst in the present process comprises a substantially similar enzyme having at least 90% identity, and most preferably at least 95% amino acid identity to the sequences defined in the claims. The nucleic acid molecules encoding the present CE-7 carbohydrate esterases are also disclosed herein. In a further embodiment, the perhydrolase catalyst useful in the present process is encoded by a nucleic acid molecule that hybridizes under stringent conditions to one of the present nucleic acid molecules.

As used herein, the terms "cephalosporin C deacetylase" and "cephalosporin C acetyl hydrolase" refers to an enzyme (E.C. 3.1.1.41) that catalyzes the deacetylation of cephalosporins such as cephalosporin C and 7-aminocephalosporanic acid (Mitsushima *et al., supra*)*.* As described herein, several cephalosporin C deacetylases are provided having significant perhydrolysis activity.

As used herein, "acetyl xylan esterases" "refers to an enzyme (E.C. 3.1.1.72; AXEs) that catalyzes the deacetylation of acetylated xylans and other acetylated saccharides. As illustrated herein, several enzymes classified as acetyl xylan esterases are provided having significant perhydrolase activity.

As used herein, the term *"Bacillus subtilis* (ATCC 31954™)" refers to a bacterial cell deposited to the American Type Culture Collection (ATCC) having international depository accession number ATCC 31954™. *Bacillus subtilis* ATCC 31954™ has been reported to have an ester hydrolase ("diacetinase") activity capable of hydrolyzing glycerol esters having 2 to 8 carbon acyl groups, especially diacetin (U.S. patent 4,444,886). As described herein, an enzyme having significant perhydrolase activity has been isolated from *B. subtilis* ATCC 31954™ and is provided as SEQ ID NO: 2. The amino acid sequence of the isolated enzyme has 100% amino acid identity to the cephalosporin C deacetylase provided by GenBank® Accession No. BAA01729.1.

As used herein, the term *"Bacillus subtilis* BE1010" refers to the strain of *Bacillus subtilis* as reported by Payne and Jackson (J. Bacteriol. 173:2278-2282 (1991)). *Bacillus subtilis* BE1010 is a derivative *Bacillus subtilis subsp. subtilis* strain BR151 (ATCC 33677™) having a chromosomal deletion in the genes encoding subtilisin and neutral protease. As described herein, an enzyme having significant perhydrolase activity has been isolated from *B. subtilis* BE1010 and is provided as SEQ ID NO: 6. The amino acid sequence of the isolated enzyme has 100% amino acid identity to the cephalosporin C deacetylase reported in *Bacillus subtilis subsp. subtilis* strain 168 (Kunst *et al., supra*).

As used herein, the term *"Bacillus subtilis* ATCC 29233™" refers to a strain of *Bacillus subtilis* deposited to the American Type Culture Collection (ATCC) having international depository accession number ATCC 31954™. As described herein, an enzyme having significant perhydrolase activity has been isolated and sequence from *B. subtilis* ATCC 29233™ and is provided as SEQ ID NO: 32.

As used herein, the term *"Clostridium thermocellum* ATCC 27405™" refers to a strain of *Clostridium thermocellum* deposited to the American Type Culture Collection (ATCC) having international depository accession number ATCC 27405™. The amino acid sequence of the enzyme having perhydrolase activity from *C*. *thermocellum* ATCC 27405™ is provided as SEQ ID NO: 14.

As used herein, the term *"Bacillus subtilis* ATCC 6633™ refers to a bacterial cell deposited to the American Type Culture Collection (ATCC) having international depository accession number ATCC 6633™. *Bacillus subtilis* ATCC 6633™ has been reported to have cephalosporin acetylhydrolase activity (U.S. patent 6,465,233). The amino acid sequence of the enzyme having perhydrolase activity from *B. subtilis* ATCC 6633™ is provided as SEQ ID NO: 8.

As used herein, the term *"Bacillus licheniformis* ATCC 14580™" refers to a bacterial cell deposited to the American Type Culture Collection (ATCC) having international depository accession number ATCC 14580™. *Bacillus licheniformis* ATCC 14580™ has been reported to have cephalosporin acetylhydrolase activity. The amino acid sequence of the enzyme having perhydrolase activity from *B. licheniformis* ATCC 14580™ is provided as SEQ ID NO: 10.

As used herein, the term *"Bacillus pumilus* PS213" refers to a bacterial cell reported to have acetyl xylan esterase activity (GENBANK® AJ249957). The amino acid sequence of the enzyme having perhydrolase activity from *Bacillus pumilus* PS213 is provided as SEQ ID NO: 12.

As used herein, the term *"Thermotoga neapolitana* " refers to a strain of *Thermotoga neapolitana* reported to have acetyl xylan esterase activity (GENBANK® 58632). The amino acid sequence of the enzyme having perhydrolase activity from *Thermotoga neapolitana* is provided as SEQ ID NO: 16.

As used herein, an "isolated nucleic acid molecule" and "isolated nucleic acid fragment" will be used interchangeably and refers to a polymer of RNA or DNA that is single- or double-stranded, optionally containing synthetic, non-natural or altered nucleotide bases. An isolated nucleic acid molecule in the form of a polymer of DNA may be comprised of one or more segments of cDNA, genomic DNA or synthetic DNA.

The term "amino acid" refers to the basic chemical structural unit of a protein or polypeptide. The following abbreviations are used herein to identify specific amino acids:

| Amino Acid | Three-Letter Abbreviation | One-Letter Abbreviation |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any amino acid or as defined herein | Xaa | X |

As used herein, "substantially similar" refers to nucleic acid molecules wherein changes in one or more nucleotide bases results in the addition, substitution, or deletion of one or more amino acids, but does not affect the functional properties (i.e. perhydrolytic activity) of the protein encoded by the DNA sequence. As used herein, "substantially similar" also refers to an enzyme having an amino acid sequence that is at least 40%, preferably at least 50%, more preferably at least 60%, more preferably at least 70%, even more preferably at least 80%, yet even more preferably at least 90%, and most preferably at least 95% identical to the sequences reported herein wherein the resulting enzyme retains the present functional properties (i.e., perhydrolytic activity). "Substantially similar" may also refer to an enzyme having perhydrolytic activity encoded by nucleic acid molecules that hybridizes under stringent conditions to the nucleic acid molecules reported herein. It is therefore understood that the invention encompasses more than the specific exemplary sequences.

For example, it is well known in the art that alterations in a gene which result in the production of a chemically equivalent amino acid at a given site, but do not affect the functional properties of the encoded protein are common. For the purposes of the present invention substitutions are defined as exchanges within one of the following five groups:
1. Small aliphatic, nonpolar or slightly polar residues: Ala, Ser, Thr (Pro, Gly);
2. Polar, negatively charged residues and their amides: Asp, Asn, Glu, Gln;
3. Polar, positively charged residues: His, Arg, Lys;
4. Large aliphatic, nonpolar residues: Met, Leu, Ile, Val (Cys); and
5. Large aromatic residues: Phe, Tyr, Trp.

Thus, a codon for the amino acid alanine, a hydrophobic amino acid, may be substituted by a codon encoding another less hydrophobic residue (such as glycine) or a more hydrophobic residue (such as valine, leucine, negatively charged residue for another (such as aspartic acid for glutamic acid) or one positively charged residue for another (such as lysine for arginine) can also be expected to produce a functionally equivalent product. In many cases, nucleotide changes which result in alteration of the N-terminal and C-terminal portions of the protein molecule would also not be expected to alter the activity of the protein.

Each of the proposed modifications is well within the routine skill in the art, as is determination of retention of biological activity of the encoded products. In one embodiment, substantially similar sequences are defined by their ability to hybridize, under stringent conditions (0.1 X SSC, 0.1 % SDS, 65°C and washed with 2X SSC, 0.1 % SDS followed by 0.1 X SSC, 0.1% SDS, 65°C) with the sequences exemplified herein. In one embodiment, disclosed herein are processes using enzymes having perhydrolase activity encoded by isolated nucleic acid molecules that hybridize under stringent conditions to the nucleic acid molecules reported herein. In a preferred embodiment, disclosed herein is an enzyme having perhydrolase activity encoded by isolated nucleic acid molecule that hybridize under stringent conditions to a nucleic acid molecule having an nucleic acid sequence selected from the group consisting of SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, and SEQ ID NO: 25.

As used herein, a nucleic acid molecule is "hybridizable" to another nucleic acid molecule, such as a cDNA, genomic DNA, or RNA, when a single strand of the first molecule can anneal to the other molecule under appropriate conditions of temperature and solution ionic strength. Hybridization and washing conditions are well known and exemplified in Sambrook, J. and Russell, D., T. Molecular Cloning: A Laboratory Manual, Third Edition, Cold Spring Harbor Laboratory Press, Cold Spring Harbor (2001). The conditions of temperature and ionic strength determine the "stringency" of the hybridization. Stringency conditions can be adjusted to screen for moderately similar molecules, such as homologous sequences from distantly related organisms, to highly similar molecules, such as genes that duplicate functional enzymes from closely related organisms. Post-hybridization washes typically determine stringency conditions.

Stringent hybridization conditions are0.1X SSC, 0.1% SDS, 65°C and washed with 2X SSC, 0.1 % SDS followed by a final wash of 0.1X SSC, 0.1% SDS, 65°C with the sequences exemplified herein.

Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of T*m* for hybrids of nucleic acids having those sequences. The relative stability (corresponding to higher T*m*) of nucleic acid hybridizations decreases in the following order: RNA:RNA, DNA:RNA, DNA:DNA. For hybrids of greater than 100 nucleotides in length, equations for calculating T*m* have been derived (Sambrook and Russell, *supra*)*.* For hybridizations with shorter nucleic acids, *i.e.,* oligonucleotides, the position of mismatches becomes more important, and the length of the oligonucleotide determines its specificity (Sambrook and Russell, *supra*)*.* In one aspect, the length for a hybridizable nucleic acid is at least about 10 nucleotides. Preferably, a minimum length for a hybridizable nucleic acid is at least about 15 nucleotides in length, more preferably at least about 20 nucleotides in length, even more preferably at least 30 nucleotides in length, even more preferably at least 300 nucleotides in length, and most preferably at least 800 nucleotides in length. Furthermore, the skilled artisan will recognize that the temperature and wash solution salt concentration may be adjusted as necessary according to factors such as length of the probe.

As used herein, the term "percent identity" is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in: Computational Molecular Biology (Lesk, A. M., ed.) Oxford University Press, NY (1988); Biocomputing: Informatics and Genome Projects (Smith, D. W., ed.) Academic Press, NY (1993); Computer Analysis of Sequence Data, Part I (Griffin, A. M., and Griffin, H. G., eds.) Humana Press, NJ (1994); Sequence Analysis in Molecular Biology (von Heinje, G., ed.) Academic Press (1987); and Sequence Analysis Primer (Gribskov, M. and Devereux, J., eds.) Stockton Press, NY (1991). Methods to determine identity and similarity are codified in publicly available computer programs. Sequence alignments and percent identity calculations may be performed using the Megalign program of the LASERGENE bioinformatics computing suite (DNASTAR Inc., Madison, WI), the AlignX program of Vector NTI v. 7.0 (Informax, Inc., Bethesda, MD), or the EMBOSS Open Software Suite (EMBL-EBI; Rice et al., Trends in Genetics 16, (6) pp276-277 (2000)). Multiple alignment of the sequences can be performed using the Clustal method (i.e. CLUSTALW; for example version 1.83) of alignment (Higgins and Sharp, CABIOS, 5:151-153 (1989); Higgins et al., Nucleic Acids Res. 22:4673-4680 (1994); and Chenna et al., Nucleic Acids Res 31 (13):3497-500 (2003)), available from the European Molecular Biology Laboratory via the European Bioinformatics Institute) with the default parameters. Suitable parameters for CLUSTALW protein alignments include GAP Existence penalty=15, GAP extension =0.2, matrix = Gonnet (e.g. Gonnet250), protein ENDGAP = -1, Protein GAPDIST=4, and KTUPLE=1. In one embodiment, a fast or slow alignment is used with the default settings where a slow alignment is preferred. Alternatively, the parameters using the CLUSTALW method (version 1.83) may be modified to also use KTUPLE =1, GAP PENALTY=10, GAP extension =1, matrix = BLOSUM (e.g. BLOSUM64), WINDOW=5, and TOP DIAGONALS SAVED=5.

In one aspect of the present invention, suitable isolated nucleic acid molecules (isolated polynucleotides of the present invention) encode a polypeptide having an amino acid sequence that is at least 90%, and most preferably at least 95% identical to the amino acid sequences reported herein. Suitable nucleic acid molecules of the present invention not only have the above homologies, but also typically encode a polypeptide having about 300 to about 340 amino acids, more preferably about 310 to about 330 amino acids, and most preferably about 318 amino acids.

As used herein, the terms "signature motif", "CE-7 signature motif, and "diagnostic motif" refer to conserved structures shared among a family of enzymes having a defined activity. The signature motif can be used to define and/or identify the family of structurally related enzymes having similar enzymatic activity for a defined family of substrates. The signature motif can be a single contiguous amino acid sequence or a collection of discontiguous, conserved motifs that together form the signature motif. Typically, the conserved motif(s) is represented by an amino acid sequence. As described herein, the present perhydrolases belong to the family of CE-7 carbohydrate esterases. This family of enzymes can be defined by the presence of a signature motif (Vincent et al., *supra*)*.* As defined herein, the signature motif for CE-7 esterases comprises 3 conserved motifs (residue position numbering relative to reference sequence SEQ ID NO: 2):
a) Arg118-Gly119-Gln120;
b) Gly179-Xaa180-**Ser181**-Gln182-Gly183; and
c) **His298**-Glu299.

The Xaa at amino acid residue position 180 is typically glycine or alanine. Amino acid residues belonging to the catalytic triad are in bold.

Further analysis of the conserved motifs within the CE-7 carbohydrate esterase family indicates the presence of an additional conserved motif (LXD at amino acid positions 267-269 of SEQ ID NO: 2) that may be to further define a perhydrolase belonging to the CE-7 carbohydrate esterase family (Figures 1a-c). In a further embodiment, the signature motif defined above includes a forth conserved motif defined as:
Leu267-Xaa268**-Asp269**;

The Xaa at amino acid residue position 268 is typically isoleucine, valine, or methionine. The forth motif includes the aspartic acid residue (bold) belonging to the catalytic triad (Ser181-Asp269-His298).

A number of well-known global alignment algorithms (i.e. sequence analysis software) may be used to align two or more amino acid sequences representing enzymes having perhydrolase activity to determine is the enzyme is comprised of the present signature motif. The aligned sequence(s) are compared to the reference sequence (SEQ ID NO: 2) to determine the existence of the signature motif. In one embodiment, a CLUSTAL alignment (CLUSTALW) using a reference amino acid sequence (as used herein the perhydrolase sequence (SEQ ID NO: 2) from the *Bacillus subtilis* ATCC 31954™) is used to identify perhydrolases belonging to the CE-7 esterase family. The relative numbering of the conserved amino acid residues is based on the residue numbering of the reference amino acid sequence to account for small insertions or deletions (for example, 5 amino acids of less) within the aligned sequence.

Examples of other suitable algorithms that may be used to identify sequences comprising the present signature motif (when compared to the reference sequence) include, but are not limited to Needleman and Wunsch (J. Mol. Biol. 48, 443-453 (1970); a global alignment tool) and Smith-Waterman (J. Mol. Biol. 147:195-197 (1981); a local alignment tool). In one embodiment, a Smith-Waterman alignment is implemented using default parameters. An example of suitable default parameters include the use of a BLOSUM62 scoring matrix with GAP open penalty = 10 and a GAP extension penalty = 0.5.

A comparison of the overall percent identity among perhydrolases exemplified herein indicates that enzymes having as little as 42% identity to SEQ ID NO: 2 (while retaining the signature motif) exhibit significant perhydrolase activity and are structurally classified as CE-7 carbohydrate esterases.

Alternatively, a contiguous amino acid sequence comprising the region encompassing the conserved motifs (i.e. amino acid residues 118-299 of SEQ ID NO: 2; provided separately as SEQ ID NO: 63) may also be used to identify CE-7 carbohydrate esterase family members.

As used herein, "codon degeneracy" refers to the nature of the genetic code permitting variation of the nucleotide sequence without affecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

As used herein, "synthetic genes" can be assembled from oligonucleotide building blocks that are chemically synthesized using procedures known to those skilled in the art. These building blocks are ligated and annealed to form gene segments that are then enzymatically assembled to construct the entire gene. "Chemically synthesized", as pertaining to a DNA sequence, means that the component nucleotides were assembled *in vitro.* Manual chemical synthesis of DNA may be accomplished using well-established procedures, or automated chemical synthesis can be performed using one of a number of commercially available machines. Accordingly, the genes can be tailored for optimal gene expression based on optimization of nucleotide sequences to reflect the codon bias of the host cell. The skilled artisan appreciates the likelihood of successful gene expression if codon usage is biased towards those codons favored by the host. Determination of preferred codons can be based on a survey of genes derived from the host cell where sequence information is available.

As used herein, "gene" refers to a nucleic acid molecule that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different from that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

As used herein, "coding sequence" refers to a DNA sequence that codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, RNA processing site, effector binding site and stem-loop structure.

As used herein, "promoter" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene at different stages of development, or in response to different environmental or physiological conditions. Promoters that cause a gene to be expressed at most times are commonly referred to as "constitutive promoters". It is further recognized that since in most cases the exact boundaries of regulatory sequences have not been completely defined, DNA fragments of different lengths may have identical promoter activity.

As used herein, the "3' non-coding sequences" refer to DNA sequences located downstream of a coding sequence and include polyadenylation recognition sequences (normally limited to eukaryotes) and other sequences encoding regulatory signals capable of affecting mRNA processing or gene expression. The polyadenylation signal is usually characterized by affecting the addition of polyadenylic acid tracts (normally limited to eukaryotes) to the 3' end of the mRNA precursor.

As used herein, the term "operably linked" refers to the association of nucleic acid sequences on a single nucleic acid molecule so that the function of one is affected by the other. For example, a promoter is operably linked with a coding sequence when it is capable of affecting the expression of that coding sequence, *i.e.,* that the coding sequence is under the transcriptional control of the promoter. Coding sequences can be operably linked to regulatory sequences in sense or antisense orientation.

As used herein, the term "expression" refers to the transcription and stable accumulation of sense (mRNA) or antisense RNA derived from the nucleic acid molecule of the invention. Expression may also refer to translation of mRNA into a polypeptide.

As used herein, "transformation" refers to the transfer of a nucleic acid molecule into the genome of a host organism, resulting in genetically stable inheritance. In the present invention, the host cell's genome includes chromosomal and extrachromosomal (e.g. plasmid) genes. Host organisms containing the transformed nucleic acid molecules are referred to as "transgenic" or "recombinant" or "transformed" organisms.

As used herein, the terms "plasmid", "vector" and "cassette" refer to an extrachromosomal element often carrying genes which are not part of the central metabolism of the cell, and usually in the form of circular double-stranded DNA molecules. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell. "Transformation cassette" refers to a specific vector containing a foreign gene and having elements in addition to the foreign gene that facilitate transformation of a particular host cell. "Expression cassette" refers to a specific vector containing a foreign gene and having elements in addition to the foreign gene that allow for enhanced expression of that gene in a foreign host.

As used herein, the term "sequence analysis software" refers to any computer algorithm or software program that is useful for the analysis of nucleotide or amino acid sequences. "Sequence analysis software" may be commercially available or independently developed. Typical sequence analysis software will include, but is not limited to, the GCG suite of programs (Wisconsin Package Version 9.0, Genetics Computer Group (GCG), Madison. WI), BLASTP, BLASTN, BLASTX (Altschul et al., J. Mol. Biol. 215:403-410 (1990), and DNASTAR (DNASTAR, Inc. 1228 S. Park St. Madison, WI 53715 USA), CLUSTALW (for example, version 1.83; Thompson et al., Nucleic Acids Research, 22(22):4673-4680 (1994), and the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Publisher: Plenum, New York, NY), Vector NTI (Informax, Bethesda, MD) and Sequencher v. 4.05. Within the context of this application it will be understood that where sequence analysis software is used for analysis, that the results of the analysis will be based on the "default values" of the program referenced, unless otherwise specified. As used herein "default values" will mean any set of values or parameters set by the software manufacturer that originally load with the software when first initialized.

As used herein, the term "biological contaminants" refers to one or more unwanted and/or pathogenic biological entities including, but not limited to microorganisms, spores, viruses, prions, and mixtures thereof. The process produces an efficacious concentration of at least one percarboxylic acid useful to reduce and/or eliminate the presence of the viable biological contaminants. In a preferred embodiment, the microbial contaminant is a viable pathogenic microorganism.

As used herein, the term "disinfect" refers to the process of destruction of or prevention of the growth of biological contaminants. As used herein, the term "disinfectant" refers to an agent that disinfects by destroying, neutralizing, or inhibiting the growth of biological contaminants. Typically, disinfectants are used to treat inanimate objects or surfaces. As used herein, the term "antiseptic" refers to a chemical agent that inhibits the growth of disease-carrying microorganisms. In one aspect of the embodiment, the biological contaminants are pathogenic microorganisms.

As used herein, the term "virucide" refers to an agent that inhibits or destroys viruses, and is synonymous with "viricide". An agent that exhibits the ability to inhibit or destroy viruses is described as having "virucidal" activity. Peracids can have virucidal activity. Typical alternative virucides known in the art which may be suitable for use with the present invention include, for example, alcohols, ethers, chloroform, formaldehyde, phenols, beta propiolactone, iodine, chlorine, mercury salts, hydroxylamine, ethylene oxide, ethylene glycol, quaternary ammonium compounds, enzymes, and detergents.

As used herein, the term "biocide" refers to a chemical agent, typically broad spectrum, which inactivates or destroys microorganisms. A chemical agent that exhibits the ability to inactivate or destroy microorganisms is described as having "biocidal" activity. Peracids can have biocidal activity. Typical alternative biocides known in the art, which may be suitable for use in the present invention include, for example, chlorine, chlorine dioxide, chloroisocyanurates, hypochlorites, ozone, acrolein, amines, chlorinated phenolics, copper salts, organo-sulphur compounds, and quaternary ammonium salts.

As used herein, the phrase "minimum biocidal concentration" refers to the minimum concentration of a biocidal agent that, for a specific contact time, will produce a desired lethal, irreversible reduction in the viable population of the targeted microorganisms. The effectiveness can be measured by the log₁₀ reduction in viable microorganisms after treatment. In one aspect, the targeted reduction in viable microorganisms after treatment is at least a 3-log reduction, more preferably at least a 4-log reduction, and most preferably at least a 5-log reduction. In another aspect, the minimum biocidal concentration is at least a 6-log reduction in viable microbial cells.

As used herein, the terms "peroxygen source" and "source of peroxygen" refer to compounds capable of providing hydrogen peroxide at a concentration of about 1 mM or more when in an aqueous solution including, but not limited to hydrogen peroxide, hydrogen peroxide adducts (e.g., urea-hydrogen peroxide adduct (carbamide peroxide)), perborates, and percarbonates. As described herein, the concentration of the hydrogen peroxide provided by the peroxygen compound in the aqueous reaction mixture is initially at least 1 mM or more upon combining the reaction components. In one embodiment, the hydrogen peroxide concentration in the aqueous reaction mixture is at least 10 mM. In another embodiment, the hydrogen peroxide concentration in the aqueous reaction mixture is at least 100 mM. In another embodiment, the hydrogen peroxide concentration in the aqueous reaction mixture is at least 200 mM. In another embodiment, the hydrogen peroxide concentration in the aqueous reaction mixture is 500 mM or more. In yet another embodiment, the hydrogen peroxide concentration in the aqueous reaction mixture is 1000 mM or more. The molar ratio of the hydrogen peroxide to enzyme substrate, e.g. triglyceride, (H₂O₂aubstrate) in the aqueous reaction mixture may be from about 0.002 to 20, preferably about 0.1 to 10, and most preferably about 0.5 to 5.

### Suitable Reaction Conditions for the Enzyme-catalyzed Preparation of Peracids from Carboxylic Acid Esters and Hydrogen Peroxide

In one aspect of the invention, a process is provided to produce an aqueous mixture comprising a peracid by reacting carboxylic acid esters and an inorganic peroxide, not limited to hydrogen peroxide, sodium perborate or sodium percarbonate, in the presence of an enzyme catalyst having perhydrolysis activity. In one embodiment, the enzyme catalyst comprises a perhydrolase having a structure belonging to the CE-7 carbohydrate esterase family. In another embodiment, the perhydrolase catalyst is structurally classified as a cephalosporin C deacetylase. In another embodiment, the perhydrolase catalyst is structurally classified as an acetyl xylan esterase.

In an embodiment, the perhydrolase catalyst comprises an enzyme having perhydrolase activity selected from the group consisting of SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, and SEQ ID NO: 26.

In another embodiment, the perhydrolase catalyst comprises an enzyme having an amino acid sequence selected from the group consisting of SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, and SEQ ID NO: 26.

Suitable carboxylic acid esters have a formula selected from the group consisting of:
a) esters of the formula 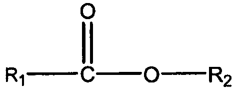 wherein R₁= C1 to C7 straight chain or branched chain alkyl optionally substituted with an hydroxyl or a C1 to C4 alkoxy group and R₂= C1 to C10 straight chain or branched chain alkyl, alkenyl, alkynyl, aryl, alkylaryl, alkylheteroaryl, heteroaryl, (CH₂CH₂-O)ₙH or (CH₂CH(CH₃)-O)ₙH and n=1 to 10;
b) glycerides of the formula 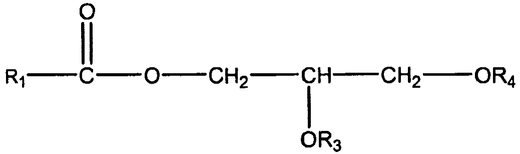 wherein R₁= C1 to C7 straight chain or branched chain alkyl optionally substituted with an hydroxyl or a C1 to C4 alkoxy group and R₃ and R₄ are individually H or R₁C(O); and
c) acetylated carbohydrates.

In a preferred embodiment, the acetylated carbohydrates include acetylated mono- , di-, and polysaccharides. In another embodiment, the acetylated carbohydrates are selected from the group consisting of acetylated xylan, fragments of acetylated xylan, acetylated xylose(such as xylose tetraacetate), acetylated glucose (such as glucose pentaacetate), β-D-ribofuranose-1,2,3,5-tetraacetate, tri-O-acetyl-D-galactal, and tri-O-acetyl-D-glucal, and acetylated cellulose. In a preferred embodiment, the acetylated carbohydrate is selected from the group consisting of P-D-ribofuranose-1,2,3,5-tetraacetate, tri-O-acetyl-D-galactal, and tri-O-acetyl-D-glucal, and acetylated cellulose. As such, acetylated carbohydrates may be suitable substrates for generating percarboxylic acids using the present process (i.e., in the presence of a peroxygen source).

In another aspect, the carboxylic acid ester is selected from the group consisting of ethyl acetate, methyl lactate, ethyl lactate, methyl glycolate, ethyl glycolate, methyl methoxyacetate, ethyl methoxyacetate, methyl 3-hydroxybutyrate, ethyl 3-hydroxybutyrate, triethyl 2-acetyl citrate, glucose pentaacetate, gluconolactone, glycerides (mono-, di-, and triglycerides) such as monoacetin, diacetin, triacetin, monopropionin, dipropionin (glyceryl dipropionate), tripropionin (1,2,3-tripropionylglycerol), monobutyrin, dibutyrin (glyceryl dibutyrate), tributyrin (1,2,3-tributyrylglycerol), acetylated saccharides, and mixtures thereof. In another aspect, the carboxylic acid ester substrates are selected from the group consisting of monoacetin, diacetin, triacetin, monopropionin, dipropionin, tripropionin, monobutyrin, dibutyrin, tributyrin, ethyl acetate, and ethyl lactate. In yet another aspect, the carboxylic acid ester substrates are selected from the group consisting of diacetin, triacetin, ethyl acetate, and ethyl lactate. In a preferred aspect, the carboxylic acid ester is a glyceride selected from the group consisting of monoacetin, diacetin, triacetin, and mixtures thereof.

The carboxylic acid ester is present in the reaction mixture at a concentration sufficient to produce the desired concentration of peracid upon enzyme-catalyzed perhydrolysis. The carboxylic acid ester need not be completely soluble in the reaction mixture, but has sufficient solubility to permit conversion of the ester by the perhydrolase catalyst to the corresponding peracid. The carboxylic acid ester is present in the reaction mixture at a concentration of 0.05 wt % to 40 wt % of the reaction mixture, preferably at a concentration of 0.1 wt % to 20 wt % of the reaction mixture, and more preferably at a concentration of 0.5 wt % to 10 wt % of the reaction mixture.

The peroxygen source may include, but is not limited to, hydrogen peroxide, hydrogen peroxide adducts (e.g., urea-hydrogen peroxide adduct (carbamide peroxide)) perborate salts and percarbonate salts. The concentration of peroxygen compound in the reaction mixture may range from 0.0033 wt % to about 50 wt %, preferably from 0.033 wt % to about 40 wt %, more preferably from 0.33 wt % to about 30 wt %.

Many perhydrolase catalysts (whole cells, permeabilized whole cells, and partially purified whole cell extracts) have been reported to have catalase activity (EC 1.11.1.6). Catalases catalyze the conversion of hydrogen peroxide into oxygen and water. In one aspect, the perhydrolysis catalyst lacks catalase activity. In another aspect, a catalase inhibitor is added to the reaction mixture. Examples of catalase inhibitors include, but are not limited to, sodium azide and hydroxylamine sulfate. One of skill in the art can adjust the concentration of catalase inhibitor as needed. The concentration of the catalase inhibitor typically ranges from 0.1 mM to about 1 M; preferably about 1 mM to about 50 mM; more preferably from about 1 mM to about 20 mM. In one aspect, sodium azide concentration typically ranges from about 20 mM to about 60 mM while hydroxylamine sulfate is concentration is typically about 0.5 mM to about 30 mM, preferably about 10 mM.

In another embodiment, the enzyme catalyst lacks significant catalase activity or is engineered to decrease or eliminate catalase activity. The catalase activity in a host cell can be down-regulated or eliminated by disrupting expression of the gene(s) responsible for the catalase activity using well known techniques including, but not limited to, transposon mutagenesis, RNA antisense expression, targeted mutagenesis, and random mutagenesis. In a preferred embodiment, the gene(s) encoding the endogenous catalase activity are down-regulated or disrupted (i.e. knocked-out). As used herein, a "disrupted" gene is one where the activity and/or function of the protein encoded by the modified gene is no longer present. Means to disrupt a gene are well-known in the art and may include, but are not limited to insertions, deletions, or mutations to the gene so long as the activity and/or function of the corresponding protein is no longer present. In a further preferred embodiment, the production host is an *E*. *coli* production host comprising a disrupted catalase gene selected from the group consisting of *kat*G (SEQ ID NO: 47) and *kat*E (SEQ ID NO: 56). In another embodiment, the production host is an *E. coli* strain comprising a down-regulation and/or disruption in both *kat*g1 and a *kat*E catalase genes. An *E. coli* strain comprising a double-knockout of *kat*G and *kat*E is provided herein (see Example 15; *E.* coli strain KLP18).

The catalase negative *E. coli* strain KLP18 (*kat*G and *kat*E double knockout) that was constructed (Example 15) was demonstrated to be a superior host for large scale (10-L and greater) production of perhydrolase enzymes compared to the catalase negative strain UM2 (*E*. *coli* Genetic Stock Center #7156, Yale University, New Haven CT), as determined by growth under fermenter conditions (Examples 17-19). Although both KLP18 and UM2 are catalase-negative strains, UM2 is known to have numerous nutritional auxotrophies, and therefore requires media that is enriched with yeast extract and peptone. Even when employing enriched media for fermentation, UM2 grew poorly and to a limited maximum cell density (OD). In contrast, KLP18 had no special nutritional requirements and grew to high cell densities on mineral media alone or with additional yeast extract (Example 20).

The concentration of the catalyst in the aqueous reaction mixture depends on the specific catalytic activity of the catalyst, and is chosen to obtain the desired rate of reaction. The weight of catalyst in perhydrolysis reactions typically ranges from 0.0005 mg to 10 mg per mL of total reaction volume, preferably from 0.010 mg to 2.0 mg per mL. The catalyst may also be immobilized on a soluble or insoluble support using methods well-known to those skilled in the art; see for example, Immobilization of Enzymes and Cells; Gordon F. Bickerstaff, Editor; Humana Press, Totowa, NJ, USA; 1997. The use of immobilized catalysts permits the recovery and reuse of the catalyst in subsequent reactions. The enzyme catalyst may be in the form of whole microbial cells, permeabilized microbial cells, microbial cell extracts, partially-purified or purified enzymes, and mixtures thereof.

In one aspect, the concentration of peracid generated by the combination of chemical perhydrolysis and enzymatic perhydrolysis of the carboxylic acid ester is sufficient to provide an effective concentration of peracid for bleaching or disinfection at a desired pH. In another aspect, the present methods provide combinations of enzymes and enzyme substrates to produce the desired effective concentration of peracid, where, in the absence of added enzyme, there is a significantly lower concentration of peracid produced. Although there may in some cases be substantial chemical perhydrolysis of the enzyme substrate by direct chemical reaction of inorganic peroxide with the enzyme substrate, there may not be a sufficient concentration of peracid generated to provide an effective concentration of peracid in the desired applications, and a significant increase in total peracid concentration is achieved by the addition of an appropriate perhydrolase catalyst to the reaction mixture.

The concentration of peracid generated (*e.g*. peracetic acid) by the perhydrolysis of at least one carboxylic acid ester is at least about 20 ppm, preferably at least 100 ppm, more preferably at least about 200 ppm peracid, more preferably at least 300 ppm, more preferably at least 500 ppm. more preferably at least 700 ppm, more preferably at least about 1000 ppm peracid, most preferably at least 2000 ppm peracid within 10 minutes, preferably within 5 minutes, of initiating the perhydrolysis reaction. The product mixture comprising the peracid may be optionally diluted with water, or a solution predominantly comprised of water, to produce a mixture with the desired lower concentration of peracid. In one aspect, the reaction time required to produce the desired concentration of peracid is not greater than about two hours, preferably not greater than about 30 minutes, more preferably not greater than about 10 minutes, and most preferably in about 5 minutes or less. In other aspects, a hard surface or inanimate object contaminated with a concentration of a microbial population is contacted with the peracid formed in accordance with the processes described herein within about 5 minutes to about 168 hours of combining said reaction components, or within about 5 minutes to about 48 hours, or within about 5 minutes to 2 hours of combining said reaction components, or any such time interval therein.

The temperature of the reaction is chosen to control both the reaction rate and the stability of the enzyme catalyst activity. The temperature of the reaction may range from just above the freezing point of the reaction mixture (approximately 0 °C) to about 75 °C, with a preferred range of reaction temperature of from about 5 °C to about 55 °C.

The pH of the final reaction mixture containing peracid is from about 2 to about 9, preferably from about 3 to about 8, more preferably from about 5 to about 8, even more preferably about 6 to about 8, and yet even more preferably about 6.5 to about 7.5. In another embodiment, the pH of the reaction mixture is acidic (pH <7). The pH of the reaction, and of the final reaction mixture, may optionally be controlled by the addition of a suitable buffer, including, but not limited to phosphate, pyrophosphate, bicarbonate, acetate, or citrate. The concentration of buffer, when employed, is typically from 0.1 mM to 1.0 M, preferably from 1 mM to 300 mM, most preferably from 10 mM to 100 mM.

In another aspect, the enzymatic perhydrolysis product may contain additional components that provide desirable functionality. These additional components include, but are not limited to buffers, detergent builders, thickening agents, emulsifiers, surfactants, wetting agents, corrosion inhibitors (e.g., benzotriazole), enzyme stabilizers, and peroxide stabilizers (*e.g.*, metal ion chelating agents). Many of the additional components are well known in the detergent industry (see for example U.S. Patent 5,932,532). Examples of emulsifiers include, but are not limited to polyvinyl alcohol or polyvinylpyrrolidone. Examples of thickening agents include, but are not limited to LAPONITE® RD, corn starch, PVP, CARBOWAX®, CARBOPOL®, CABOSIL®, polysorbate 20, PVA, and lecithin. Examples of buffering systems include, but are not limited to sodium phosphate monobasic/sodium phosphate dibasic; sulfamic acid/triethanolamine; citric acid/triethanolamine; tartaric acid/triethanolamine; succinic acid/triethanolamine; and acetic acid/triethanolamine. Examples of surfactants include, but are not limited to a) non-ionic surfactants such as block copolymers of ethylene oxide or propylene oxide, ethoxylated or propoxylated linear and branched primary and secondary alcohols, and aliphatic phosphine oxides b) cationic surfactants such as quaternary ammonium compounds, particularly quaternary ammonium compounds having a C8-C20 alkyl group bound to a nitrogen atom additionally bound to three C1-C2 alkyl groups, c) anionic surfactants such as alkane carboxylic acids (e.g., C8-C20 fatty acids), alkyl phosphonates, alkane sulfonates (e.g., sodium dodecylsulphate "SDS") or linear or branched alkyl benzene sulfonates, alkene sulfonates and d) amphoteric and zwitterionic surfactants such as aminocarboxylic acids, aminodicarboxylic acids, alkybetaines, and mixtures thereof. Additional components may include fragrances, dyes, stabilizers of hydrogen peroxide (*e.g*., metal chelators such as 1-hydroxyethylidene-1,1-diphosphonic acid (DEQUEST® 2010, Solutia Inc., St. Louis, MO and ethylenediaminetetraacetic acid (EDTA)), TURPINAL® SL, DEQUEST® 0520, DEQUEST® 0531, stabilizers of enzyme activity (*e.g*., polyethyleneglycol (PEG)), and detergent builders.

### In Situ Production of Peracids using a Perhydrolase Catalyst

Cephalosporin C deacetylases (E.C. 3.1.1.41; systematic name cephalosporin C acetylhydrolases; CAHs) are enzymes having the ability to hydrolyze the acetyl ester bond on cephalosporins such as cephalosporin C, 7-aminocephalosporanic acid, and 7-(thiophene-2-acetamido)cephalosporanic acid (Abbott, B. and Fukuda, D., Appl. Microbiol. 30(3):413-419 (1975)). CAHs belong to a larger family of structurally related enzymes referred to as the carbohydrate esterase family seven (CE-7; see Coutinho, P.M., Henrissat, B. "Carbohydrate-active enzymes: an integrated database approach" in Recent Advances in Carbohydrate Bioengineering, H.J. Gilbert, G. Davies, B. Henrissat and B. Svensson eds., (1999) The Royal Society of Chemistry, Cambridge, pp. 3-12.)

The CE-7 family includes both CAHs and acetyl xylan esterases (AXEs; E.C. 3.1.1.72). CE-7 family members share a common structural motif and are quite unusual in that they typically exhibit ester hydrolysis activity for both acetylated xylooliogsaccharides and cephalosporin C, suggesting that the CE-7 family represents a single class of proteins with a multifunctional deacetylase activity against a range of small substrates (Vincent et al., J. Mol. Biol., 330:593-606 (2003)). Vincent *et al.* describes the structural similarity among the members of this family and defines a signature sequence motif characteristic of the CE-7 family.

Members of the CE-7 family are found in plants, fungi (e.g., *Cephalosporidium acremonium*), yeasts (e.g., *Rhodosporidium toruloides*, *Rhodotorula glutinis*), and bacteria such as *Thermoanaerobacterium* sp.; *Norcardia lactamdurans*, and various members of the genus *Bacillus* (Politino et al., Appl. Environ. Microbiol., 63(12):4807-4811 (1997); Sakai et al., J. Ferment. Bioeng. 85:53-57 (1998); Lorenz, W. and Wiegel, J., J. Bacteriol 179:5436-5441 (1997); Cardoza et al., Appl. Microbiol. Biotechnol., 54(3):406-412 (2000); Mitshushima *et al.*, *supra*, Abbott, B. and Fukuda, D., Appl. Microbiol. 30(3):413-419 (1975); Vincent *et al., supra,* Takami et al., NAR, 28(21):4317-4331 (2000); Rey et al., Genome Biol., 5(10): article 77 (2004); Degrassi et al., Microbiology., 146:1585-1591 (2000); U.S. Patent 6,645,233;. U.S. Patent 5,281,525; U.S. Patent 5,338,676; and WO 99/03984. A non-comprehensive list of CE-7 carbohydrate esterase family members having significant homology to SEQ ID NO: 2 are provided in Table 1.

**Table 1. Example of CE-7 Enzymes Having Significant Homology to SEQ ID NO:2.**

| Source Organism (GENBANK® Accession No. of the CE-7 enzyme) | Nucleotide Sequence (SEQ ID NO:) | Amino Acid Sequence (SEQ ID NO:) | % Amino Acid Identity to SEQ ID NO: 2. | Reference |
|---|---|---|---|---|
| *B. subtilis* ATCC 31954™ | 1 | 2 | 100 | *B. subtilis* SHS 0133 Mitshushima *et al. supra* |
| *B. subtilis subsp. subtilis* str. 168 (NP_388200) | 5 | 6 | 98 | Kunst *et al., supra.* WO99/03984 |
| *B. subtilis* BE1010 | | | | Payne and Jackson, J. Bacteriol. 173:2278-2282 (1991)) |
| *B. subtilis* ATCC 6633 (YP_077621.1) | 7 | 8 | 96 | U.S. 6,465,233 |
| *B. licheniformis* ATCC 14580 (YP_077621.1) | 9 | 10 | 77 | Rey *et al., supra* |
| *B. pumilus* PS213 (CAB76451.2) | 11, 60 | 12 | 76 | Degrassi *et al., supra* |
| *Clostridium thermocellum* ATCC 27405 (ZP_00504991) | 13 | 14 | 57 | Copeland *et al.* US Dept. of Energy Joint Genome Institute (JGI-PGF) Direct Submission GENBANK® ZP 00504991 |
| *Thermotoga neapolitana* (AAB70869.1) | 15, 41 | 16 | 42 | See GENBANK® AAB70869.1 |
| *Thermotoga maritima* MSB8 (NP_227893.1) | 17 | 18 | 42 | Nelson et al., Nature 399 (6734):323-329 (1999) |
| *Bacillus sp.* NRRL B-14911 (ZP_01168674) | 21 | 22 | 40 | Siefert *et al.* J. Craig Venter Institute. Direct Submission Under GENBANK® ZP 01168674 |
| *Thermoanaerobac terium* sp. (AAB68821.1) | 19 | 20 | 37 | Lorenz and Wiegel, *supra* |
| *Bacillus halodurans* C-125 (NP_244192) | 23 | 24 | 36 | Takami *et al., supra* |
| *Bacillus clausii* KSM-K16 (YP_175265) | 25 | 26 | 33 | Kobayashi et al., Appl. Microbiol. Biotechnol. 43 (3), 473-481 (1995) |

The present perhydrolases are all members of the CE-7 carbohydrate esterase family. As described by Vincent *et al.* (*supra*), members of the family share a common signature motif that is characteristic of this family. A CLUSTALW alignment of the present perhydrolases illustrates that all of the members belong to the CE-7 carbohydrate esterase family (Figures 1a-c). A comparison of the overall percent amino acid identity amount the present perhydrolases is provided in Table 2.

**Table 2: Percent Amino Acid Identity Between Perhydrolases¹**

| | *B. subtilis* ATCC 31954 | *B. subtilis* BE1010 | *B. subtilis* ATCC 29233 | *B. subtilis* ATCC 6633 | *B. Licheniformis* ATCC 14580 | *B. pumilus* PS213 | C. *Thermocellum* ATCC 27405 | *Thermotoga Neapolitana* |
|---|---|---|---|---|---|---|---|---|
| *B. subtilis* ATCC 31954 (SEQ ID NO:2) | 100 | 98 | 99 | 96 | 77 | 76 | 57 | 42 |
| *B. subtilis* BE1010 (SEQ ID NO:6) | 98 | 100 | 99 | 96 | 76 | 77 | 57 | 43 |
| *B. subtilis* ATCC 29233 (SEQ ID NO :34 ) | 99 | 99 | 100 | 96 | 77 | 76 | 57 | 43 |
| *B. subtilis* ATCC 6633 (SEQ ID NO:8) | 96 | 96 | 96 | 100 | 76 | 76 | 56 | 43 |
| *B. licheniformis* ATCC 14580 (SEQ ID NO:10) | 77 | 76 | 77 | 76 | 100 | 69 | 56 | 45 |
| *B. pumilus* PS213 (SEQ ID NO: 12) | 76 | 77 | 76 | 76 | 69 | 100 | 57 | 42 |
| *Clostridium Thermocellum* ATCC 27405 (SEQ ID NO: 14) | 57 | 57 | 57 | 56 | 56 | 57 | 100 | 45 |
| *Thermotoga neapolitana* (SEQ ID NO: 16 ) | 42 | 43 | 43 | 43 | 45 | 42 | 45 | 100 |

| | | | | | | | | |
|---|---|---|---|---|---|---|---|---|
| =Percent identity determined using blast2seq algorithm using BLOSUM62, gap open=11, gap extension = 1, x_drop = 0, expect=10, and wordsize=3. Tatiana A. Tatusova, Thomas L. Madder (1999), "Blast 2 sequences - a new tool for comparing protein and nucleotide sequences", *FEMS Microbiol Lett.* 174:247-250 | | | | | | | | |

Although variation is observed in terms of overall percent amino acid identity (*i.e.* the *Clostridium thermocellum* ATCC 27405™ perhydrolase; SEQ ID NO: 14 shares only 57% amino acid identity with the *Bacillus subtilis* ATCC 31954™ perhydrolase; SEQ ID NO: 2 while the *Thermotoga neapolitana* perhydrolase (SEQ ID NO: 16) shares only 42% identity with SEQ ID NO: 2), each of the present perhydrolase enzymes share the CE-7 signature motif. Accordingly, the perhydrolase catalyst of the present invention is an enzyme structurally classified as belonging to the CE-7 carbohydrate esterase family. Each of the present perhydrolase enzymes comprise the CE-7 signature (diagnostic) motif.

Vincent *et al.* (*supra*) analyzed the structure CE-7 esterases and has identified several highly conserved motifs that are diagnostic for the family. As shown in Figure 1, the highly conserved motifs (underlined in Figure 1; position numbering relative to SEQ ID NO: 2) include the Arg118-Gly119-Gln120 (RGQ), Gly179-Xaa180-Ser181-Gln182-Gly183 (GXSQG), and His298-Glu299 (HE). In addition, Figure 1 illustrates an additional highly conserved Lys267-Xaa268-Asp269 (LXD) motif that may be used to further characterize the signature motif. All of the numbering is relative to the numbering of a reference sequence (*B. subtilis* ATCC 31954™ perhydrolase; SEQ.ID NO: 2).

In one embodiment, suitable perhydrolytic enzymes can be identified by the presence of the CE-7 signature motif (Vincent *et al.*, *supra*). In a preferred embodiment, perhydrolases comprising the CE-7 signature motif are identified using a CLUSTALW alignment against the *Bacillus subtilis* ATCC 31954™ perhydrolase (SEQ ID NO: 2; i.e. the reference sequence used for relative amino acid position numbering). As per the amino acid residue numbering of SEQ ID NO: 2, the CE-7 signature motif comprises
3 conserved motifs defined as:
a) Arg118-Gly119-Gln120;
a) Gly179-Xaa180-**Ser181**-Gln182-Gly183; and
b) **His298**-Glu299.

Typically, the Xaa at amino acid residue position 180 is glycine or alanine. Amino acid residues belonging to the catalytic triad are in bold.

Further analysis of the conserved motifs within the CE-7 carbohydrate esterase family indicates the presence of an additional conserved motif (LXD at amino acid positions 267-269 of SEQ ID NO: 2) that may be to further define a perhydrolase belonging to the CE-7 carbohydrate esterase family (Figures ***1a******-c).*** In a further embodiment, the signature motif defined above includes a forth conserved motif defined as:
Leu267-Xaa268-**Asp269.**

The Xaa at amino acid residue position 268 is typically isoleucine, valine, or methionine. The forth motif includes the aspartic acid residue (bold) belonging to the catalytic triad (Ser181-Asp269-His298).

Any number of well-known global alignment algorithms (i.e. sequence analysis software) may be used to align two or more amino acid sequences (representing enzymes having perhydrolase activity) to determine the existence of the present signature motif (for example, CLUSTALW or Needleman and Wunsch (J. Mol. Biol., 48:443-453 (1970)). The aligned sequence(s) is compared to the reference sequence (SEQ ID NO: 2). In one embodiment, a CLUSTAL alignment (CLUSTALW) using a reference amino acid sequence (as used herein the CAH sequence (SEQ ID NO: 2) from the *Bacillus subtilis* ATCC 31954™) is used to identify perhydrolases belonging to the CE-7 esterase family. The relative numbering of the conserved amino acid residues is based on the residue numbering of the reference amino acid sequence to account for small insertions or deletions (5 amino acids or less) within the aligned sequence.

A comparison of the overall percent identity among perhydrolases exemplified herein indicates that enzymes having as little as 42% identity to SEQ ID NO: 2 (while retaining the signature motif) exhibit significant perhydrolase activity and are structurally classified as CE-7 carbohydrate esterases. In one embodiment, the present perhydrolases include enzymes comprising the present signature motif and at least 40%, preferably at least 42%, more preferably at least 50%, even more preferably at least 60%, yet even more preferably at least 70%, even more preferably at least 80%, yet even more preferably at least 90%, and most preferably at least 95% amino acid identity to SEQ ID NO: 2.

All of the present perhydrolases are comprised of the above signature motif as shown in Table 3.

**Table 3. Conserved motifs found within the present enzymes having perhydrolase activity.**

| Perhydrolase Sequence | RGQ motif (Residue #s) | GXSQG motif^{a} (Residue #s) | LXD motif^{b} Residue #s) | HE motif (Residue #s) |
|---|---|---|---|---|
| SEQ ID NO: 2 | 118-120 | 179-183 | 267-269 | 298-299 |
| SEQ ID NO: 6 | 118-120 | 179-183 | 267-269 | 298-299 |
| SEQ ID NO: 8 | 118-120 | 179-183 | 267-269 | 298-299 |
| SEQ ID NO: 10 | 119-121 | 180-184 | 268-270 | 299-300 |
| SEQ ID NO: 12 | 118-120 | 179-183 | 267-269 | 298-299 |
| SEQ ID NO: 14 | 119-121 | 181-185 | 269-271 | 300-301 |
| SEQ ID NO: 16 | 118-120 | 186-190 | 272-274 | 303-305 |
| SEQ ID NO: 32 | 118-120 | 179-183 | 267-269 | 298-299 |

| | | | | |
|---|---|---|---|---|
| ^{a}= Conserved motifs defined by Vincent *et al., supra* used to define the signature motif. ^{b}= an additional motif identified herein useful in further defining the signature motif defined by Vincent et al., *supra.* | | | | |

Alternatively, a contiguous signature motif (SEQ ID NO: 61) comprising the 4 conserved motifs (RGQ, GXSQG, LXD, and HE; Amino acids residues 118-299 of SEQ ID NO: 2) may also be used as a contiguous signature motif to identify CE-7 carbohydrate esterases (Figures 1a-c). As such, suitable enzymes expected to have perhydrolase activity may also be identified as having at least 40%, preferably at least 50%, more preferably at least 60%, more preferably at least 70%, more preferably at least 80%, even more preferably at least 90%, and most preferably at least 95% amino acid identity to SEQ ID NO: 61 (the 4 conserved motifs found in CE-7 carbohydrate esterases are underlined).

A comparison using the contiguous signature sequence against the present CE-7 esterases having perhydrolase activity is provided in Table 4. BLASTP using default parameters was used.

**Table 4. Percent Amino Acid Identity of Various CE-7 Carbohydrate Esterases having Perhydrolysis Activity Versus the Contiguous Signature Sequence (SEQ ID NO: 61).**

| Perhydrolase Sequence | % Identity using BLASTP | E-score (expected) |
|---|---|---|
| SEQ ID NO: 2 | 100 | 3e-92 |
| SEQ ID NO: 6 | 98 | 6e-91 |
| SEQ ID NO: 8 | 98 | 4e-98 |
| SEQ ID NO: 10 | 78 | 1e-78 |
| SEQ ID NO: 12 | 80 | 3e-76 |
| SEQ ID NO: 14 | 63 | 2e-56 |
| SEQ ID NO: 16 | 51 | 1e-41 |
| SEQ ID NO: 32 | 99 | 2e-90 |

Alternatively, the percent amino acid identity to the complete length of one or more of the present perhydrolases may also be used. Accordingly, suitable enzymes having perhydrolase activity have at least 40%, preferably at least 42%, more preferably at least 50%, more preferably at least 60%, more preferably at least 70%, even more preferably at least 80%, yet even more preferably at least 90%, and most preferably at least 95% amino acid identity to SEQ ID NO: 2. In a further embodiment, suitable perhydrolase catalysts comprise an amino acid sequence selected from the group consisting of SEQ ID NO: 8, SEQ ID NO: 10, SEQ ID NO: 12, SEQ ID NO: 14, and SEQ ID NO: 16.

Suitable perhydrolase enzymes may also include enzymes having one or more deletions, substitutions, and/or insertions to one of the present perhydrolase enzymes (e.g. SEQ ID NOs 8, 10, 12, 14, 16). As shown in Table 3, CE-7 carbohydrates esterases having perhydrolase activity share as little as 42% overall amino acid identity. Based on the data provided in the present examples, additional enzymes having perhydrolase activity belonging to the CE-7 carbohydrate esterase family may have even lower percent identity, so long as the enzyme retains the conserved signature motif. As such, the numbers of deletions, substitutions, and/or insertions may vary so long as the conserved signature motifs (see Table 2) are found in their relative positions within enzyme.

Additionally, it is well within one of skill in the art to identify suitable enzymes according to the structural similarity found within the corresponding nucleic acid sequence. Hybridization techniques can be used to identify similar gene sequences. Accordingly, suitable perhydrolase catalysts comprise an amino acid sequence encoded by a nucleic acid molecule that hybridizes under stringent conditions to a nucleic acid molecule having a nucleic acid sequence selected from the group consisting of SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, and SEQ ID NO: 25.

The present method produces industrially-useful, efficacious concentrations of peracids *in situ* under aqueous reaction conditions using the perhydrolase activity of an enzyme belonging to the CE-7 family of carbohydrate esterases. In one embodiment, the enzyme having perhydrolase activity is also classified structurally and functionally as a cephalosporin C deacetylase (CAH). In another embodiment, the enzyme having perhydrolase activity is classified structurally and functionally as an acetyl xylan esterase (AXE).

The peracids produced are quite reactive and generally decrease in concentration over time. As such, it may be desirable to keep the various reaction components separated, especially for liquid formulations. In one aspect, the hydrogen peroxide source is separate from either the substrate or the perhydrolase catalyst, preferably from both. This can be accomplished using a variety of techniques including, but not limited to the use of multicompartment chambered dispensers (U.S. patent 4,585,150) and at the time of use physically combining the perhydrolase catalyst with an inorganic peroxide and the present substrates to initiate the aqueous enzymatic perhydrolysis reaction. The perhydrolase catalyst may optionally be immobilized within the body of reaction chamber or separated (e.g. filtered, etc.) from the reaction product comprising the peracid prior to contacting the surface and/or object targeted for treatment. The perhydrolase catalyst may be in a liquid matrix or in a solid form (i.e. powdered, tablet) or embedded within a solid matrix that is subsequently mixed with the substrates to initiate the enzymatic perhydrolysis reaction. In a further aspect, the perhydrolase catalyst may be contained within a dissolvable or porous pouch that may be added to the aqueous substrate matrix to initiate enzymatic perhydrolysis. In an additional further aspect, a powder comprising the enzyme catalyst is suspended in the substrate (*e.g*., triacetin), and at time of use is mixed with a source of peroxygen in water.

### HPLC Assay Method for Determining the Concentration of Peracid and Hydrogen Peroxide.

A variety of analytical methods can be used in the present method to analyze the reactants and products including, but not limited to titration, high performance liquid chromatography (HPLC), gas chromatography (GC), mass spectroscopy (MS), capillary electrophoresis (CE), the analytical procedure described by U. Karst et al., (Anal. Chem., 69(17):3623-3627 (1997)), and the 2,2'-azino-bis (3-ethylbenzothazoline)-6-sulfonate (ABTS) assay (S. Minning, et al., Analytica Chimica Acta 378:293-298 (1999) and WO 2004/058961 A1) as described in the present examples.

### Determination of Minimum Biocidal Concentration of Peracids

The method described by J. Gabrielson, et al. (J. Microbiol. Methods 50: 63-73 (2002)) can be employed for determination of the Minimum Biocidal Concentration (MBC) of peracids, or of hydrogen peroxide and enzyme substrates. The assay method is based on XTT reduction inhibition, where XTT ((2,3-bis[2-methoxy-4-nitro-5-sulfophenyl]-5-[(phenylamino)carbonyl]-2H-tetrazolium, inner salt, monosodium salt) is a redox dye that indicates microbial respiratory activity by a change in optical density (OD) measured at 490 nm or 450 nm. However, there are a variety of other methods available for testing the activity of disinfectants and antiseptics including, but not limited to viable plate counts, direct microscopic counts, dry weight, turbidity measurements, absorbance, and bioluminescence (see, for example Brock, Semour S., Disinfection, Sterilization, and Preservation, 5th edition, Lippincott Williams & Wilkins, Philadelphia, PA, USA; 2001).

### Uses of Enzymatically-Prepared Peracid Compositions

The enzyme catalyst-generated peracid produced according to the present methods can be used in a variety of hard surface/inanimate object applications for reduction of concentrations of microbial, fungal, prion-related, and viral contamination, such as decontamination of medical instruments (e.g., endoscopes), textiles (e.g., garments, carpets), food preparation surfaces, food storage and food-packaging equipment, materials used for the packaging of food products, chicken hatcheries and grow-out facilities, animal enclosures, and spent process waters that have microbial and/or virucidal activity. The enzyme-generated peracids may be used in formulations designed to inactivate prions (e.g. certain proteases) to additionally provide biocidal activity. In a preferred aspect, the present peracid compositions are particularly useful as a disinfecting agent for non-autoclavable medical instruments and food packaging equipment. As the peracid-containing formulation may be prepared using GRAS or food-grade components (enzyme, enzyme substrate, hydrogen peroxide, and buffer), the enzyme-generated peracid may also be used for decontamination of animal carcasses, meat, fruits and vegetables, or for decontamination of prepared foods. The enzyme-generated peracid may be incorporated into a product whose final form is a powder, liquid, gel, film, solid or aerosol. The enzyme-generated peracid may be diluted to a concentration that still provides an efficacious decontamination.

The compositions comprising an efficacious concentration of peracid can be used to disinfect surfaces and/or objects contaminated (or suspected of being contaminated) with viable pathogenic microbial contaminants by contacting the surface or object with the products produced by the present processes. As used herein, "contacting" refers to placing a disinfecting composition comprising an effective concentration of peracid in contact with the surface or inanimate object suspected of contamination with a disease-causing entity for a period of time sufficient to clean and disinfect. Contacting includes spraying, treating, immersing, flushing, pouring on or in, mixing, combining, painting, coating, applying, affixing to and otherwise communicating a peracid solution or composition comprising an efficacious concentration of peracid, or a solution or composition that forms an efficacious concentration of peracid, with the surface or inanimate object suspected of being contaminated with a concentration of a microbial population. The disinfectant compositions may be combined with a cleaning composition to provide both cleaning and disinfection. Alternatively, a cleaning agent (*e.g.*, a surfactant or detergent) may be incorporated into the formulation to provide both cleaning and disinfection in a single composition.

The compositions comprising an efficacious concentration of peracid can also contain at least one additional antimicrobial agent, combinations of prion-degrading proteases, a virucide, a sporicide, or a biocide. Combinations of these agents with the peracid produced by the claimed processes can provide for increased and/or synergistic effects when used to clean and disinfect surfaces and/or objects contaminated (or suspected of being contaminated) with pathogenic microorganisms, spores, viruses, fungi, and/or prions. Suitable antimicrobial agents include carboxylic esters (e.g., *p*-hydroxy alkyl benzoates and alkyl cinnamates), sulfonic acids (e.g., dodecylbenzene sulfonic acid), iodo-compounds or active halogen compounds (e.g., elemental halogens, halogen oxides (e.g., NaOCl, HOCl, HOBr, ClO₂), iodine, interhalides (e.g., iodine monochloride, iodine dichloride, iodine trichloride, iodine tetrachloride, bromine chloride, iodine monobromide, or iodine dibromide), polyhalides, hypochlorite salts, hypochlorous acid, hypobromite salts, hypobromous acid, chloro- and bromo-hydantoins, chlorine dioxide, and sodium chlorite), organic peroxides including benzoyl peroxide, alkyl benzoyl peroxides, ozone, singlet oxygen generators, and mixtures thereof, phenolic derivatives (e.g., *o*-phenyl phenol, *o*-benzyl-*p*-chlorophenol, *tert*-amyl phenol and C₁-C₆ alkyl hydroxy benzoates), quaternary ammonium compounds (e.g., alkyldimethylbenzyl ammonium chloride, dialkyldimethyl ammonium chloride and mixtures thereof), and mixtures of such antimicrobial agents, in an amount sufficient to provide the desired degree of microbial protection. Effective amounts of antimicrobial agents include about 0.001 wt% to about 60 wt% antimicrobial agent, about 0.01 wt% to about 15 wt% antimicrobial agent, or about 0.08 wt% to about 2.5 wt% antimicrobial agent.

In one aspect, the peracids formed by the present process can be used to reduce the concentration of viable microbial contaminants (e.g. a viable microbial population) when applied on and/or at a locus. As used herein, a "locus" of the invention comprises part or all of a target surface suitable for disinfecting or bleaching. Target surfaces include all surfaces that can potentially be contaminated with microorganisms, viruses, fungi, prions or combinations thereof. Non-limiting examples include equipment surfaces found in the food or beverage industry (such as tanks, conveyors, floors, drains, coolers, freezers, equipment surfaces, walls, valves, belts, pipes, drains, joints, crevasses, combinations thereof, and the like); building surfaces (such as walls, floors and windows); non-food-industry related pipes and drains, including water treatment facilities, pools and spas, and fermentation tanks; hospital or veterinary surfaces (such as walls, floors, beds, equipment, (such as endoscopes) clothing worn in hospital/veterinary or other healthcare settings, including clothing, scrubs, shoes, and other hospital or veterinary surfaces); restaurant surfaces; bathroom surfaces; toilets; clothes and shoes; surfaces of barns or stables for livestock, such as poultry, cattle, dairy cows, goats, horses and pigs; hatcheries for poultry or for shrimp; and pharmaceutical or biopharmaceutical surfaces (*e.g.*, pharmaceutical or biopharmaceutical manufacturing equipment, pharmaceutical or biopharmaceutical ingredients, pharmaceutical or biopharmaceutical excipients). Additional hard surfaces also include food products, such as beef, poultry, pork, vegetables, fruits, seafood, combinations thereof, and the like. The locus can also include water absorbent materials such as infected linens or other textiles. The locus also includes harvested plants or plant products including seeds, corms, tubers, fruit, and vegetables, growing plants, and especially crop growing plants, including cereals, leaf vegetables and salad crops, root vegetables, legumes, berried fruits, citrus fruits and hard fruits.

Non-limiting examples of hard surface materials are metals (e.g., steel, stainless steel, chrome, titanium, iron, copper, brass, aluminum, and alloys thereof), minerals (e.g., concrete), polymers and plastics (e.g., polyolefins, such as polyethylene, polypropylene, polystyrene, poly(meth)acrylate, polyacrylonitrile, polybutadiene, poly(acrylonitrile, butadiene, styrene), poly(acrylonitrile, butadiene), acrylonitrile butadiene; polyesters such as polyethylene terephthalate; and polyamides such as nylon). Additional surfaces include brick, tile, ceramic, porcelain, wood, vinyl, linoleum, and carpet.

### Recombinant Microbial Expression

The genes and gene products of the instant sequences may be produced in heterologous host cells, particularly in the cells of microbial hosts. Preferred heterologous host cells for expression of the instant genes and nucleic acid molecules are microbial hosts that can be found within the fungal or bacterial families and which grow over a wide range of temperature, pH values, and solvent tolerances. For example, it is contemplated that any of bacteria, yeast, and filamentous fungi may suitably host the expression of the present nucleic acid molecules. The perhydrolase may be expressed intracellularly, extracellularly, or a combination of both intracellularly and extracellularly, where extracellular expression renders recovery of the desired protein from a fermentation product more facile than methods for recovery of protein produced by intracellular expression. Transcription, translation and the protein biosynthetic apparatus remain invariant relative to the cellular feedstock used to generate cellular biomass; functional genes will be expressed regardless. Examples of host strains include, but are not limited to bacterial, fungal or yeast species such as *Aspergillus, Trichoderma, Saccharomyces, Pichia, Phaffia, Candida, Hansenula, Yarrowia, Salmonella, Bacillus, Acinetobacter, Zymomonas, Agrobacterium, Erythrobacter, Chlorobium, Chromatium, Flavobacterium, Cytophaga, Rhodobacter, Rhodococcus, Streptomyces, Brevibacterium, Corynebacteria, Mycobacterium, Deinococcus, Escherichia, Erwinia, Pantoea, Pseudomonas, Sphingomonas, Methylomonas, Methylobacter, Methylococcus, Methylosinus, Methylomicrobium, Methylocystis, Alcaligenes, Synechocystis, Synechococcus, Anabaena, Thiobacillus, Methanobacterium, Klebsiella,* and Myxococcus. In one embodiment, bacterial host strains include *Escherichia, Bacillus,* and *Pseudomonas.* In a preferred embodiment, the bacterial host cell is *Escherichia coli.*

Large-scale microbial growth and functional gene expression may use a wide range of simple or complex carbohydrates, organic acids and alcohols or saturated hydrocarbons, such as methane or carbon dioxide in the case of photosynthetic or chemoautotrophic hosts, the form and amount of nitrogen, phosphorous, sulfur, oxygen, carbon or any trace micronutrient including small inorganic ions. The regulation of growth rate may be affected by the addition, or not, of specific regulatory molecules to the culture and which are not typically considered nutrient or energy sources.

Vectors or cassettes useful for the transformation of suitable host cells are well known in the art. Typically the vector or cassette contains sequences directing transcription and translation of the relevant gene, a selectable marker, and sequences allowing autonomous replication or chromosomal integration. Suitable vectors comprise a region 5' of the gene which harbors transcriptional initiation controls and a region 3' of the DNA fragment which controls transcriptional termination. It is most preferred when both control regions are derived from genes homologous to the transformed host cell and/or native to the production host, although such control regions need not be so derived.

Initiation control regions or promoters, which are useful to drive expression of the present cephalosporin C deacetylase coding region in the desired host cell are numerous and familiar to those skilled in the art. Virtually any promoter capable of driving these genes is suitable for the present invention including but not limited to *CYC1, HIS3, GAL1, GAL10, ADH1, PGK, PHO5, GAPDH, ADC1*, *TRP1, URA3, LEU2, ENO, TPI* (useful for expression in *Saccharomyces); AOX1* (useful for expression in *Pichia*); and *lac, ara, tet, trp, IP_{L}, IP_{R}, T7, tac,* and *trc* (useful for expression in *Escherichia coli*) as well as the *amy, apr, npr* promoters and various phage promoters useful for expression in *Bacillus.*

Termination control regions may also be derived from various genes native to the preferred host cell. In one embodiment, the inclusion of a termination control region is optional. In another embodiment, the chimeric gene includes a termination control region derived the preferred host cell.

### Industrial Production

A variety of culture methodologies may be applied to produce the present perhydrolase catalysts. For example, large-scale production of a specific gene product overexpressed from a recombinant microbial host may be produced by both batch and continuous culture methodologies.

A classical batch culturing method is a closed system where the composition of the media is set at the beginning of the culture and not subject to artificial alterations during the culturing process. Thus, at the beginning of the culturing process, the media is inoculated with the desired organism or organisms and growth or metabolic activity may occur without adding anything further to the system. Typically, however, a "batch" culture is batch with respect to the addition of carbon source and attempts are often made to control factors such as pH and oxygen concentration. In batch systems the metabolite and biomass compositions of the system change constantly up to the time the culture is terminated. Within batch cultures cells moderate through a static lag phase to a high growth log phase and finally to a stationary phase where growth rate is diminished or halted. If untreated, cells in the stationary phase will eventually die. Cells in log phase are often responsible for the bulk of production of end product or intermediate in some systems. Stationary or post-exponential phase production can be obtained in other systems.

A variation on the standard batch system is the fed-batch system. Fed-batch culture processes are also suitable in the present invention and comprise a typical batch system except that the substrate is added in increments as the culture progresses. Fed-batch systems are useful when catabolite repression is apt to inhibit the metabolism of the cells and where it is desirable to have limited amounts of substrate in the media. Measurement of the actual substrate concentration in fed-batch systems is difficult and is estimated on the basis of the changes of measurable factors such as pH, dissolved oxygen and the partial pressure of waste gases such as CO₂. Batch and fed-batch culturing methods are common and well known in the art and examples may be found in Thomas D. Brock in Biotechnology: A Textbook of Industrial Microbiology, Second Edition, Sinauer Associates, Inc., Sunderland, MA (1989) and Deshpande, Mukund V., *Appl. Biochem. Biotechnol.,* 36:227 (1992).

Commercial production of the desired products may also be accomplished with a continuous culture. Continuous cultures are an open system where a defined culture media is added continuously to a bioreactor and an equal amount of conditioned media is removed simultaneously for processing. Continuous cultures generally maintain the cells at a constant high liquid phase density where cells are primarily in log phase growth. Alternatively, continuous culture may be practiced with immobilized cells where carbon and nutrients are continuously added, and valuable products, byproducts or waste products are continuously removed from the cell mass. Cell immobilization may be performed using a wide range of solid supports composed of natural and/or synthetic materials.

Continuous or semi-continuous culture allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by media turbidity, is kept constant. Continuous systems strive to maintain steady state growth conditions and thus the cell loss due to media being drawn off must be balanced against the cell growth rate in the culture. Methods of modulating nutrients and growth factors for continuous culture processes as well as techniques for maximizing the rate of product formation are well known in the art of industrial microbiology and a variety of methods are detailed by Brock, *supra.*

Fermentation media in the present invention must contain suitable carbon substrates. Suitable substrates may include but are not limited to monosaccharides such as glucose and fructose, disaccharides such as lactose or sucrose, polysaccharides such as starch or cellulose or mixtures thereof and unpurified mixtures from renewable feedstocks such as cheese whey permeate, cornsteep liquor, sugar beet molasses, and barley malt. Additionally, the carbon substrate may also be one-carbon substrates such as carbon dioxide, methane or methanol (for example, when the host cell is a methylotrophic microorganism). Similarly, various species of *Candida* will metabolize alanine or oleic acid (Sulter et al., Arch. Microbiol., 153:485-489 (1990)). Hence, it is contemplated that the source of carbon utilized in the present invention may encompass a wide variety of carbon-containing substrates and will only be limited by the choice of organism.

Further, when an amount, concentration, or other value or parameter is given either as a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

### GENERAL METHODS

The following examples are provided to demonstrate preferred aspects of the invention. It should be appreciated by those of skill in the art that the techniques disclosed in the examples which follow represent techniques discovered by the inventor to function well in the practice of the invention, and thus can be considered to constitute preferred modes for its practice.

All reagents and materials were obtained from DIFCO Laboratories (Detroit, MI), GIBCO/BRL (Gaithersburg, MD), TCl America (Portland, OR), Roche Diagnostics Corporation (Indianapolis, IN) or Sigma/Aldrich Chemical Company (St. Louis, MO), unless otherwise specified.

The following abbreviations in the specification correspond to units of measure, techniques, properties, or compounds as follows: "sec" or "s" means second(s), "min" means minute(s), "h" or "hr" means hour(s), "µL" means microliters, "mL" means milliliters, "L" means liters, "mM" means millimolar, "M" means molar, "mmol" means millimole(s), "ppm" means parts per million, "wt" means weight, "wt%" means weight percent, "g" means grams, "µg" means micrograms, "g" means gravity, "HPLC" means high performance liquid chromatography, "dd H₂O" means distilled and deionized water, "dcw" means dry cell weight, "ATCC" or "ATCC®" means the American Type Culture Collection (Manassas, VA), "U" means units of perhydrolase activity, "rpm" means revolutions per minute, and "EDTA" means ethylenediaminetetraacetic acid.

### EXAMPLE 1

### Growth of Bacillus subtilis ATCC 31954™ and Preparation of Cell Extract

A culture of *Bacillus subtilis* (ATCC 31954™) was revived following suspension of the dried culture in 5 mL of nutrient broth (DIFCO; 0003-01-6) and incubation for 3 days at 30 °C. Following the third day of incubation, an aliquot of the culture was streaked onto a trypticase soy agar culture plate (Becton, Dickinson, and Company; Franklin Lakes, NJ) and incubated at 35 °C for 24 h. Several single colonies were scraped onto a 1 microliter inoculation loop (Becton Dickinson; catalog # 220215) and transferred into 50 mL of *Lactobacillus* MRS broth (Hardy Diagnostics, Santa Maria, CA; catalog # C5931). The culture was then grown at 30 °C and a 200-rpm agitation rate for 12 h. After 12 h of growth, 2 mL of the culture was transferred into an unbaffled 500-mL shake flask containing 100 mL of MRS broth for growth at 30 °C and 200-rpm agitation for 12-14 h. The cells were subsequently harvested by centrifugation at 15,000 x g for 25 min at 5 °C and the resulting cell paste stored at -80 °C.

For cell extract preparation, 0.9 g of cell paste was suspended at 25 wt % (wet cell weight) in 0.05 M potassium phosphate buffer (pH 7.0) containing dithiothreitol (1 mM) and EDTA (1 mM). The cell suspension was passed twice through a French press having a working pressure of 16,000 psi. The crude extract was then centrifuged at 20,000 x g to remove cellular debris, producing a clear cell extract that was assayed for total soluble protein (Bicinchoninic Acid Kit for Protein Determination, Sigma Aldrich, Sigma catalog # BCA1-KT), then frozen and stored at - 80 °C.

### EXAMPLE 2

### Determination of Perhydrolysis Activity of Bacillus subtilis ATCC 31954™ Semi-purified Cell Extract

A 1.0-mL aliquot of *Bacillus subtilis* (ATCC 31954™) cell extract (10 mg total protein/mL, prepared as described in Example 1) was diluted with an equal volume of 50 mM phosphate buffer (pH 7.0) and filtered through a 100,000 Molecular Weight Cutoff (MWCO) Centricon membrane unit (Millipore Corp, Bedford, MA). The resulting filtrate (semi-purified cell extract) contained 1.5 mg total protein/mL assayed for total soluble protein (Bicinchoninic Acid Kit for Protein Determination, Sigma catalog # BCA1-KT), and an assay of this filtrate indicated no measurable catalase activity.

A 1-mL reaction mixture containing triacetin (250 mM), hydrogen peroxide (2.5 M) and 0.100 mL of semi-purified cell extract (0.15 mg extract total protein) in 50 mM phosphate buffer (pH 6.5) was mixed at 25 °C. A control reaction was run by substituting 50 mM phosphate buffer (pH 6.5) for semi-purified cell extract to determine the concentration of peracetic acid produced by chemical perhydrolysis of triacetin by hydrogen peroxide in the absence of added semi-purified cell extract.

Determination of the concentration of peracetic acid in the reaction mixture was performed according to the method described by Karst *et al..* Aliquots (0.250 mL) of the reaction mixture were removed at 10 min and 30 min and filtered using an Ultrafree® MC-filter unit (30,000 Normal Molecular Weight Limit (NMWL), Millipore cat # UFC3LKT 00) by centrifugation for 2 min at 12,000 rpm; removal of the protein component of the aliquot by filtration terminated the reaction. An aliquot (0.100 mL) of the resulting filtrate was transferred to 1.5-mL screw cap HPLC vial (Agilent Technologies, Palo Alto, CA; #5182-0715) containing 0.300 mL of deionized water, then 0.100 mL of 20 mM MTS (methyl-*p*-tolyl-sulfide) in acetonitrile was added, the vials capped, and the contents briefly mixed prior to a 10 min incubation at ca. 25 °C in the absence of light. To each vial was then added 0.400 mL of acetonitrile and 0.100 mL of a solution of triphenylphosphine (TPP, 40 mM) in acetonitrile, the vials re-capped, and the resulting solution mixed and incubated at ca. 25 °C for 30 min in the absence of light. To each vial was then added 0.100 mL of 10 mM N,N-diethyl-*m*-toluamide (DEET; HPLC external standard) and the resulting solution analyzed by HPLC as described below. The peracetic acid concentrations produced in 10 min and 30 min is listed in Table 5.

### HPLC Method:

Supelco Discovery C8 column (10-cm X 4.0-mm, 5 µm) (cat. #569422-U) w/precolumn Supelco Supelguard Discovery C8 (Sigma-Aldrich; cat # 59590-U); 10 microliter injection volume; gradient method with CH₃CN (Sigma-Aldrich; #270717) and deionized H₂O at 1.0 mL/min and ambient temperature:

| Time (min:sec) | (% CH₃CN) |
|---|---|
| 0:00 | 40 |
| 3:00 | 40 |
| 3:10 | 100 |
| 4:00 | 100 |
| 4:10 | 40 |
| 7:00 (stop) | 40 |

**Table 5. Peracetic acid (PAA) produced by reaction of triacetin (250 mM) and hydrogen peroxide (2.5 M) at pH 6.5 in the presence or absence of B. subtilis (ATCC 31954™) semi-purified cell extract.**

| *B. subtilis* (ATCC 31954™) semi-purified cell extract (mg total protein/mL) | peracetic acid (ppm) in 10 min | peracetic acid (ppm) in 30 min |
|---|---|---|
| 0 | 641 | 1343 |
| 0.15 | 3492 | 3032 |

### EXAMPLE 3

### Perhydrolysis Activity of Semi-Purified Enzyme from Bacillus subtilis ATCC 31954™ Cell Extract

*Bacillus subtilis* ATCC 31954™ growth and extract preparation was performed as described in Example 1, except that the crude extract was not centrifuged. The crude extract was fractionated with cold n-propanol (- 20 °C). A flask containing the cell-free extract was stirred in an ice bath for 15 min, then the n-propanol (- 20 °C) was added drop-wise (to prevent freezing of the extract) to a concentration of 40 % (v/v). The resulting extract/propanol mixture was stirred in the ice bath for 30 min, then centrifuged at 12,000 x *g* for 10 min at 5 °C, and the supernatant returned to the flask and placed into the ice bath. Additional n-propanol (- 20 °C) was slowly added to the supernatant with stirring to a concentration of 60 % (v/v), and the resulting mixture stirred for 30 min in the ice bath and then centrifuged as before. The pellet from this second fraction was saved on ice and the supernatant returned to the flask and placed into the ice bath. Cold n-propanol was slowly added to the supernatant with stirring to a concentration of 80 % (v/v), the mixture stirred for 30 min and centrifuged as before. The pellet from the 60-80 % fraction was saved on ice. The pellets from the 40 - 60 % (v/v) n-propanol fractions and the 60 - 80 % (v/v) n-propanol fractions were dissolved in a minimum amount of 0.05 M phosphate buffer (pH 6.5) and the resulting solutions assayed for total soluble protein (Bicinchoninic Acid Kit for Protein Determination, catalog # BCA1-KT), then frozen and stored at - 80 °C.

A 1-mL reaction mixture containing triacetin (250 mM), hydrogen peroxide (1.0 M) and 0.10 mg/mL of total soluble protein from either the 40 - 60 % (v/v) or 60 - 80 % (v/v) n-propanol fractions of the cell extract (prepared as described above) in 50 mM phosphate buffer (pH 6.5) was mixed at 25 °C. A control reaction was run by substituting 50 mM phosphate buffer (pH 6.5) for the n-propanol fractions of the cell extract containing semi-purified enzyme to determine the concentration of peracetic acid produced by chemical perhydrolysis of triacetin by hydrogen peroxide in the absence of added semi-purifed enzyme. The reaction mixture was assayed for peracetic acid at 5 min and 30 min using the procedure described in Example 2, and the concentrations of peracetic acid produced by added enzyme are listed in Table 6.

**Table 6. Peracetic acid (PAA) produced by reaction of triacetin (250 mM) and hydrogen peroxide (1.0 M) at pH 6.5 in the presence or absence of B. subtilis (ATCC 31954™) semi-purified cell extracts.**

| *n*-propanol fraction of cell extract | total protein (mg/mL reaction) | peracetic acid (ppm) in 5 min | peracetic acid (ppm) in 30 min |
|---|---|---|---|
| no extract | 0 | 221 | 803 |
| 40-60% | 0.1 | 2829 | 4727 |
| 60-80% | 0.1 | 1832 | 3777 |

### EXAMPLE 4

### Identification of a Cephalosporin C Deacetylase Having Perhydrolysis Activity from Bacillus subtilis ATCC 31954™ Cell Extract

A 0.1 mL sample (500 µg total protein) of the 40-60% n-propanol fraction described in Example 3 was mixed at room temperature with an equal volume of 2x non-denaturing (native) sample buffer (Invitrogen) and loaded into the preparative sample well of a 1.5 mm 8-16% Tris-Glycine polyacrylamide mini-gel (2D gels; Invitrogen). The native gel electrophoresis was operated at 125 V for 90 min using Tris-Glycine running buffer (Invitrogen). Following electrophoresis, the gel was prepared for an *in situ* esterase activity assay using the pH indicator, bromothymol blue.

The gel was washed for 10 min x 2 with deionized water and slow mechanical mixing. The gel was then washed for 10 min using 10 mM phosphate buffer. Following the removal of the phosphate buffer, 50 mL of 10 mM phosphate buffer containing 665 µL of saturated bromothymol blue (in water) was incubated with the gel for 10 min followed by the addition of 1 mL of neat triacetin (Sigma Aldrich). Within 10 min of incubation one yellow band at 146 kDa appeared on the gel indicating esterase activity.

The esterase-positive band was excised from the gel and transferred into a 50 mL polypropylene conical tube (Falcon). The yellow bromothymol blue stain was removed from the gel slice following two 5-mL deionized water washes with gentle mixing. The gel slice was then treated for 30 min with 0.9 mL of 2X Novex Tris-Glycine SDS sample buffer plus 100 µL of 10X NuPAGE reducing agent (Invitrogen) with gentle mixing. Following the sample treatment, the gel slice and sample buffer were incubated at 85 °C for 5 min using a hot water bath. The gel slice was then removed from the incubation tube and carefully placed in the single preparative well of a 1.5 mm 8-16% Tris-Gly mini-gel. Care was taken to exclude air bubbles and to have direct contact with the stacking gel. The gel slice was then immobilized in place following the addition of 250-300 µL of a warm 0.5 % agarose solution prepared in deionized water into the preparative well. The single molecular marker lane was loaded with 15 µL of SeeBlue® Plus2 pre-stained MW marker (Invitrogen).

The electrophoresis of the gel slice was operated at 30 V for 30 min for electro-elution of the protein from the gel slice into the slab gel. The voltage was then ramped up from 30 V to 125 V over 10 min followed by 90 min operation at 125 V. Following electrophoresis, the resolved protein bands on the gel were blotted onto a PVDF membrane as described in the XCell II™ blotting manual (Invitrogen) and the blotting buffer was 10 mM CAPS, pH 11.0. The electro-blotting procedure was operated at 25 V for 2 hr at room temperature with ice water in the jacket of the transfer apparatus.

Following the transfer, the PVDF membrane was stained with ProBlot staining solution (Applied Biosystems, Foster City, CA) for 1 min followed by de-staining with methanol:water (50:50). Six protein bands were identified and each was N-terminal sequenced. Following a Blast search of the GenBank® amino acid sequence database, the only band having esterase-related sequence homology was identified as Band 1 and the 17 N-terminal amino acid calls had 100 % amino acid identity to a *Bacillus subtilis* cephalosporin C deacetylase (GENBANK® BAA01729; Mitsushima *et al.*, *supra*; U.S. patent 5,528,152; and U.S. patent 5,338,676).

### EXAMPLE 5

### Cloning and Expression of Perhydrolase from Bacillus subtilis ATCC 31954™

Genomic DNA was isolated from *Bacillus subtilis* ATCC 31954™ using the PUREGENE® DNA purification system (Gentra Systems, Minneapolis MN). The perhydrolase gene was amplified from the genomic DNA by PCR (0.5 min at 94 °C, 0.5 min at 55 °C, 1 min at 70 °C, 30 cycles) using primers identified as SEQ ID NO: 3 and SEQ ID NO: 4. The resulting nucleic acid product (SEQ ID NO: 1) was subcloned into pTrcHis2-TOPO® (Invitrogen, Carlsbad CA) to generate the plasmid identified as pSW186. The perhydrolase gene was also amplified from the genomic DNA by PCR (0.5 min at 94 °C, 0.5 min at 55 °C, 1 min at 70 °C, 30 cycles) using primers identified as SEQ ID NO: 27 and SEQ ID NO: 28. The resulting nucleic acid product (SEQ ID NO: 29) was cut with restriction enzymes *Pst*I and *Xbal* and subcloned between the *Pst*I and *Xbal* sites in pUC19 to generate the plasmid identified as pSW194. The plasmids pSW186 and pSW194 were used to transform *E. coli* TOP10 (invitrogen, Carlsbad CA), E. *coli* MG1655 (ATCC 47076™), *E. coli* UM2 (E. *coli* Genetic Stock Center #7156, Yale University, New Haven CT) and *E. coli* KLP18 (see EXAMPLE 15) to generate the strains identified as TOP10/pSW186, MG1655/pSW186, UM2/pSW186, KLP18/pSW186, TOP10/pSW194, MG1655/pSW194, UM2/pSW194 and KLP18/pSW194, respectively. TOP10/pSW186, MG1655/pSW186, UM2/pSW186, KLP18/pSW186, TOP10/pSW194, MG1655/pSW194, UM2/pSW194 and KLP18/pSW194 were gown in LB media at 37 °C with shaking up to OD₆₀₀ₙₘ = 0.4-0.5, at which time IPTG was added to a final concentration of 1 mM, and incubation continued for 2-3 h. Cells were harvested by centrifugation and SDS-PAGE was performed to confirm expression of the perhydrolase at 20-40% of total soluble protein.

### EXAMPLE 6

### Cloning and Expression of Perhydrolase from Bacillus subtilis BE1010

Genomic DNA was isolated from *Bacillus subtilis* BE1010 (Payne and Jackson 1991 J. Bacteriol. 173:2278-2282) using the PUREGENE® DNA purification system (Gentra Systems). The perhydrolase gene was amplified from the genomic DNA by PCR (0.5 min at 94 °C, 0.5 min at 55 °C, 1 min at 70 °C, 30 cycles) using primers identified as SEQ ID NO: 3 and SEQ ID NO: 4. The resulting nucleic acid product (SEQ ID NO: 5) was subcloned into pTrcHis2-TOPO® (Invitrogen) to generate the plasmid identified as pSW187. The perhydrolase gene was also amplified from the genomic DNA by PCR (0.5 min at 94 °C, 0.5 min at 55 °C, 1 min at 70 °C, 30 cycles) using primers identified as SEQ ID NO: 27 and SEQ ID NO: 28. The resulting nucleic acid product (SEQ ID NO: 30) was cut with restriction enzymes *Pst*I and *Xbal* and subcloned between the *Pst*I and *Xbal* sites in pUC19 to generate the plasmid identified as pSW189. The plasmids pSW187 and pSW189 were used to transform *E. coli* TOP10 (Invitrogen), *E. coli* MG1655 (ATCC 47076™), *E. coli* UM2 (*E. coli* Genetic Stock Center #7156, Yale University, New Haven CT) and *E. coli* KLP18 (see EXAMPLE 15) to generate the strains identified as TOP10/pSW187, MG1655/pSW187, UM2/pSW187, KLP18/pSW187, TOP10/pSW189, MG1655/pSW189, UM2/pSW189 and KLP18/pSW19, respectively. TOP10/pSW187, MG1655/pSW187, UM2/pSW187, KLP18/pSW187, TOP10/pSW189, MG1655/pSW189, UM2/pSW189 and KLP18/pSW189 were gown in LB media at 37 °C with shaking up to OD₆₀₀ₙₘ = 0.4 - 0.5, at which time IPTG was added to a final concentration of 1mM, and incubation continued for 2-3 h. Cells were harvested by centrifugation and SDS-PAGE was performed to confirm expression of the perhydrolase at 20-40% of total soluble protein.

### EXAMPLE 7

### Cloning and Expression of Perhydrolase from Bacillus subtilis ATCC 6633™

Genomic DNA was isolated from *Bacillus subtilis* ATCC 6633™ using the PUREGENE® DNA purification system. The perhydrolase gene was amplified from the genomic DNA by PCR (0.5 min at 94 °C, 0.5 min at 55 °C, 1 min at 70 °C, 30 cycles) using primers identified as SEQ ID NO: 3 and SEQ ID NO: 4. The resulting nucleic acid product (SEQ ID NO: 7) was subcloned into pTrcHis2-TOPO® to generate the plasmid-identified as pSW188. The plasmid pSW188 was used to transform *E*. *coli* MG1655 (ATCC 47076™) and E. *coli* UM2 (E. *coli* Genetic Stock Center #7156, Yale University, New Haven CT) to generate the strains identified as MG1655/pSW188 and UM2/pSW188, respectively. MG1655/pSW188 and UM2/pSW188 were gown in LB media at 37 °C with shaking up to OD₆₀₀ₙₘ = 0.4-0.5, at which time IPTG was added to a final concentration of 1mM, and incubation continued for 2 - 3 h. Cells were harvested by centrifugation and SDS-PAGE was performed to confirm expression of the perhydrolase at 20-40% of total soluble protein.

### EXAMPLE 8

### Cloning and Expression of Perhydrolase from Bacillus subtilis ATCC 29233™

Genomic DNA was isolated from *Bacillus subtilis* ATCC 29233™ using the PUREGENE® DNA purification system. The perhydrolase gene was amplified from the genomic DNA by PCR (0.5 min at 94 °C, 0.5 min at 55 °C, 1 min at 70 °C, 30 cycles) using primers identified as SEQ ID NO: 3 and SEQ ID NO: 4. The resulting nucleic acid product (SEQ ID NO: 31) was subcloned into pTrcHis2-TOPO® to generate the plasmid identified as pSW190. The plasmid pSW190 was used to transform *E. coli* MG1655 (ATCC 47076™), E. *coli* UM2 (*E. coli* Genetic Stock Center #7156, Yale University, New Haven CT) and *E. coli* KLP18 (see EXAMPLE 15) to generate the strains identified as MG1655/pSW190, UM2/pSW190 and KLP18/pSW190, respectively. MG1655/pSW190, UM2/pSW190 and KLP18/pSW190 were gown in LB media at 37 °C with shaking up to OD₆₀₀ₙₘ = 0.4 - 0.5, at which time IPTG was added to a final concentration of 1 mM, and incubation continued for 2-3 h. Cells were harvested by centrifugation and SDS-PAGE was performed to confirm expression of the perhydrolase at 20-40% of total soluble protein.

### EXAMPLE 9

### Cloning and Expression of Perhydrolase from Bacillus licheniformis ATCC 14580™

Genomic DNA was isolated from *Bacillus licheniformis* ATCC 14580™ using the PUREGENE® DNA purification system. The perhydrolase gene was amplified from the genomic DNA by PCR (0.5 min at 94 °C. 0.5 min at 55 °C, 1 min at 70 °C, 30 cycles) using primers identified as SEQ ID NO: 33 and SEQ ID NO: 34. The resulting nucleic acid product (SEQ ID NO: 9) was subcloned into pTrcHis2-TOPO® to generate the plasmid identified as pSW191. The plasmid pSW191 was used to transform *E. coli* MG1655 (ATCC 47076™), *E. coli* UM2 (*E. coli* Genetic Stock Center #7156, Yale University, New Haven CT), *E. coli* PIR1 (Invitrogen, Carlsbad CA) and *E. coli KLP18* (see EXAMPLE 15) to generate the strains identified as MG1655/pSW191, UM2/pSW191, PIR1/pSW191 and KLP18/pSW191, respectively. MG1655/pSW191, UM2/pSW191, PIR1/pSW191 and KLP18/pSW191 were gown in LB media at 37°C with shaking up to OD₆₀₀ₙₘ = 0.4-0.5, at which time IPTG was added to a final concentration of 1 mM, and incubation continued for 2-3 h. Cells were harvested by centrifugation and SDS-PAGE was performed to confirm expression of the perhydrolase at 20-40% of total soluble protein.

### EXAMPLE 10

### Cloning and Expression of Perhydrolase from Clostridia thermocellum ATCC 27405™

Genomic DNA was isolated from *Clostridia thermocellum* ATCC 27405™ using the PUREGENE® DNA purification system. The perhydrolase gene was amplified from the genomic DNA by PCR (0.5 min at 94 °C, 0.5 min at 55 °C, 1 min at 70 °C, 30 cycles) using primers identified as SEQ ID NO: 35 and SEQ ID NO: 36. The resulting nucleic acid product (SEQ ID NO: 13) was subcloned into pTrcHis2-TOPO® to generate the plasmid identified as pSW193. The plasmid pSW193 was used to transform *E. coli* MG1655 (ATCC 47076™), *E. coli* UM2 (*E. coli* Genetic Stock Center #7156, Yale University, New Haven CT) and *E. coli* KLP18 (see EXAMPLE 15) to generate the strains identified as MG1655/pSW193, UM2/pSW193 and KLP18/pSW193, respectively. MG1655/pSW193. UM2/pSW193 and KLP18/pSW193 were gown in LB media at 37 °C with shaking up to OD₆₀₀ₙₘ = 0.4-0.5, at which time IPTG was added to a final concentration of 1 mM, and incubation continued for 2-3 h. Cells were harvested by centrifugation and SDS-PAGE was performed to confirm expression of the perhydrolase at 20-40% of total soluble protein.

### EXAMPLE 11

### Cloning and Expression of Perhydrolase from Bacillus pumilus PS213

The gene encoding acetyl xylan esterase (axe1) from *B. pumilus* PS213 as reported in GENBANK® (accession # AJ249957) was synthesized using codons optimized for expression in *E. coli* (DNA 2.0, Menlo Park CA). The gene was subsequently amplified by PCR (0.5 min at 94 °C, 0.5 min at 55 °C, 1 min at 70 °C, 30 cycles) using primers identified as SEQ ID NO: 37 and SEQ ID NO: 38. The resulting nucleic acid product (SEQ ID NO: 60) was subcloned into pTrcHis2-TOPO® (Invitrogen, Carlsbad CA) to generate the plasmid identified as pSW195. The plasmid pSW195 was used to transform *E. coli* MG1655 (ATCC 47076™), *E. coli* UM2 (*E. coli* Genetic Stock Center #7156, Yale University, New Haven CT) and *E. coli* KLP18 (see EXAMPLE 15) to generate the strains identified as MG1655/pSW195, UM2/pSW195 and KLP18/pSW195, respectively. MG1655/pSW195, UM2/pSW195 and KLP18/pSW195 were gown in LB media at 37°C with shaking up to OD600nm = 0.4-0.5, at which time IPTG was added to a final concentration of 1mM, and incubation continued for 2-3 h. Cells were harvested by centrifugation and SDS-PAGE was performed to confirm expression of the perhydrolase at 20-40% of total soluble protein.

### EXAMPLE 12

### Cloning and Expression of Perhydrolase from Thermotoga neapolitana

The gene encoding acetyl xylan esterase from *Thermotoga neapolitana* as reported in GENBANK® (accession # 58632) was synthesized using codons optimized for expression in *E. coli* (DNA 2.0, Menlo Park, CA). The gene was subsequently amplified by PCR (0.5 min at 94°C, 0.5 min at 55 °C, 1 min at 70 °C, 30 cycles) using primers identified as SEQ ID NO: 39 and SEQ ID NO: 40. The resulting nucleic acid product (SEQ ID NO: 41) was subcloned into pTrcHis2-TOPO® to generate the plasmid identified as pSW196. The plasmid pSW196 was used to transform *E. coli* MG1655 (ATCC 47076™), *E. coli* UM2 (*E. coli* Genetic Stock Center #7156, Yale University, New Haven CT) and *E. coli KLP18* (see EXAMPLE 15) to generate the strains identified as MG1655/pSW196, UM2/pSW196 and KLP18/pSW196, respectively. MG1655/pSW196, UM2/pSW196 And KLP18/pSW196 were gown in LB media at 37°C with shaking up to OD600nm = 0.4-0.5, at which time IPTG was added to a final concentration of 1mM, and incubation continued for 2-3 h. Cells were harvested by centrifugation and SDS-PAGE was performed to confirm expression of the perhydrolase at 20-40% of total soluble protein.

### EXAMPLE 13

### Construction of a katG Catalase Disrupted E. coli strain

The kanamycin resistance gene (*kan*; SEQ ID NO: 42) was amplified from the plasmid pKD13. (SEQ ID NO: 43) by PCR (0.5 min at 94 °C, 0.5 min at 55 °C, 1 min at 70 °C, 30 cycles) using primers identified as SEQ ID NO: 44 and SEQ ID NO: 45 to generate the PCR product identified as SEQ ID NO: 46. The *katG* nucleic acid sequence is provided as SEQ ID NO: 47 and the corresponding amino acid sequence is SEQ ID NO: 48. *E. coli* MG1655 (ATCC 47076™) was transformed with the temperature-sensitive plasmid pKD46 (SEQ ID NO: 49), which contains the λ-Red recombinase genes (Datsenko and Wanner, 2000, PNAS USA 97:6640-6645), and selected on LB-amp plates for 24 h at 30 °C. MG1655/pKD46 was transformed with 50-500 ng of the PCR product by electroporation (BioRad Gene Pulser, 0.2 cm cuvette, 2.5 kV, 200 W, 25 uF), and selected on LB-kan plates for 24 h at 37 °C. Several colonies were streaked onto LB-kan plates and incubated overnight at 42 °C to cure the pKD46 plasmid. Colonies were checked to confirm a phenotype of kanR/ampS. Genomic DNA was isolated from several colonies using the PUREGENE® DNA purification system, and checked by PCR to confirm disruption of the *katG* gene using primers identified as SEQ ID NO: 50and SEQ ID NO: 51. Several *katG*-disrupted strains were transformed with the temperature-sensitive plasmid pCP20 (SEQ ID NO: 52), which contains the FLP recombinase, used to excise the *kan* gene, and selected on LB-amp plates for 24 h at 37 °C. Several colonies were streaked onto LB plates and incubated overnight at 42 °C to cure the pCP20 plasmid. Two colonies were checked to confirm a phenotype of kanS/ampS, and called MG1655 KatG1 and MG1655 KatG2.

### EXAMPLE 14

### Construction of a katE Catalase Disrupted E. coli strain

The kanamycin resistance gene (SEQ ID NO: 42) was amplified from the plasmid pKD13 (SEQ ID NO: 43) by PCR (0.5 min at 94 °C, 0.5 min at 55 °C, 1 min at 70 °C, 30 cycles) using primers identified as SEQ ID NO: 53 and SEQ ID NO: 54 to generate the PCR product identified as SEQ ID NO: 55. The *katE* nucleic acid sequence is provided as SEQ ID NO: 56 and the corresponding amino acid sequence is SEQ ID NO: 57. *E. coli* MG1655 (ATCC 47076™) was transformed with the temperature-sensitive plasmid pKD46 (SEQ ID NO: 49), which contains the λ-Red recombinase genes, and selected on LB-amp plates for 24 h at 30 °C. MG1655/pKD46 was transformed with 50-500 ng of the PCR product by electroporation (BioRad Gene Pulser, 0.2 cm cuvette, 2.5 kV, 200 W, 25 uF), and selected on LB-kan plates for 24 h at 37 °C. Several colonies were streaked onto LB-kan plates and incubated overnight at 42 °C to cure the pKD46 plasmid. Colonies were checked to confirm a phenotype of kanR/ampS. Genomic DNA was isolated from several colonies using the PUREGENE DNA purification system, and checked by PCR to confirm disruption of the *katE* gene using primers identified as SEQ ID NO: 58 and SEQ ID NO: 59. Several *katE*-disrupted strains were transformed with the temperature-sensitive plasmid pCP20 (SEQ ID NO: 52), which contains the FLP recombinase, used to excise the kan gene, and selected on LB-amp plates for 24 h at 37 °C. Several colonies were streaked onto LB plates and incubated overnight at 42 °C to cure the pCP20 plasmid. Two colonies were checked to confirm a phenotype of kanS/ampS, and called MG1655 KatE1 and MG1655 KatE2

### EXAMPLE 15

### Construction of a katG catalase and katE Catalase Disrupted E. coli strain (KLP18)

The kanamycin resistance gene (SEQ ID NO: 42) was amplified from the plasmid pKD13 (SEQ ID NO: 43) by PCR (0.5 min at 94 °C, 0.5 min at 55 °C, 1 min at 70 °C, 30 cycles) using primers identified as SEQ ID NO: 53 and SEQ ID NO: 54 to generate the PCR product identified as SEQ ID NO: 55. *E. coli* MG1655 KatG1 (EXAMPLE 13) was transformed with the temperature-sensitive plasmid pKD46 (SEQ ID NO: 49), which contains the λ-Red recombinase genes, and selected on LB-amp plates for 24 h at 30 °C. MG1655 KatG1/pKD46 was transformed with 50-500 ng of the PCR product by electroporation (BioRad Gene Pulser, 0.2 cm cuvette, 2.5 kV, 200 W, 25 uF), and selected on LB-kan plates for 24 h at 37 °C. Several colonies were streaked onto LB-kan plates and incubated overnight at 42 °C to cure the pKD46 plasmid. Colonies were checked to confirm a phenotype of kanR/ampS. Genomic DNA was isolated from several colonies using the PUREGENE® DNA purification system, and checked by PCR to confirm disruption of the *katE* gene using primers identified as SEQ ID NO: 58 and SEQ ID NO: 59. Several *kat*E-disrupted strains (Δ *katE*) were transformed with the temperature-sensitive plasmid pCP20 (SEQ ID NO: 52), which contains the FLP recombinase, used to excise the kan gene, and selected on LB-amp plates for 24 h at 37 °C. Several colonies were streaked onto LB plates and incubated overnight at 42 °C to cure the pCP20 plasmid. Two colonies were checked to confirm a phenotype of kanS/ampS, and called MG1655 KatG1KatE18.1 and MG1655 KatG1KatE23. MG1655 KatG1KatE18.1 is designated *E. coli* KLP18.

### EXAMPLE 16

### Estimation of Perhydrolase Molecular Mass

Cell pellets obtained from shake flask growths of *E. coli* KLP18, a catalase double knockout of *E. coli* MG1655, expressing perhydrolase genes from *Bacillus subtilis, Bacillus licheniformis* and *Clostridium thermocellum*, were suspended in 2.2 mL of 0.05 M potassium phosphate buffer (pH 7.0) containing dithiothreitol (1 mM). Each cell suspension was passed twice through a French press having a working pressure of 16,000 psi (~110.3 MPa). The crude extracts were centrifuged at 20,000 x g to remove cellular debris, producing clear crude extracts that were assayed for total soluble protein (Bicinchoninic Acid Kit [BCA] for Protein Determination, Sigma Aldrich, BCA1-KT).

Clarified crude extracts (5 µL) containing 20 µg total protein were mixed at room temperature with an equal volume of 2x non-denaturing (native) sample buffer (Invitrogen) and loaded into sample wells of a 1.5 mm x 10 well 4-12% Tris-Glycine polyacrylamide mini-gel (Invitrogen), and 7.5 µL of NATIVEMARK™ Unstained Protein Standard (Invitrogen) was loaded into two separate wells. Native gel electrophoresis was performed at 125 V for 105 min using Tris-Glycine running buffer (Invitrogen). Following electrophoresis, the gel was prepared for an *in situ* esterase activity assay using the pH indicator bromothymol blue.

The gel was washed for 10 min x 2 with deionized water and slow mechanical mixing. The gel was then washed for 10 min using 10 mM pH 7.0 phosphate buffer and slow mechanical mixing. Following the removal of the phosphate buffer, 30 mL of 10 mM pH 7.0 phosphate buffer containing 400 µL of saturated bromothymol) blue in water was incubated with the gel for 10 min followed by the addition of 1 mL of neat triacetin (Tessenderlo Fine Chemicals; Staffordshire, UK). Within 2 minutes of incubation yellow bands developed at the active perhydrolase enzyme sites. All *B. subtilis* species and *B. licheniformis* had intense bands around a molecular mass of 216 kDa. The *C. thermocellum* displayed an intense major primary band around 432 kDa and a minor secondary band around 576 kDa, indicating esterase activity. All bands were marked by punching a small hole in the gel adjacent to the bands. The gel was washed for 10 min x 2 with deionized water and slow mechanical mixing to remove the esterase activity stain. The gel was then washed for 10 min using 10 mM phosphate buffer with slow mechanical mixing to prepare for protein staining. Coomassie blue stain was added to cover the gel. After 5 minutes of slow mechanical mixing, the Coomassie blue was decanted and replaced with 40 mL de-stain (10% acetic acid, 30% methanol, 60% de-ionized water). After de-staining, the molecular masses of the active areas were estimated. The results are summarized in Table 7.

**Table 7. Estimation of Perhydrolase Molecular Mass.**

| Transformant strain | Perhydrolase source | Primary native gel activity stain, estimated molecular mass (kDa) | Secondary native gel activity stain, estimated molecular mass (kDa) | Calculated sub-unit molecular mass (kDa) |
|---|---|---|---|---|
| KLP18 | none | none | none | - |
| KLP18/pSW186 | *B. subtilis* ATCC 31954™ | 216 | none detected | 35.8 |
| KLP18/pSW189 | *B. subtilis* BE1010 | 216 | none detected | 35.9 |
| KLP18/pSW190 | *B. subtilis* ATCC 29233™ | 216 | none detected | 35.8 |
| KLP18/pSW191 | *B. licheniformis* ATCC14580™ | 216 | none detected | 35.8 |
| KLP18/pSW193 | *C. thermocellum* ATCC 27405™ | 432 | 648 | 36.0 |

### EXAMPLE 17

### Fermentation of E. coli UM2/pSW187 Expressing B. subtilis BE1010 Perhydrolase

A fermenter seed culture was prepared by charging a 2-L shake flask with 0.5 L seed medium containing LB Miller medium (DIFCO). The pH of the medium was adjusted to 6.8 and sterilized in the flask. Post-sterilization, 1 mL of ampicillin stock solution (25 mg/mL) was added. The seed medium was inoculated with a 1-mL culture of *E. coli* UM2/pSW187 in 20% glycerol, and cultivated at 36 °C and 300 rpm. The seed culture was transferred at ca. 1-2 OD₅₅₀ to a 14L fermentor (Braun) with 8 L of medium at 35 °C containing KH₂PO₄ (3.50 g/L), FeSO₄ heptahydrate (0.05 g/L), MgSO₄ heptahydrate (2.0 g/L), sodium citrate dihydrate (1.90 g/L), yeast extract (Ambrex 695, 5.0 g/L), Biospumex153K antifoam (0.25 mL/L, Cognis Corporation), NaCl (1.0 g/L), CaCl₂ dihydrate (10 g/L), and NIT trace elements solution (10 mL/L). The trace elements solution contained citric acid monohydrate (10 g/L), MnSO₄ hydrate (2 g/L), NaCl (2 g/L), FeSO₄ heptahydrate (0.5 g/L), ZnSO₄ heptahydrate (0.2 g/L), CuSO₄ pentahydrate (0.02 g/L) and NaMoO₄ dihydrate (0.02 g/L). Post sterilization addition included 60 g fed batch solution (see below) and 16.0 mL ampicillin stock solution (25 mg/mL). A fed-batch solution contained 2.4 kg of 60% w/w glucose, 0.6L of 25-g/L yeast extract and 50 g/L Bacto peptone (DIFCO). Glucose feed was initiated when the glucose concentration decreased below 0.5 g/L, starting at 0.3 g/min, and increased progressively each hour to 0.35, 0.40, 0.47, and 0.53 g/min, respectively; the rate remained constant afterwards. Glucose concentration in the medium was monitored and if the concentration exceeded 0.1 g/L the addition rate was decreased or stopped temporarily. Induction was initiated at OD₅₅₀ = 7 with the addition of 16 mL IPTG (0.5 M). The temperature was controlled at 36 °C, the aeration was fixed at 2 slpm (standard liters per minute) with agitation at 400 rpm. The pH was controlled at 6.8; NH₄OH (29% w/w) and H₂SO₄ (20% w/v) were used for pH control. The head pressure was 0.5 bar. The cells were harvested by centrifugation at 8 h post IPTG addition.

### EXAMPLE 18

### Fermentation of E. coli UM2/pSW186 Expressing B. subtilis ATCC 31954™ Perhydrolase or E. coli UM2/pSW191 Expressing B. licheniformis ATCC 14580™ Perhydrolase

The seed culture was prepared as described in Example 17 using *E. coli* UM2/pSW186 expressing *B. subtilis* ATCC 31954™ perhydrolase or *E. coli* UM2/pSW191 expressing *B. licheniformis* ATCC 14580™ perhydrolase. The fermentation medium was LB Miller (25 g/L, DIFCO). Post sterilization additions included 50 g glucose solution (50% w/w) and 16.0 mL ampicillin stock solution (25 mg/mL). Glucose (50% w/w) was used for fed batch fermentation. Glucose feed was initiated when the glucose concentration decreased below 0.5 g/L, at a constant rate of 0.3 g/min. Glucose concentration in the medium was monitored and if the concentration exceeded 0.1 g/L the addition rate was decreased or stopped temporarily. Induction was initiated at OD ₅₅₀ = 2 with addition of 16 mL IPTG (0.5 M). The temperature was controlled at 36 °C, the aeration was fixed at 2 slpm with agitation at 400 rpm. The pH was controlled at 6.8; NH₄OH (29% w/w) and H₂SO₄ (20% w/v) were used for pH control. The head pressure was 0.5 bar. The cells were harvested by centrifugation at 8 h post IPTG addition.

### EXAMPLE 19

### Fermentation of E. coli KLP18/PSW189 Expressing B. subtilis BE1010 Perhydrolase or E. coli KLP18/PSW191 Expressing B. licheniformis ATCC 14580™ Perhydrolase

A fermentor seed culture was prepared by charging a 2-L shake flask with 0.5 L seed medium containing yeast extract (Amberx 695, 5.0 g/L), K₂HPO₄ (10.0 g/L), KH₂PO₄ (7.0 g/L), sodium citrate dihydrate (1.0 g/L), (NH₄)₂SO₄ (4.0 g/L), MgSO₄ heptahydrate (1.0 g/L) and ferric ammonium citrate (0.10 g/L). The pH of the medium was adjusted to 6.8 and the medium was sterilized in the flask. Post sterilization additions included glucose (50 wt %, 10.0 mL) and 1 mL ampicillin (25 mg/mL) stock solution. The seed medium was inoculated with a 1-mL culture of *E. coli* KLP18/PSW189 or KLP18/PSW191 in 20% glycerol, and cultivated at 35 °C and 300 rpm. The seed culture was transferred at ca. 1-2 OD₅₅₀ to a 14L fermentor (Braun) with 8 L of medium at 35 °C containing KH₂PO₄ (3.50 g/L), FeSO₄ heptahydrate (0.05 g/L), MgSO₄ heptahydrate (2.0 g/L), sodium citrate dihydrate (1.90 g/L), yeast extract (Ambrex 695, 5.0 g/L), Biospumex153K antifoam (0.25 mL/L, Cognis Corporation), NaCl (1.0 g/L), CaCl₂ dihydrate (10 g/L), and NIT trace elements solution (10 mL/L). The trace elements solution contained citric acid monohydrate (10 g/L), MnSO₄ hydrate (2 g/L), NaCl (2 g/L), FeSO₄ heptahydrate (0.5 g/L), ZnSO₄ heptahydrate (0.2 g/L), CuSO₄ pentahydrate (0.02 g/L) and NaMoO₄ dihydrate (0.02 g/L). Post sterilization additions included glucose solution (50% w/w, 80.0 g) and ampicillin (25 mg/mL) stock solution (16.00 mL). Glucose solution (50% w/w) was used for fed batch. Glucose feed was initiated when glucose concentration decreased to 0.5 g/L, starting at 0.31 g feed/min and increasing progressively each hour to 0.36, 0.42, 0.49, 0.57, 0.66, 0.77, 0.90, 1.04, 1.21, 1.41 1.63 g/min respectively; the rate remained constant afterwards. Glucose concentration in the medium was monitored and if the concentration exceeded 0.1 g/L the feed rate was decreased or stopped temporarily. For *E*. *coli* KLP18/PSW191, the induction was initiated at OD ₅₅₀ = 80 with addition of 16 mL IPTG (0.5 M), for *E. coli* KLP18/PSW189 the growth was slower and induction was initiated at OD ₅₅₀ = 56. The dissolved oxygen (DO) concentration was controlled at 25% of air saturation. The DO was controlled first by impeller agitation rate (400 to 1400 rpm) and later by aeration rate (2 to 10 slpm). The pH was controlled at 6.8. NH₄OH (29% w/w) and H₂SO₄ (20% w/v) were used for pH control. The head pressure was 0.5 bars. The cells were harvested by centrifugation 16 h post IPTG addition.

### EXAMPLE 20

### E. coli KLP18 versus E. coli UM2 as Fermentation Host for Perhydrolase Production

*E. coli* KLP18 (EXAMPLE 15) was used to produce transformants (EXAMPLES 5, 8, 9 and 10) that were grown in multiple 10-L fermentations following the method described in EXAMPLE 19. The final OD for these runs is compared to fermentations that produced *E. coli* UM2 transformants (EXAMPLES 5, 8, 9 and 10) expressing these same perhydrolases that were run following the fermentation methods described in EXAMPLES 17 and 18. Table 8 summarizes 10-L fermentation runs with both UM2 and KLP18 as host, and demonstrates the superior performance of KLP18 compared to UM2.

**Table 8.**

| run ID | host | plasmid | perhydrolase | run time, (h) | final OD₅₀₀ |
|---|---|---|---|---|---|
| PAA25 | UM2 | pSW186 | SEQ ID NO: 2 | 21.6 | 11.9 |
| PAA26 | UM2 | pSW186 | SEQ ID NO: 2 | 7.4 | 11.9 |
| PAA42 | UM2 | pSW186 | SEQ ID NO: 2 | 12.4 | 5.5 |
| PAA43 | UM2 | pSW186 | SEQ ID NO: 2 | 12.4 | 5.5 |
| PAA48 | KLP18 | pSW186 | SEQ ID NO: 2 | 33.1 | 181.0 |
| PAA30 | UM2 | pSW190 | SEQ ID NO: 32 | 12.1 | 6.2 |
| PAA31 | UM2 | pSW190 | SEQ ID NO: 32 | 12.3 | 8.8 |
| PAA40 | UM2 | pSW190 | SEQ ID NO: 32 | 12.7 | 4.6 |
| PAA41 | UM2 | pSW190 | SEQ ID NO: 32 | 12.6 | 5.3 |
| PAA49 | KLP18 | pSW190 | SEQ ID NO: 32 | 33.6 | 128.0 |
| PAA39 | UM2 | pSW191 | SEQ ID NO: 10 | 10.6 | 6.5 |
| PAA46 | KLP18 | pSW191 | SEQ ID NO: 10 | 33.6 | 140.0 |
| PAA50 | KLP18 | pSW191 | SEQ ID NO: 10 | 36.2 | 155.0 |
| PAA45 | UM2 | pSW193 | SEQ ID NO: 14 | 12.4 | 5.7 |
| PAA51 | KLP18 | pSW193 | SEQ ID NO: 14 | 35.7 | 147.0 |

### EXAMPLE 21

### Evaluation of Bacillus subtilis ATCC 31954™ Perhydrolase Expressed in E. coli Transformants

The three transformants *E*. *coli* TOP10/pSW186, *E. coli* MG1655/pSW186 and *E. coli* UM2/pSW186 described in Example 5 were grown in unbaffled shake flasks containing Miller's LB broth (50 mL; Mediatech, Inc, Hemdon, VA) with ampicillin (100 µg/mL) for 14-16 h at 35-37 °C with 200 rpm agitation. Following the overnight growth of the three transformants, each culture was sub-cultured by preparing a 1:100 dilution of each culture into fresh Miller's LB broth containing ampicillin (100 µg/mL). Following a 3 h growth at 35-37 °C with 200 rpm agitation, each culture was induced by the addition of IPTG to a final concentration of 1 mM. After an additional 3 h growth under the same conditions, the cell paste from each culture was harvested by centrifugation at 26,000 x g for 20 min at 5 °C. Cell extracts of each of the transformants were prepared according to the procedure described in Example 1, except that the extraction buffer used to prepare the 25 wt% wet cell suspension was composed of 0.05 M potassium phosphate (pH 7.0) and 1 mM dithiothreitol.

Separate 1-mL reactions containing triacetin (250 mM), hydrogen peroxide (1.0 M) and 50 µg of extract total protein from one of the three cell extracts (prepared as described above) in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. A second set of control reactions was run using 50 µg of extract total protein prepared from extracts of untransformed *E. coli* TOP10, *E. coli* MG1655 and *E. coli* UM2 to determine the background level of peracid produced by each strain in the absence of expressed perhydrolase. The concentration of peracetic acid in the reaction mixtures was determined according to the method of Karst *et al.* described in Example 2 (Table 9).

**Table 9: Peracetic acid (PAA) produced by reaction of triacetin (250 mM) and hydrogen peroxide (1.0 M) at pH 6.5 in the presence of cell extracts of E. coli TOP10/pSW186, E. coli MG1655/pSW186 and E. coli UM2/pSW186.**

| total protein extract source | total protein (□g/mL reaction) | peracetic acid (ppm) in 5 min | peracetic acid (ppm) in 30 min |
|---|---|---|---|
| no extract | 0 | 188 | 598 |
| TOP10 | 50 | 181 | 654 |
| TOP10/pSW186 | 50 | 2684 | 5363 |
| MG1655 | 50 | 173 | 638 |
| MG1655/pSW186 | 50 | 1354 | 4333 |
| UM2 | 50 | 175 | 655 |
| UM2/pSW186 | 50 | 3002 | 6529 |

### EXAMPLE 22

### Perhydrolytic Activity of E. coli TOP10/pSW186 Extract Expressing Bacillus subtilis ATCC 31954™ Perhydrolase

Separate 1.0 mL triacetin perhydrolysis reactions were run as described in Example 21 using the *E. coli* TOP10/pSW186 transformant extract to provide one of the following total protein concentrations in the reaction: 196 µg/mL, 98 µg/mL, 49 µg/mL, 25 µg/mL, 12.5 µg/mL, 6.25 µg/mL, 3.0 µg/mL, or 1.5 µg/mL total protein concentration in each reaction (Table 10).

**Table 10: Dependence of peracetic acid (PAA) concentration on total protein concentration derived from E. coli TOP10/pSW186 transformant extract in reactions containing triacetin (250 mM) and hydrogen peroxide (1.0 M) at pH 6.5.**

| total protein extract source | total protein (□g/mL reaction) | peracetic acid (ppm) in 5 min | peracetic acid (ppm) in 30 min |
|---|---|---|---|
| no extract | 0 | 193 | 854 |
| TOP10 | 50 | 181 | 654 |
| TOP10/pSW186 | 1.5 | 580 | 1710 |
| TOP10/pSW186 | 3.0 | 824 | 2233 |
| TOP10/pSW186 | 6.3 | 1371 | 3029 |
| TOP10/pSW186 | 12.5 | 2052 | 4587 |
| TOP10/pSW186 | 25 | 2849 | 4957 |
| TOP10/pSW186 | 49 | 4294 | |
| TOP10/pSW186 | 98 | 4244 | |
| TOP10/pSW186 | 196 | 4294 | |

### EXAMPLE 23

### Perhydrolytic Activity of E. coli UM2/pSW186 Extract Expressing Bacillus subtilis ATCC 31954™ Perhydrolase

An extract of *E. coli* UM2/pSW186 transformant (20 mg total protein/mL extract, prepared as described in Example 21) was employed in 1.0 mL perhydrolysis reactions (run as described in Example 21) containing triacetin (40 mM or 100 mM), hydrogen peroxide (40 mM or 100 mM) and extract total protein (0.1 mg/mL or 1.0 mg/mL) in phosphate buffer (Pi, 100 mM, 200 mM or 300 mM) at pH 6.5 or 7.5 at 25 °C each reaction (Table 11).

**Table 11: Dependence of peracetic acid (PAA) concentration on triacetin and hydrogen peroxide concentrations using perhydrolase derived from E. coli UM2/pSW186 transformant extract at pH 6.5 or 7.5.**

| total protein (mg/mL) | H₂O₂ (mM) | triacetin (mM) | Pi (mM) | pH | PAA (ppm) in 5 min | PAA (ppm) in 30 min |
|---|---|---|---|---|---|---|
| 0 | 40 | 40 | 100 | 6.5 | 0 | 0 |
| 0 | 40 | 100 | 100 | 6.5 | 0 | 0 |
| 0.1 | 40 | 40 | 100 | 6.5 | 49 | 0 |
| 1 | 40 | 40 | 100 | 6.5 | 239 | 160 |
| 1 | 40 | 100 | 100 | 6.5 | 439 | 560 |
| 0 | 40 | 100 | 200 | 6.5 | 0 | 0 |
| 0 | 100 | 100 | 200 | 6.5 | 1 | 30 |
| 0 | 100 | 100 | 200 | 7.5 | 14 | 1 |
| 0 | 100 | 100 | 300 | 7.5 | 5 | 4 |
| 1 | 100 | 40 | 200 | 6.5 | 75 | 9 |
| 1 | 100 | 100 | 200 | 6.5 | 1150 | 925 |
| 1 | 40 | 100 | 200 | 7.5 | 290 | 80 |
| 1 | 100 | 100 | 300 | 7.5 | 332 | 58 |

### EXAMPLE 24

### Evaluation of Perhydrolase Expressed in E. coli transformants derived from Bacillus subtilis BE1010

The *E. coli* TOP10/pSW187, *E. coli* MG1655/pSW187 and *E. coli* UM2/pSW187 transformants described in Example 6 were grown in unbaffled shake flasks containing Miller's LB broth (50 mL; Mediatech, Inc, Herndon, VA) with ampicillin (100 µg/mL) for 14-16 h at 35-37 °C with 200 rpm agitation. Following the overnight growth of the three transformants, each culture was sub-cultured by preparing a 1:100 dilution of each culture into fresh Miller's LB broth containing ampicillin (100 µg/mL). Following a 3 hour growth at 35-37 °C with 200 rpm agitation, each culture was induced by the addition of IPTG to a final concentration of 1 mM. After an additional 3 hours growth under the same conditions, the cell paste from each culture was harvested by centrifugation at 26,000 x g for 20 min at 5°C. For cell extract preparation, the procedure described in Example 1 was repeated except that the extraction buffer used to prepare the 25 wt % wet cell suspension was composed of 0.05 M potassium phosphate (pH 7.0) and 1 mM dithiothreitol.

Separate 1.0 mL reactions containing triacetin (250 mM), hydrogen peroxide (1.0 M) and 50 µg of extract total protein in 50 mM phosphate buffer (pH 6.5) were run at 25 °C with each transformant extract. A control reaction was run substituting 50 mM phosphate buffer (pH 6.5) for the extract total protein solution to determine the concentration of peracetic acid produced by chemical perhydrolysis of triacetin with hydrogen peroxide. A second set of control reactions was run using 50 µg of extract total protein prepared from extracts of untransformed *E. coli* TOP10, *E*. *coli* MG1655 and *E. coli* UM2 to determine the background level of peracid produced by each strain in the absence of expressed perhydrolase. The concentration of peracetic acid in the reaction mixtures (Table 12) was determined according to the method of Karst *et al.* as described in Example 2.

**Table 12: Peracetic acid (PAA) produced by reaction of triacetin (250 mM) and hydrogen peroxide (1.0 M) at pH 6.5 in the presence of cell extracts of E. coli TOP10/pSW187, E. coli MG1655/pSW187 and E. coli UM2/pSW187.**

| total protein extract source | total protein (□g/mL reaction) | peracetic acid (ppm) in 5 min | peracetic acid (ppm) in 30 min |
|---|---|---|---|
| no extract | 0 | 159 | 626 |
| TOP10 | 50 | 181 | 654 |
| TOP10/pSW187 | 50 | 3192 | 6663 |
| MG1655 | 50 | 173 | 638 |
| MG1655/pSW187 | 50 | 3472 | 7349 |
| UM2 | 50 | 175 | 655 |
| UM2/pSW187 | 50 | 3741 | 7626 |

### EXAMPLE 25

### Evaluation of Perhydrolases Expressed in E. coli Transformants

The transformants were prepared as described in Examples 5, 6, 7, 8, 9, 10, 18 and 19. Cell extracts of each of the transformants were prepared according to the procedure described in Example 1, except that the extraction buffer used to prepare the 25 wt% wet cell suspension was composed of 0.05 M potassium phosphate (pH 7.0) and 1 mM dithiothreitol.

Separate 1-mL reactions containing triacetin (250 mM), hydrogen peroxide (1.0 M) and 50 µg of extract total protein from a cell extract (prepared as described above) in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. A second set of control reactions was run using 50 µg of extract total protein prepared from extracts of untransformed *E. coli* TOP10, *E. coli* MG1655, *E. coli* UM2 and *E. coli* KLP18 to determine the background level of peracid produced by each strain in the absence of expressed perhydrolase. The concentration of peracetic acid in the reaction mixtures (Table 13) was determined according to the method of Karst *et al.* described in Example 2.

**Table 13: Peracetic acid (PAA) produced by reaction of triacetin (250 mM) and hydrogen peroxide (1.0 M) at pH 6.5 in the presence of 50 µg of total extract protein/mL from transformant cell extracts of E. coli TOP10, E. coli MG1655, E. coli UM2, E. coli PIR2, and E. coli KLP18.**

| transformant cell extract | perhydrolase source | PAA (ppm) 5 min | PAA (ppm) 5 min, no extract | PAA (ppm) 30 min | PAA (ppm) 30 min, no extract |
|---|---|---|---|---|---|
| TOP10 | none (control) | 181 | 188 | 654 | 598 |
| MG1655 | none (control) | 173 | 188 | 638 | 598 |
| UM2 | none (control) | 175 | 188 | 655 | 598 |
| PIR2 | none (control) | 144 | 276 | 515 | 677 |
| KLP18 | none (control) | 200 | 100 | 555 | 330 |
| TOP10/pSW186 | *B. subtilis* ATCC 31954™ | 2684 | 188 | 5363 | 598 |
| MG1655/pSW186 | *B. subtilis* ATCC 31954™ | 1354 | 188 | 4333 | 598 |
| UM2/pSW186 | *B. subtilis* ATCC 31954™ | 3002 | 188 | 6529 | 598 |
| KLP18/pSW186 | *B. subtilis* ATCC 31954™ | 1033 | 268 | 2641 | 792 |
| TOP10/pSW187 | *B. subtilis* BE1010 | 3192 | 159 | 6663 | 626 |
| MG1655/pSW187 | *B. subtilis* | 3472 | 159 | 7349 | 626 |
| | BE1010 | | | | |
| UM2/pSW187 | *B. subtilis* BE1010 | 3741 | 159 | 7626 | 626 |
| KLP18/pSW189 | *B. subtilis* BE1010 | 2631 | 146 | 6579 | 625 |
| UM2/pSW188 | *B. subtilis* ATCC 6633™ | 4617 | 289 | 8742 | 306 |
| UM2/pSW190 | *B. subtilis* ATCC 29233™ | 5314 | 320 | 8845 | 738 |
| UM2/pSW190a | *B. subtilis* ATCC 29233™ | 2622 | 234 | 3553 | 642 |
| KLP18/pSW190 | *B. subtilis* ATCC 29233™ | 1006 | 146 | 3285 | 625 |
| PIR2/pSW191 | *B. licheniformis* ATCC 14580™ | 3125 | 276 | 6338 | 677 |
| UM2/pSW191 | *B. licheniformis* ATCC 14580™ | 1632 | 276 | 4640 | 677 |
| KLP18/pSW191 | *B. licheniformis* ATCC 14580™ | 3936 | 146 | 8016 | 625 |
| MG1655/pSW193 | *C. thermocellum* ATCC 27405™ | 2279 | 349 | 3178 | 645 |
| UM2/pSW193 | *C. thermocellum* ATCC 27405™ | 2738 | 349 | 3597 | 645 |
| KLP18/pSW193 | *C. thermocellum* ATCC 27405™ | 1687 | 146 | 2407 | 625 |
| UM2/pSW195 | *B. pumilus PS213* | 2226 | 360 | 6354 | 776 |
| KLP18/pSW195 | *B. pumilus PS213* | 5023 | 100 | 9642 | 394 |
| UM2/pSW196 | *T. neapolitana* | 1347 | 360 | 2553 | 776 |
| KLP18/pSW196 | *T. neapolitana* | 878 | 100 | 2023 | 394 |

### EXAMPLE 26 (COMPARATIVE)

### Evaluation of Commercial Lipases for Perhydrolysis

Separate 1-mL reactions containing triacetin (250 mM), hydrogen peroxide (1.0 M) and 50 µg of commercial lipases in 50 mM phosphate buffer (pH 6.5) were run at 25 °C. Control reactions were run without commercial lipase to determine the concentration of peracetic acid produced by chemical perhydrolysis of triacetin by hydrogen peroxide in the absence of added lipase. The concentration of peracetic acid in the reaction mixtures (Table 14) was determined according to the method of Karst *et al.* described in Example 2. The commercial lipases were obtained from Sigma/Aldrich Chemical Company (St. Louis, MO), BioCatalytics (Pasadena, CA), Meito Sangyo Co. (Nagoya, Japan), Amano Enzymes (Lombard, IL), Novozymes (Franklinton, NC), Valley Research (South Bend, IN), and Enzyme Development Corporation (ENZECO®; New York, NY).

**Table 14: Peracetic acid (PAA) produced by reaction of triacetin (250 mM) and hydrogen peroxide (1.0 M) at pH 6.5 in the presence of 50 µg/mL of commercial lipases.**

| commercial lipase | lipase source | PAA (ppm); 5 min | PAA (ppm); 30 min |
|---|---|---|---|
| no enzyme | control | 105 | 105 |
| Meito MY | *Candida rugosa* | 155 | 280 |
| Meito OF | *Candida rugosa* | 120 | 340 |
| Meito AL | *Achromobacter sp.* | 165 | 315 |
| Meito PL | *Alcaligines sp.* | 165 | 430 |
| Meito SL | *Pseudomonas cepacia* | 210 | 440 |
| Meito TL | *Pseudomonas stutzeri* | 225 | 500 |
| Meito QLC | *Alcaligines sp.* | 195 | 240 |
| Meito QLM | *Alcaligines sp.* | 225 | 555 |
| | | | |
| no enzyme | control | 150 | 205 |
| Amano F-DS | *Rhizopus oryzae* | 180 | 265 |
| Amano R | *Penicillium roqueforti* | 170 | 160 |
| Amano M 10 | *Mucor javanicus* | 255 | 425 |
| Amano G 50 | *Penicillium cambertii* | 40 | 40 |
| Amano F-AP15 | *Rhizopus oryzae* | 120 | 50 |
| Amano AY 30 | *Candida rugosa* | 140 | 300 |
| Amano PS | *Burkholder cepacia* | 150 | 150 |
| Amano DS | *Aspergillus niger* | 140 | 125 |
| Amano AY | *Candida rugosa* | 180 | 390 |
| Amano AK-20 | *Pseudomonas fluorescens* | 215 | 500 |
| Amano LPS | *Burkholder cepacia* | 315 | 350 |
| Amano A 12 | *Aspergillus niger* | 245 | 490 |
| no enzyme | control | 30 | 55 |
| BioCatalytics ICR 110 | *Candida antartica B* | 145 | 245 |
| Novozymes Lipolase 100 L type EX | *Thermomyces lanuginosus* | 10 | 0 |
| Novozymes Lipozyme TL 100 L | *Thermomyces lanuginosus* | 125 | 370 |
| Novozymes Lipozyme CALB L | *Candida antartica* | 0 | 180 |
| Novozymes Palatase 20000L | *Aspergillus oryzae* | 95 | 220 |
| Valley Research CR | *Candida rugosa* | 70 | 320 |
| Valley Research MJ | *Mucor javanicus* | 140 | 440 |
| Valley Research AN | *Aspergillus niger* | 165 | 240 |
| Enzeco LC | *Candida rugosa* | 105 | 120 |
| Enzeco MLC | *Aspergillus niger* | 140 | 370 |
| Enzeco R0 20 | *Rhizopus oryzae* | 55 | 100 |

### EXAMPLE 27

### Evaluation of Perhydrolases Expressed in E. coli transformants

Cell extracts of transformants expressing perhydrolase were prepared according to the procedure described in Example 21. Separate 1-mL reactions containing triacetin (105 mM), hydrogen peroxide (78 mM) and 1 mg or 2 mg of extract total protein from a cell extract (prepared as described above) in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. The concentration of peracetic acid in the reaction mixtures (Table 15) was determined according to the method of Karst *et al.* described in Example 2.

**Table 15: Peracetic acid (PAA) produced by reaction of triacetin (105 mM) and hydrogen peroxide (78 mM) at pH 6.5 or 7.5 in the presence of 1 mg or 2 mg of total extract protein/mL from transformant cell extracts of E. coli MG1655, E. coli UM2, E. coli PIR2 and E. coli KLP18.**

| transformant cell extract | source of perhydrolase | total protein (mg/mL) | pH | PAA (ppm); 5 min | PAA (ppm); 30 min |
|---|---|---|---|---|---|
| no extract | control | 0 | 6.5 | 0 | 0 |
| no extract | control | 0 | 7.5 | 8 | 12 |
| UM2/pSW186 | *B. subtilis* ATCC 31954™ | 1.0 | 6.5 | 945 | 1420 |
| UM2/pSW186 | *B. subtilis* ATCC 31954™ | 2.0 | 6.5 | 1000 | 1250 |
| UM2/pSW186 | *B. subtilis* ATCC 31954™ | 1.0 | 7.5 | 1001 | 1215 |
| UM2/pSW186 | *B. subtilis* ATCC 31954™ | 2.0 | 7.5 | 1036 | 1050 |
| no extract | control | 0 | 6.5 | 0 | 0 |
| no extract | control | 0 | 7.5 | 45 | 0 |
| MG1655/pSW187 | *B. subtilis* BE1010 | 1.0 | 6.5 | 690 | 265 |
| UM2/pSW187 | *B. subtilis* BE1010 | 1.0 | 6.5 | 730 | 755 |
| UM2/pSW187 | *B. subtilis* BE1010 | 2.0 | 6.5 | 1400 | 1990 |
| UM2/pSW187 | *B. subtilis* BE1010 | 2.0 | 7.5 | 1710 | 2105 |
| KLP18/pSW189 | *B. subtilis* BE1010 | 1.0 | 6.5 | 885 | 1288 |
| KLP18/pSW189 | *B. subtilis* BE1010 | 2.0 | 6.5 | 950 | 1263 |
| no extract | control | 0 | 6.5 | 0 | 0 |
| UM2/pSW190 | *B. subtilis* ATCC 29233™ | 1.0 | 6.5 | 940 | 685 |
| no extract | control | 0 | 6.5 | 0 | 0 |
| PIR2/pSW191 | *B. lichen.* ATCC 14580™ | 1.0 | 6.5 | 860 | 1305 |
| UM2/pSW191 | *B. lichen.* ATCC 14580™ | 1.0 | 6.5 | 675 | 1530 |
| no extract | control | 0 | 6.5 | 0 | 0 |
| UM2/pSW195 | *B. pumilus* PS213 | 1.0 | 6.5 | 400 | 850 |
| UM2/pSW195 | *B. pumilus* PS213 | 2.0 | 6.5 | 460 | 790 |
| no extract | control | 0 | 6.5 | 0 | 0 |
| UM2/pSW196 | *T. neapolitana* | 1.0 | 6.5 | 1100 | 1685 |
| UM2/pSW196 | *T. neapolitana* | 2.0 | 6.5 | 1190 | 1900 |

### EXAMPLE 28 (COMPARATIVE)

### Evaluation of Commercial Lipases for Perhydrolysis

Separate 1-mL reactions containing triacetin (105 mM), hydrogen peroxide (78 mM) and 1 mg of commercial lipases in 50 mM phosphate buffer (pH 6.5) were run at 25 °C. A control reaction was run without commercial lipase to determine the concentration of peracetic acid produced by chemical perhydrolysis of triacetin by hydrogen peroxide in the absence of added lipase. The concentration of peracetic acid in the reaction mixtures (Table 16) was determined according to the method of Karst *et al.* described in Example 2.

**Table 16: Peracetic acid (PAA) produced by reaction of triacetin (105 mM) and hydrogen peroxide (78 mM) at pH 6.5 in the presence of 1 mg/mL of commercial lipases.**

| commercial lipase | lipase source | PAA (ppm); 5 min | PAA (ppm); 30 min, |
|---|---|---|---|
| no enzyme | control | 15 | 20 |
| Meito MY | *Candida rugosa* | 25 | 45 |
| Meito SL | *Pseudomonas cepacia* | 0 | 0 |
| Meito QLM | *Alcaligines sp.* | 35 | 85 |
| Amano F-DS | *Rhizopus oryzae* | 20 | 50 |
| Amano M 10 | *Mucor javanicus* | 20 | 40 |
| Amano A 12 | *Aspergillus niger* | 70 | 140 |
| BioCatalytics ICR 110 | *Candida antartica B* | 55 | 110 |

### EXAMPLE 29

### B. subtilis ATCC31954™ Perhydrolase Activity with Wetting Agents

A cell extract of E. coli UM2/pSW186 transformant expressing *B. subtilis* ATCC 31954™ perhydrolase was prepared according to the procedure described in Example 21. Separate 1-mL reactions containing triacetin (105 mM), hydrogen peroxide (78 mM), wetting agent COLATERIC® MSC-NA (mixed short chain sodium dipropionate; Colonial Chemical Co.), SURFYNOL® 2502 (an ethoxylated/propoxylated acetylenic-based surfactant; Air Products and Chemicals; Utrecht, NL), SURFYNOL® MD-20, SILWET® L7650 (a polyalkyleneoxide modified polydimethylsiloxane; Chemtura Corp, Middlebury, CT) or SILWET® L8620; a siloxane-based surfactant), and 1 mg of extract total protein in 50 mM phosphate buffer (pH 7.5) were run at 25°C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. The concentration of peracetic acid in the reaction mixtures (Table 17) was determined according to the method of Karst *et al.* described in Example 2.

**Table 17: Peracetic acid (PAA) produced by reaction of triacetin (105 mM) and hydrogen peroxide (78 mM) at pH 7.5 in the presence of 1 mg of total extract protein/mL from transformant cell extracts of E. coli UM2/pSW186 expressing B. subtilis ATCC 31954™ perhydrolase.**

| wetting agent | wetting agent conc. (ppm) | total protein (mg/mL) | PAA (ppm); 5 min | PAA (ppm); 30 min |
|---|---|---|---|---|
| none | 0 | 0 | 130 | 170 |
| COLATERIC MSC-NA | 1000 | 0 | 80 | 70 |
| COLATERIC MSC-NA | 1000 | 1.0 | 745 | 1520 |
| SURFYNOL® 2502 | 1000 | 0 | 35 | 10 |
| SURFYNOL® 2502 | 1000 | 1.0. | 650 | 1210 |
| SURFYNOL® MD-20 | 1000 | 0 | 110 | 150 |
| SURFYNOL® MD-20 | 1000 | 1.0 | 555 | 1110 |
| SILWET® L7650 | 1000 | 0 | 50 | 0 |
| SILWET® L7650 | 1000 | 1.0 | 830 | 1360 |
| SILWET® L8620 | 1000 | 0 | 60 | 135 |
| SILWET® L8620 | 1000 | 1.0 | 735 | 1145 |

### EXAMPLE 30

### B. subtilis BE1010 Perhydrolase Activity with Wetting Agents

A cell extract of *E*. *coli* UM2/pSW187 transformant expressing *B. subtilis* ATCC 31954™ perhydrolase was prepared according to the procedure described in Example 21. Separate 1-mL reactions containing triacetin (105 mM), hydrogen peroxide (78 mM), wetting agent (PLURONIC® 17R4 (a polyoxyalkylene ether surfactant; BASF, Mount Olive, NJ), PLURONIC® L43 (a difunctional block copolymer surfactant), or SILWET® L7650), and 1 mg of extract total protein in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. The concentration of peracetic acid in the reaction mixtures (Table 18) was determined according to the method of Karst *et al.* described in Example 2.

**Table 18: Peracetic acid (PAA) produced by reaction of triacetin (105 mM) and hydrogen peroxide (78 mM) at pH 6.5 in the presence of 1 mg of total extract protein/mL from transformant cell extracts of E. coli UM2/pSW187 expressing B. subtilis BE1010 perhydrolase.**

| wetting agent | wetting agent conc. (ppm) | total protein (mg/mL) | PAA (ppm); 5 min | PAA (ppm); 30 min |
|---|---|---|---|---|
| none | 0 | 0 | 0 | 0 |
| none | 0 | 1.0 | 975 | 1345 |
| PLURONIC® 17R4 | 2500 | 0 | 0 | 0 |
| PLURONIC®17R4 | 2500 | 1.0 | 860 | 1360 |
| PLURONIC® L43 | 2500 | 0 | 0 | 0 |
| PLURONIC® L43 | 2500 | 1.0 | 855 | 1360 |
| SILWET® L7650 | 2500 | 0 | 0 | 0 |
| SILWET® L7650 | 2500 | 1.0 | 975 | 1205 |

### EXAMPLE 31

### Perhydrolase Activity with Wetting and Chelating Agents

A cell extract of *E. coli* UM2/pSW187 transformant expressing *B. subtilis* BE1010 perhydrolase was prepared according to the procedure described in Example 21. Separate 1-mL reactions containing triacetin (105 mM), hydrogen peroxide (78 mM), wetting agent (SILWET® L7650), chelating agent (TURPINAL® SL; etidronic acid; Solutia Inc., St. Louis, MO), and 1 mg of extract total protein in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. The concentration of peracetic acid in the reaction mixtures (Table 19) was determined according to the method of Karst *et al.* described in Example 2.

**Table 19: Peracetic acid (PAA) produced by reaction of triacetin (105 mM) and hydrogen peroxide (78 mM) at pH 6.5 in the presence of 1 mg of total extract protein/mL from transformant cell extracts of E. coli UM2/pSW187 expressing B. subtilis BE1010 perhydrolase.**

| SILWET® L7650 (ppm) | Turpinal® SL (ppm) | total protein (mg/mL) | PAA (ppm); 5 min | PAA (ppm); 30 min |
|---|---|---|---|---|
| 0 | 0 | 0 | 21 | 50 |
| 1000 | 0 | 0 | 20 | 26 |
| 0 | 500 | 0 | 10 | 45 |
| 1000 | 500 | 0 | 0 | 100 |
| | | | | |
| 0 | 0 | 1.0 | 1600 | 2245 |
| 1000 | 0 | 1.0 | 1550 | 2136 |
| 0 | 500 | 1.0 | 1520 | 2130 |
| 1000 | 500 | 1.0 | 1505 | 2080 |

### EXAMPLE 32

### Perhydrolase Activity with Wetting Agent, Chelating Agent and Corrosion Inhibitor

A cell extract of *E. coli* UM2/pSW187 transformant expressing *B. subtilis* BE1010 perhydrolase was prepared according to the procedure described in Example 21. Separate 1-mL reactions containing triacetin (105 mM), hydrogen peroxide (78 mM), wetting agent (SILWET® L7650), chelating agent (TURPINAL® SL), corrosion inhibitor (benzotriazole) and 1 mg of extract total protein in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. The concentration of peracetic acid in the reaction mixtures (Table 20) was determined according to the method of Karst *et al.* described in Example 2.

**Table 20: Peracetic acid (PAA) produced by reaction of triacetin (105 mM) and hydrogen peroxide (78 mM) at pH 6.5 in the presence of 1 mg of total extract protein/mL from transformant cell extracts of E. coli UM2/pSW187 expressing B. subtilis BE1010 perhydrolase.**

| SILWET® L7650 (ppm) | Turpinal® SL (ppm) | benzotriazole (ppm) | total protein (mg/mL) | PAA (ppm): 5 min | PAA (ppm); 30 min |
|---|---|---|---|---|---|
| 0 | 0 | 0 | 0 | 0 | 0 |
| 0 | 0 | 0 | 1.0 | 795 | 1205 |
| 1000 | 500 | 1000 | 0 | 0 | 20 |
| 1000 | 500 | 1000 | 1.0 | 825 | 960 |
| 1000 | 500 | 2500 | 0 | 0 | 24 |
| 1000 | 500 | 2500 | 1.0 | 795 | 960 |
| 1000 | 2000 | 2500 | 0 | 0 | 0 |
| 1000 | 2000 | 2500 | 1.0 | 270 | 450 |

### EXAMPLE 33

### Peracetic Acid Production using Immobilized B. subtilis ATCC 31954™ or BE1010 Perhydrolase

A suspension of 0.50 g of AMBERZYME® Oxirane enzyme immobilization polymeric support (Rohm and Haas, Philadelphia, PA) in 5.0 mL of 0.225 M sodium phosphate buffer (pH 8.0) containing 10 mg/mL of total soluble protein from extracts (prepared as described in Example 21) of either *E. coli* KMP/pSW189 (expressing *B. subtilis* BE1010 perhydrolase) or *E. coli* UM2/pSW186 (expressing *B. subtilis ATCC* 31954™ perhydrolase) was mixed on a rotating platform at room temperature for 24 h. The supernatant was then decanted from the immobilized enzyme, which was washed with four 40-mL volumes of phosphate buffer (50 mM, pH 6.5) and stored at 5 °C in this same buffer. The immobilized enzyme was dried by vacuum filtration prior to use.

Separate 1-mL reactions containing triacetin (250 mM), hydrogen peroxide (1.0 M) and either 1.5 mg/mL or 5.0 mg/ml of immobilized perhydrolase (prepared as described above) in 50 mM phosphate buffer (pH 6.5) were run at 25 °C. A control reaction was run to determine the concentration of peracetic acid produced by chemical perhydrolysis of triacetin by hydrogen peroxide in the absence of added immobilized enzyme. The concentration of peracetic acid in the reaction mixtures (Table 21) was determined according to the method of Karst *et al.* described in Example 2.

**Table 21: Peracetic acid (PAA) produced by reaction of triacetin (250 mM) and hydrogen peroxide (1.0 M) at pH 6.5 in the presence of immobilized B. subtilis ATCC 31954™ or BE1010 perhydrolase.**

| immobilized perhydrolase | catalyst loading (mg immob. enzyme/mL) | PAA (ppm); 5 min | PAA (ppm); 30 min |
|---|---|---|---|
| no enzyme | 0 | 83 | 240 |
| *B. subtilis* ATCC 31954™ | 1.5 | 185 | 700 |
| *B. subtilis* BE1010 | 1.5 | 502 | 1715 |
| | | | |
| no enzyme | 0 | 99 | 319 |
| *B. subtilis* ATCC 31954™ | 5.0 | 596 | 972 |
| *B. subtilis* BE1010 | 5.0 | 1669 | 2610 |

### EXAMPLE 34

### Perhydrolysis of a Mixture of Diacetin, Triacetin, and Monoacetin Using Perhydrolases from B. subtilis. B. licheniformis and C. thermocellum

Separate 1-mL reactions containing a mixture of diacetin (118 mM), triacetin (42 mM) and monoacetin (90 mM), hydrogen peroxide (1.0 M) and 50 µg of extract total protein from an *E. coli* UM2 cell extract (prepared as described Example 21) that contained perhydrolase in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. A second control reaction was run using 50 µg of extract total protein prepared from an extract of untransformed *E. coli* UM2 to determine the background level of peracid produced by the *E. coli* strain in the absence of expressed perhydrolase. The concentration of peracetic acid in the reaction mixtures (Table 22) was determined according to the method of Karst *et al.* described in Example 2.

**Table 22: Peracetic acid (PAA) produced by reaction of a mixture of diacetin (118 mM), triacetin (42 mM) and monoacetin (90 mM) with hydrogen peroxide (1.0 M) at pH 6.5 in the presence of 50 µg of total extract protein/mL from transformant cell extracts of E. coli UM2 expressing perhydrolase.**

| transformant cell extract | perhydrolase source | PAA (ppm); 5 min | PAA (ppm); 30 min |
|---|---|---|---|
| no extract | control | 76 | 270 |
| UM2 | none (control) | 110 | 276 |
| UM2/pSW186 | *B. subtilis* ATCC 31954™ | 2352 | 4341 |
| UM2/pSW187 | *B. subtilis* BE1010 | 2710 | 4713 |
| UM2/pSW188 | *B. subtilis* ATCC 6633™ | 2685 | 4234 |
| UM2/pSW190 | *B. subtilis* ATCC 29233™ | 641 | 1889 |
| UM2/pSW191 | *B. licheniformis ATCC* 14580™ | 1183 | 2608 |
| UM2/pSW193 | *C. thermocellum* ATCC 27405™ | 1498 | 1708 |

### EXAMPLE 35

### Perhydrolysis of a Mixture of Diacetin. Triacetin, and Monoacetin Using Perhydrolase from B. subtilis BE1010

Separate 1-mL reactions containing a mixture of diacetin (49.6 mM), triacetin (17.6 mM) and monoacetin (37.8 mM), hydrogen peroxide (78 mM) and 1 mg or 2 mg of extract total protein from a cell extract (prepared as described Example 21) in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. The concentration of peracetic acid in the reaction mixtures (Table 23) was determined according to the method of Karst *et al.* described in Example 2.

**Table 23: Peracetic acid (PAA) produced by reaction of triacetin (105 mM) and hydrogen peroxide (78 mM) at pH 6.5 in the presence of 1 mg or 2 mg of total extract protein/mL from transformant cell extracts of E. coli KLP18/pSW189 expressing B. subtilis BE1010 perhydrolase.**

| transformant cell extract | source of perhydrolase | total protein (mg/mL) | pH | PAA (ppm); 5 min | PAA (ppm); 30 min |
|---|---|---|---|---|---|
| no extract | control | 0 | 6.5 | 0 | 0 |
| KLP18/pSW189 | *B. subtilis* BE1010 | 1.0 | 6.5 | 475 | 423 |
| KLP18/pSW189 | *B. subtilis* BE1010 | 2.0 | 6.5 | 505 | 463 |

### EXAMPLE 36

### Perhydrolysis of Acetytated Sugars by B. subtilis ATCC 31954™Perhydrolase

A cell extract of *E. coli* UM2/pSW186 transformant expressing *B. subtilis* ATCC 31954™ perhydrolase was prepared according to the procedure described in Example 21. Separate 1-mL reactions containing 0.1 M acetylated sugar (β-D-ribofuranose-1,2,3,5-tetraacetate, tri-O-acetyl-D-galactal, or tri-O-acetyl-D-glucal (Aldrich)), hydrogen peroxide (100 or 500 mM), 2 mg of extract total protein in 50 mM phosphate buffer (pH 6.5) were run at 25°C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. The concentration of peracetic acid in the reaction mixtures (Table 24) was determined according to the method of Karst *et al.* described in Example 2.

**Table 24: Peracetic acid (PAA) produced by reaction of acetylated sugar (100 mM) and hydrogen peroxide (100 or 500 mM) at pH 6.5 in the presence of 2 mg of total extract protein/mL from transformant cell extracts of E. coli UM2/pSW186 expressing B. subtilis ATCC 31954™ perhydrolase.**

| acetylated sugar | hydrogen peroxide (mM) | protein (mg/mL) | PAA (ppm); 5 min | PAA (ppm); 30 min |
|---|---|---|---|---|
| β-D-ribofuranose-1,2,3,5-tetraacetate | 500 | 0 | 550 | 705 |
| β-D-ribofuranose-1,2,3,5-tetraacetate | 500 | 2.0 | 1115 | 1540 |
| tri-O-acetyl-D-galactal | 500 | 0 | 220 | 225 |
| tri-O-acetyl-D-galactal | 500 | 2.0 | 885 | 815 |
| tri-O-acetyl-D-glucal | 500 | 0 | 20 | 25 |
| tri-O-acetyl-D-glucal | 500 | 2.0 | 420 | 275 |
| | | | | |
| β-D-ribofuranose-1,2,3,5-tetraacetate | 100 | 0 | 52 | 37 |
| β-D-ribofuranose-1,2,3,5-tetraacetate | 100 | 2.0 | 289 | 354 |
| tri-O-acetyl-D-galactal | 100 | 0 | 5 | 95 |
| tri-O-acetyl-D-galactal | 100 | 2.0 | 185 | 175 |
| tri-O-acetyl-D-glucal | 100 | 0 | 65 | 0 |
| tri-O-acetyl-D-glucal | 100 | 2.0 | 102 | 60 |

### EXAMPLE 37

### Perhydrolysis of Acetylated Sugars by B. subtilis BE1010 Perhydrolase

A cell extract of *E. coli* KLP18/pSW189 transformant expressing B. *subtilis* BE1010 perhydrolase was prepared according to the procedure described in Example 21. Separate 1-mL reactions containing 0.1 M acetylated sugar (β-D-ribofuranose-1,2,3,5-tetraacetate, tri-O-acetyl-D-galactal, or tri-O-acetyl-D-glucal (Aldrich)), hydrogen peroxide (100 or 500 mM), 2 mg of extract total protein in 50 mM phosphate buffer (pH 6.5) were run at 25 °C. A control reaction was run by substituting 50 mM phosphate buffer (pH 6.5) for the extract total protein solution to determine the concentration of peracetic acid produced by chemical perhydrolysis of triacetin by hydrogen peroxide in the absence of added extract protein. The concentration of peracetic acid in the reaction mixtures (Table 25) was determined according to the method of Karst *et a*/*.* described in Example 2.

**Table 25: Peracetic acid (PAA) produced by reaction of acetylated sugar (100 mM) and hydrogen peroxide (100 or 500 mM) at pH 6.5 in the presence of 2 mg of total extract protein/mL from transformant cell extracts of E. coli KLP18/pSW189 transformant expressing B. subtilis BE1010 perhydrolase.**

| acetylated sugar | hydrogen peroxide (mM) | total protein (mg/mL) | PAA (ppm); 5 min | PAA (ppm); 30 min |
|---|---|---|---|---|
| β-D-ribofuranose-1,2,3,5-tetraacetate | 500 | 0 | 550 | 705 |
| β-D-ribofuranose-1,2,3,5-tetraacetate | 500 | 2.0 | 1465 | 1950 |
| tri-O-acetyl-D-galactal | 500 | 0 | 185 | 375 |
| tri-O-acetyl-D-galactal | 500 | 2.0 | 880 | 985 |
| tri-O-acetyl-D-glucal | 500 | 0 | 10 | 40 |
| tri-O-acetyl-D-glucal | 500 | 2.0 | 770 | 405 |
| | | | | |
| β-D-ribofurariose-1,2,3,5-tetraacetate | 100 | 0 | 52 | 37 |
| β-D-ribofuranose-1,2,3,5-tetraacetate | 100 | 2.0 | 360 | 437 |
| tri-O-acetyl-D-galactal | 100 | 0 | 102 | 112 |
| tri-O-acetyl-D-galactal | 100 | 2.0 | 305 | 262 |
| tri-O-acetyl-D-glucal | 100 | 0 | 12 | 17 |
| tri-O-acetyl-D-glucal | 100 | 2.0 | 240 | 137 |

## Claims

1. A process for producing a peroxycarboxylic acid from a carboxylic acid ester comprising:
a) providing a set of reaction components, said components comprising:
1) a carboxylic acid ester selected from the group consisting of:
i) esters having the structure 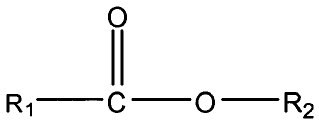 wherein R₁= C1 to C7 straight chain or branched chain alkyl optionally substituted with an hydroxyl or a C1 to C4 alkoxy group and R₂= C1 to C10 straight chain or branched chain alkyl, alkenyl, alkynyl, aryl, alkylaryl, alkylheteroaryl, heteroaryl, (CH₂CH₂-O)ₙH or (CH₂CH(CH₃)-O)ₙH and n=1 to 10;
ii) glycerides having the structure 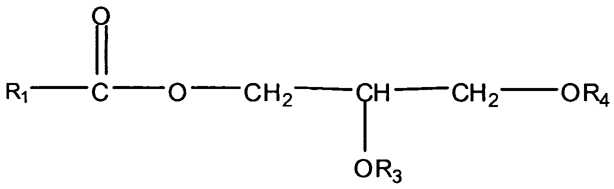 wherein R₁= C1 to C7 straight chain or branched chain alkyl optionally substituted with an hydroxyl or a C1 to C4 alkoxy group and R₃ and R₄ are individually H or R₁C(O); and
iii) acetylated carbohydrates;
2) a source of peroxygen; and
3) an enzyme catalyst having perhydrolysis activity, wherein said enzyme catalyst comprises:
an enzyme having at least 90% amino acid identity to an amino acid sequence of SEQ ID NO: 16, 18, 8, 10, 12, 14, 20, 22, 24, or 26; and
b) combining said reaction components under suitable aqueous reaction conditions, wherein said conditions comprise a pH range of about 2 to about 9, whereby a peroxycarboxylic acid is produced.

2. A process to reduce a concentration of a microbial population on a hard surface or inanimate object using an enzymatically-produced peroxycarboxylic acid composition, said process comprising:
a) providing a set of reaction components, said components comprising:
1) a substrate selected from the group consisting of:
i) esters having the structure 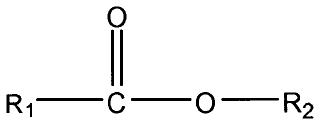 wherein R₁= C1 to C7 straight chain or branched chain alkyl optionally substituted with an hydroxyl or a C1 to C4 alkoxy group and R₂= C1 to C10 straight chain or branched chain alkyl, alkenyl, alkynyl, aryl, alkylaryl, alkylheteroaryl, heteroaryl, (CH₂CH₂-O)ₙH or (CH₂CH(CH₃)-O)ₙH and n=1 to 10;
ii) glycerides having the structure 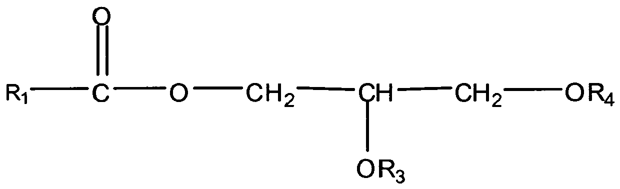 wherein R₁= C1 to C7 straight chain or branched chain alkyl optionally substituted with an hydroxyl or a C1 to C4 alkoxy group and R₃ and R₄ are individually H or R₁C(O); and
iii) acetylated carbohydrates;
2) a source of peroxygen; and
3) an enzyme catalyst having perhydrolysis activity, wherein said enzyme catalyst comprises:
an enzyme having at least 90% amino acid identity to an amino acid sequence of SEQ ID NO: 16, 18, 8, 10, 12, 14, 20, 22, 24, or 26;
b) combining said reaction components under suitable aqueous reaction conditions, wherein said conditions comprise a pH range of about 2 to about 9, whereby a peroxycarboxylic acid is formed;
c) optionally diluting said peroxycarboxylic acid product; and
d) contacting said hard surface or inanimate object with the peroxycarboxylic acid produced in step b) or step c) whereby viable microorganisms on said surface or said inanimate object are reduced at least 3 log;
wherein said hard surface or inanimate object is not a living animal.

3. A process as claimed in claim 2, wherein the hard surface or inanimate object is selected from equipment surfaces found in the food or beverage industry (such as tanks, conveyors, floors, drains, coolers, freezers, equipment surfaces, walls, valves, belts, pipes, drains, joints, crevasses, combinations thereof, and the like); building surfaces (such as walls, floors and windows); non-food-industry related pipes and drains, including water treatment facilities, pools and spas, and fermentation tanks; hospital or veterinary surfaces (such as walls, floors, beds, equipment (such as endoscopes), clothing worn in hospital/veterinary or other healthcare settings, including scrubs, shoes and other hospital or veterinary surfaces); restaurant surfaces; bathroom surfaces; toilets; clothes and shoes; surfaces of barns or stables for livestock, such as poultry, cattle, dairy cows, goats, horses and pigs; hatcheries for poultry or shrimp; food products, such as beef, poultry, pork, vegetables, fruits, seafood, combinations thereof, and the like; water absorbent materials such as infected linens or other textiles; harvested plants or plant products including seeds, corms, tubers, fruit, and vegetables, growing plants, and especially crop growing plants, including cereals, leaf vegetables and salad crops, root vegetables, legumes, berried fruits, citrus fruits and hard fruits.

4. A peroxycarboxylic acid generating system comprising:
a) a substrate selected from the group consisting of:
1) esters having the structure 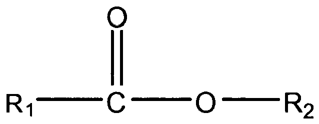 wherein R₁= C1 to C7 straight chain or branched chain alkyl optionally substituted with an hydroxyl or a C1 to C4 alkoxy group and R₂= C1 to C10 straight chain or branched chain alkyl, alkenyl, alkynyl, aryl, alkylaryl, alkylheteroaryl, heteroaryl, (CH₂CH₂-O)ₙH or (CH₂CH(CH₃)-O)ₙH and n=1 to 10;
2) glycerides having the structure 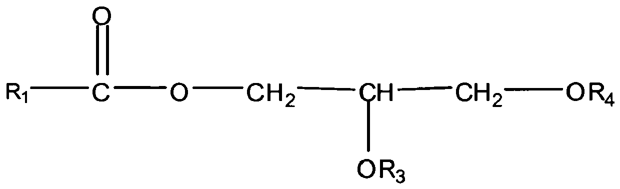 wherein R₁= C1 to C7 straight chain or branched chain alkyl optionally substituted with an hydroxyl or a C1 to C4 alkoxy group and R₃ and R₄ are individually H or R₁C(O); and
3) acetylated carbohydrates;
b) a source of peroxygen; and
c) an enzyme catalyst having perhydrolysis activity, wherein said enzyme catalyst comprises:
an enzyme having at least 90% amino acid identity to an amino acid sequence of SEQ ID NO: 16, 18, 8, 10, 12, 14, 20, 22, 24, or 26.

5. A process as claimed in any of claims 1-3, wherein the enzyme comprises an amino acid sequence which is encoded by SEQ ID NO: 15, 17, 7, 9, 11, 13, 19, 21, 23, or 25.

6. A process as claimed in any of claims 1-3, wherein the peroxycarboxylic acid is produced at a concentration of at least 20 ppm within about 5 minutes to about 2 hours of combining the reaction components.

7. A process as claimed in any of claims 1-3, wherein the peroxycarboyxlic acid produced is selected from the group consisting of peracetic acid, perpropionic acid, perbutyric acid, perlactic acid, perglycolic acid, permethoxyacetic acid, per-p-hydroxybutyric acid, and mixtures thereof.

8. A process as claimed in any of claims 1-3, wherein the peroxycarboxylic acid produced is peracetic acid.

## Patentansprüche

1. Verfahren für die Herstellung einer Peroxycarbonsäure aus einem Carbonsäurester, umfassend:
(a) das Bereitstellen eines Satzes von Reaktionskomponenten, wobei die Komponenten Folgendes umfassen:
1) einen Carbonsäureester ausgewählt aus der Gruppe bestehend aus:
i) Estern, die die Struktur 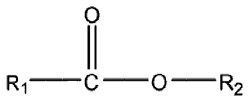 aufweisen, wobei R₁ = geradkettiges oder verzweigtkettiges C1- bis C7-Alkyl, das wahlweise mit einer Hydroxyl- oder einer C1- bis C4-Alkoxygruppe substituiert ist, und R₂ = geradkettiges oder verzweigtkettiges C1- bis C10-Alkyl,-Alkenyl, -Alkynyl, -Aryl, -Alkylaryl, -Alkylheteroaryl, -Heteroaryl, (CH₂CH₂-O)ₙH oder (CH₂CH(CH₃)-O)ₙH ist und n = 1 bis 10;
ii) Glyceriden, die die Struktur 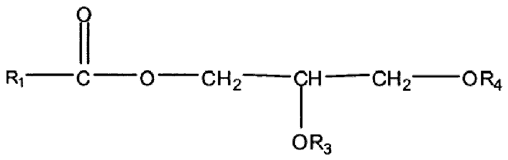 aufweisen, wobei R₁ = geradkettiges oder verzweigtkettiges Cl- bis C7-Alkyl, das wahlweise mit einer Hydroxyl- oder einer Cl- bis C4-Alkoxygruppe substituiert ist, und R₃ und R₄ einzeln H oder R₁C(O) sind; und
iii) acetylierten Kohlehydraten;
2) eine Persauerstoffquelle; und
3) einen Enzymkatalysator, der eine Perhydrolyseaktivität aufweist, wobei der Enzymkatalysator Folgendes umfasst:
ein Enzym, das mindestens 90 % Aminosäureidentität mit einer Aminosäuresequenz von SEQ ID NO: 16, 18, 8, 10, 12, 14, 20, 22, 24 oder 26 aufweist; und
b) das Kombinieren der Reaktionskomponenten unter geeigneten wässrigen Reaktionsbedingungen, wobei die Bedingungen einen pH-Wertbereich von etwa 2 bis etwa 9 umfassen, wobei eine Peroxycarbonsäure hergestellt wird.

2. Verfahren zum Reduzieren einer Konzentration einer mikrobiellen Population auf einer harten Oberfläche oder einem leblosen Objekt unter Anwendung einer enzymatisch hergestellten Peroxycarbonsäurezusammensetzung, wobei das Verfahren Folgendes umfasst:
a) das Bereitstellen eines Satzes von Reaktionskomponenten, wobei die Komponenten Folgendes umfassen:
1) ein Substrat ausgewählt aus der Gruppe bestehend aus:
i) Estern, die die Struktur 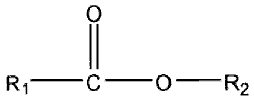 aufweisen, wobei R₁ = geradkettiges oder verzweigtkettiges Cl- bis C7-Alkyl, das wahlweise mit einer Hydroxyl- oder einer Cl- bis C4-Alkoxygruppe substituiert ist, und R₂ = geradkettiges oder verzweigtkettiges Cl- bis C10-Alkyl,-Alkenyl, -Alkynyl, -Aryl, -Alkylaryl, -Alkylheteroaryl, -Heteroaryl, (CH₂CH₂-O)ₙH oder (CH₂CH(CH₃)-O)ₙH ist und n = 1 bis 10;
ii) Glyceriden, die die Struktur 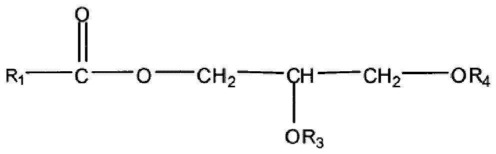 aufweisen, wobei R₁ = geradkettiges oder verzweigtkettiges C1- bis C7-Alkyl, das wahlweise mit einem Hydroxyl substituiert ist, oder eine C1- bis C4-Alkoxygruppe ist und R₃ und R₄= einzeln H oder R₁C(O) sind; und
iii) acetylierten Kohlehydraten;
2) eine Persauerstoffquelle; und
3) einen Enzymkatalysator, der eine Perhydrolyseaktivität aufweist, wobei der Enzymkatalysator Folgendes umfasst:
ein Enzym, das mindestens 90 % Aminosäureidentität mit einer Aminosäuresequenz von SEQ ID NO: 16, 18, 8, 10, 12, 14, 20, 22, 24 oder 26 aufweist; und
b) das Kombinieren der Reaktionskomponenten unter geeigneten wässrigen Reaktionsbedingungen, wobei die Bedingungen einen pH-Wertbereich von etwa 2 bis etwa 9 umfassen, wobei eine Peroxycarbonsäure gebildet wird;
c) wahlweise das Verdünnen des Peroxycarbonsäureprodukts; und
d) das Kontaktieren der harten Oberfläche oder des leblosen Objekts mit der in Schritt b) oder Schritt c) hergestellten Peroxycarbonsäure, wodurch lebefähige Mikroorganismen auf der Oberfläche oder dem leblosen Objekt um mindestens 3 log reduziert werden;
wobei die harte Oberfläche oder das leblose Objekt kein lebendiges Tier ist.

3. Verfahren nach Anspruch 2, wobei die harte Oberfläche oder das leblose Objekt ausgewählt sind unter Oberflächen von Vorrichtungen, die in der Nahrungsmittel- oder Getränkeindustrie (wie beispielsweise Behältern, Fördergeräten, Fußböden, Abflussrohren, Kühlvorrichtungen, Gefriertruhen, Oberflächen von Vorrichtungen, Wänden, Ventilen, Bändern, Rohren, Ablassrohren, Fugen, Rissen, Kombinationen davon und dergleichen) anzutreffen sind; Gebäudeoberflächen (wie Wänden, Fußböden und Fenstern); nicht mit der Nahrungsmittelindustrie verbundenen Rohren und Ablassrohren, einschließlich Wasseraufbereitungsanlagen, Schwimmbecken und Bädern und Fermentationsbehältern; Krankenhaus- oder veterinäre Flächen (wie Wänden, Fußböden, Betten, Vorrichtungen (wie Endoskopen), in Krankenhaus-/-veterinären oder anderen Gesundheitsfürsorgeumgebungen getragener Kleidung, einschließlich OP-Kitteln, Schuhen und anderen Krankenhaus- oder veterinären Oberflächen); Restaurantoberflächen; Badezimmeroberflächen; Toiletten; Kleidungsstücken und Schuhen; Oberflächen von Scheunen oder Ställen für Viehbestand wie Geflügel, Rindvieh, Milchkühe, Ziegen, Pferde und Schweine; Brutstätten/Laichplätzen für Geflügel oder Garnelen; Nahrungsmittelprodukten wie Rindfleisch, Geflügel, Schweinefleisch, Gemüse, Obst, Meeresfrüchte, Kombinationen davon und dergleichen; wasserabsorptionsfähigen Materialien wie infizierte Bettwäsche oder andere Textilien; geernteten Pflanzen oder Pflanzenprodukten, einschließlich Samen, Knollen, Wurzelknollen, Obst und Gemüse, wachsende Pflanzen und insbesondere Anbaupflanzen, einschließlich Zerealien, Blattgemüse und Salatpflanzen, Wurzelgemüse, Hülsenfrüchte, Beerenobst, Zitrusobst und Hartobst.

4. Peroxycarbonsäure erzeugendes System, umfassend:
a. ein Substrat ausgewählt aus der Gruppe bestehend aus:
1) Estern, die die Struktur 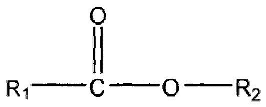 aufweisen, wobei R₁ = geradkettiges oder verzweigtkettiges C1- bis C7-Alkyl, das wahlweise mit einer Hydroxyl- oder einer C1- bis C4-Alkoxygruppe substituiert ist, und R₂ = geradkettiges oder verzweigtkettiges C1 - bis C10-Alkyl, -Alkenyl, -Alkynyl,-Aryl, -Alkylaryl, -Alkylheteroaryl, Heteroaryl, (CH₂CH₂-O)ₙH oder (CH₂CH(CH₃)-O)ₙH ist und n = 1 bis 10;
2) Glyceriden, die die Struktur 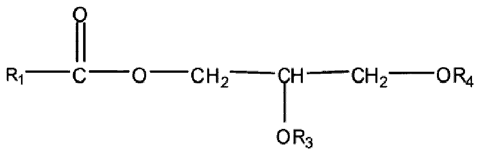 aufweisen, wobei R₁ = geradkettiges oder verzweigtkettiges C1- bis C7-Alkyl, das wahlweise mit einer Hydroxyl- oder einer C1- bis C4-Alkoxygruppe substituiert ist, und R₃ und R₄= einzeln H oder R₁C(O) sind; und
3) acetylierten Kohlehydraten;
b) eine Persauerstoffquelle; und
c) einen Enzymkatalysator, der eine Perhydolyseaktivität aufweist, wobei der Enzymkatalysator Folgendes umfasst:
ein Enzym, das mindestens 90 % Aminosäureidentität mit einer Aminosäuresequenz von SEQ ID NO: 16, 18, 8, 10, 12, 14, 20, 22, 24 oder 26 aufweist.

5. Verfahren nach einem der Ansprüche 1 - 3, wobei das Enzym eine Aminosäuresequenz aufweist, die durch SEQ ID NO: 15, 17, 7, 9, 11, 13, 19, 21, 23 oder 25 kodiert ist.

6. Verfahren nach einem der Ansprüche 1 - 3, wobei die Peroxycarbonsäure in einer Konzentration von mindestens 20 ppm innerhalb von 5 Minuten bis etwa 2 Stunden des Kombinierens der Reaktionskomponenten hergestellt wird.

7. Verfahren nach einem der Ansprüche 1 - 3, wobei die hergestellte Peroxycarbonsäure aus der Gruppe ausgewählt ist bestehend aus Peressigsäure, Perpropionsäure, Perbuttersäure, Permilchsäure, Perglycolsäure, Permethoxyessigsäure, per-ß-hydroxybuttersäure und Mischungen davon.

8. Verfahren nach einem der Ansprüche 1 - 3, wobei die hergestellte Percarbonsäure Peressigsäure ist.

## Revendications

1. Procédé de production d'un acide peroxycarboxylique à partir d'un ester d'acide carboxylique, comprenant:
a) fournir un jeu de composants de réaction, lesdits composants comprenant:
1) un ester d'acide carboxylique sélectionné parmi le groupe consistant en:
i) des esters ayant la structure 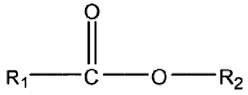 où R₁ = un alkyle à chaîne droite ou ramifiée en C1 à C7 optionnellement substitué par un hydroxyle ou un groupe alcoxy en C1 à C4 et R₂ = un alkyle à chaîne droite ou ramifiée en C1 à C10, un alcényle, alcynyle, aryle, alkylaryle, alkylhétéroaryle, hétéroaryle, (CH₂CH₂-O)ₙH ou (CH₂CH(CH₃)-O)ₙH et n = 1 à 10;
ii) des glycérides ayant la structure 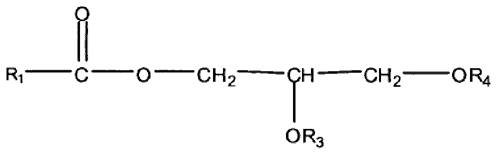 où R₁ = un alkyle à chaîne droite ou ramifiée en C1 à C7 optionnellement substitué par un hydroxyle ou un groupe alcoxy en C1 à C4 et R₃ et R₄ sont individuellement H ou R₁C(O); et
iii) des glucides acétylés;
2) une source de peroxygène; et
3) une enzyme catalyseur ayant une activité de perhydrolyse, où ladite enzyme catalyseur comprend:
une enzyme ayant au moins 90 % d'identité d'acides aminés avec une séquence d'acides aminés de la SEQ ID NO: 16, 18, 8, 10, 12, 14, 20, 22, 24 ou 26; et
b) combiner lesdits composants de réaction dans des conditions de réaction aqueuses appropriées, où lesdites conditions comprennent une plage de pH d'environ 2 à environ 9, en vertu de quoi un acide peroxycarboxylique est produit.

2. Procédé destiné à réduire une concentration d'une population microbienne sur une surface dure ou un objet inanimé en utilisant une composition d'acide peroxycarboxylique produit par voie enzymatique, ledit procédé comprenant:
a) fournir un jeu de composants de réaction, lesdits composants comprenant:
1) un substrat sélectionné parmi le groupe consistant en:
i) des esters ayant la structure 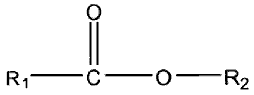 où R₁ = un alkyle à chaîne droite ou ramifiée en C1 à C7 optionnellement substitué par un hydroxyle ou un groupe alcoxy en C1 à C4 et R₂ = un alkyle à chaîne droite ou ramifiée en C1 à C10, un alcényle, alcynyle, aryle, alkylaryle, alkylhétéroaryle, hétéroaryle, (CH₂CH₂-O)ₙH ou (CH₂CH(CH₃)-O)ₙH et n = 1 à 10;
ii) des glycérides ayant la structure 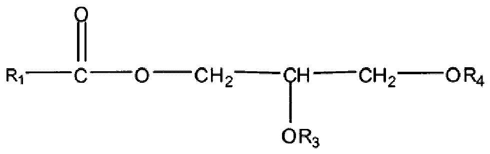 où R₁ = un alkyle à chaîne droite ou ramifiée en C1 à C7 optionnellement substitué par un hydroxyle ou un groupe alcoxy en C1 à C4 et R₃ et R₄ sont individuellement H ou R₁C(O); et
iii) des glucides acétylés;
2) une source de peroxygène; et
3) une enzyme catalyseur ayant une activité de perhydrolyse, où ladite enzyme catalyseur comprend:
une enzyme ayant au moins 90 % d'identité d'acides aminés avec une séquence d'acides aminés de la SEQ ID NO: 16, 18, 8, 10, 12, 14, 20, 22, 24 ou 26;
b) combiner lesdits composants de réaction dans des conditions de réaction aqueuses appropriées, où lesdites conditions comprennent une plage de pH d'environ 2 à environ 9, en vertu de quoi un acide peroxycarboxylique est formé;
c) optionnellement diluer ledit produit d'acide peroxycarboxylique; et
d) mettre ladite surface dure ou ledit objet inanimé en contact avec l'acide peroxycarboxylique produit à l'étape b) ou c), en vertu de quoi des micro-organismes viables sur ladite surface ou sur ledit objet inanimé sont réduits d'au moins 3 log;
où ladite surface dure ou ledit objet inanimé n'est pas un animal vivant.

3. Procédé selon la revendication 2, dans lequel ladite surface dure ou ledit objet inanimé est sélectionné parmi des surfaces d'équipements que l'on trouve dans l'industrie alimentaire et des boissons (tels que cuves, transporteurs, sols, égouts, refroidisseurs, congélateurs, surfaces d'équipements, parois/murs, robinets, courroies, tuyaux, canalisations, raccords, crevasses, des combinaisons de ceux-ci et ainsi de suite); des surfaces de bâtiment (telles que murs, sols et fenêtres); tuyaux et canalisations non associés à l'industrie alimentaire, y compris installations de traitement d'eau, bassins et sources thermales, et bacs de fermentation; surfaces hospitalières ou vétérinaires (telles que murs, sols, lits, équipements (tels qu'endoscopes), vêtements portés en milieu hospitalier/vétérinaire ou autres établissements de soins médicaux, y compris vêtements chirurgicaux, chaussures et autres surfaces hospitalières ou vétérinaires); surfaces de restaurant; surfaces de salle de bain; toilettes; vêtements et chaussures; surfaces de granges ou d'étables pour animaux sur pied, tels que volaille, bétail, vaches laitières, chèvres, chevaux et porcs; couvoirs pour volaille ou crevettes; produits alimentaires, tels que boeuf, volaille, porc, légumes, fruits, fruits de mer, des combinaisons de ceux-ci et ainsi de suite; matériaux qui absorbent l'eau tels que linges ou autres textiles infectés; plantes récoltées ou produits végétaux y compris graines, bulbes, tubercules, fruits, et légumes, plantes en croissance et surtout plantes de culture agricole, y compris céréales, légumes à feuilles comestibles et cultures de salade, légumes à racine comestible, légumineuses, fruits à baies, agrumes et fruits durs.

4. Système de production d'acide peroxycarboxylique, comprenant:
a) un substrat sélectionné parmi le groupe consistant en:
1) des esters ayant la structure 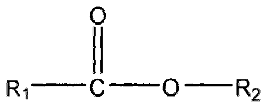 où R₁ = un alkyle à chaîne droite ou ramifiée en C1 à C7 optionnellement substitué par un hydroxyle ou un groupe alcoxy en C1 à C4 et R₂ = un alkyle à chaîne droite ou ramifiée en C1 à C10, un alcényle, alcynyle, aryle, alkylaryle, alkylhétéroaryle, hétéroaryle, (CH₂CH₂-O)ₙH ou (CH₂CH(CH₃)-O)ₙH et n = 1 à 10;
2) des glycérides ayant la structure 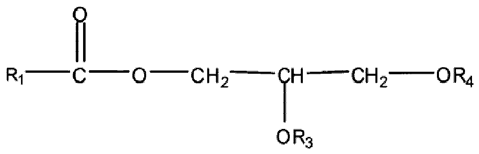 où R₁ = un alkyle à chaîne droite ou ramifiée en C1 à C7 optionnellement substitué par un hydroxyle ou un groupe alcoxy en C1 à C4 et R₃ et R₄ sont individuellement H ou R₁C(O); et
3) des glucides acétylés;
b) une source de peroxygène; et
c) une enzyme catalyseur ayant une activité de perhydrolyse, où ladite enzyme catalyseur comprend:
une enzyme ayant au moins 90 % d'identité d'acides aminés avec une séquence d'acides aminés de la SEQ ID NO: 16, 18, 8, 10, 12, 14, 20, 22, 24 ou 26.

5. Procédé selon l'une quelconque des revendications 1-3, dans lequel l'enzyme comprend une séquence d'acides aminés qui est codée par la SEQ ID NO: 15, 17, 7, 9,11, 13, 19, 21, 23 ou 25.

6. Procédé selon l'une quelconque des revendications 1-3, dans lequel l'acide peroxycarboxylique est produit à une concentration d'au moins 20 ppm en l'espace d'environ 5 minutes à environ 2 heures à compter de la combinaison des composants de réaction.

7. Procédé selon l'une quelconque des revendications 1-3, dans lequel l'acide peroxycarboxylique produit est sélectionné parmi le groupe consistant en acide peracétique, acide perpropionique, acide perbutyrique, acide perlactique, acide perglycolique, acide perméthoxyacétique, acide per-β-hydroxybutyrique et des mélanges de ceux-ci.

8. Procédé selon l'une quelconque des revendications 1-3, dans lequel l'acide peroxycarboxylique produit est de l'acide peracétique.
